Europäisches Patentamt
European Patent Office
Office européen des brevets

(19) (11) Publication number: **0 069 521 A2**

# EUROPEAN PATENT APPLICATION

(12)

(21) Application number: 82303365.9

(22) Date of filing: 28.06.82

(51) Int. Cl.[3]: C 07 D 405/06
C 07 D 409/06, C 07 D 405/04
C 07 D 405/12

(30) Priority: 01.07.81 US 279374

(43) Date of publication of application: 12.01.83 Bulletin 83/2

(84) Designated Contracting States: BE CH DE FR GB IT LI NL SE

(71) Applicant: THE UPJOHN COMPANY
301 Henrietta Street
Kalamazoo, Michigan 49001(US)

(72) Inventor: Johnson, Roy Allen
2122 Frederick Avenue
Kalamazoo Michigan(US)

(72) Inventor: Sih, John CHarles
914 Ellendale
Kalamazoo Michigan(US)

(72) Inventor: Lin, Chiu-Hong
3720 Pinetree
Portage Michigan(US)

(72) Inventor: Bundy, Gordon Leonard
7622 Ravenswood Dr.
Portage Michigan(US)

(74) Representative: Perry, Robert Edward et al,
GILL JENNINGS & EVERY 53-64 Chancery Lane
London WC2A 1HN(GB)

(54) **Pyridinyl and imidazolyl derivatives of benzofurans and benzothiophenes.**

(57) Compounds of the formula

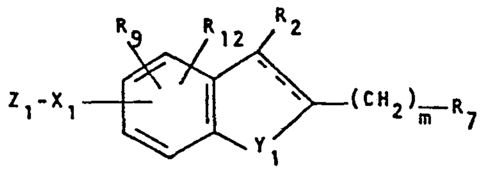

wherein $Z_1$ is pyridinyl or imidazolyl; $X_1$ is $-(CH_2)_n-$, $-O-$, $-S-$, $-SO-$, $-SO_2$, $-CH_2-O-$, $-O-CH_2-$, $-CH_2-NR_3-$, $-NR_3-CH_2-$, $-CHOH-$ or $-CO-$; $Y_1$ is $-O-$ or $-S-$; $R_2$ is H, $C_{1-3}$ alkyl, phenyl or optionally esterified carboxyl; $R_7$ is H, $CH_2OH$, optionally esterified carboxyl, CN, aminomethyl, aminocarbonyl, CHO or a ketone; either $R_9$ and $R_{12}$ are independently selected from hydrogen, hydroxy, $C_{1-4}$ alkyl, fluorine, chlorine, bromine and methoxy, or $R_9$ and $R_{12}$ are attached to adjacent carbon atoms and together are $-O-CH_2-O-$;

⩵ represents a single bond (in which case the compound may be in enantiomeric or racemic form) or a double bond; and *m* and *n* are 0, 1, 2, 3 or 4. These compounds can inhibit $TXA_2$ synthetase.

EP 0 069 521 A2

BAD ORIGINAL

# PYRIDINYL AND IMIDAZOLYL DERIVATIVES OF BENZOFURANS AND BENZOTHIOPHENES

The present invention relates to novel organic compounds which are pyridinyl and imidazolyl derivatives of benzofurans and benzothiophenes. These compounds can have utility as thromboxane $A_2$ inhibitors.

Since the discovery that human platelets convert the prostaglandin endoperoxide (PGH) into a labile pro-aggregatory molecule known as thromboxane $A_2$ ($TXA_2$), researchers have sought compounds that could selectively inhibit the biological activity of $TXA_2$. This end may be achieved in two different ways: the synthesis of $TXA_2$ can be blocked by inhibiting $TXA_2$ synthetase, or a compound can be used which is a receptor level antagonist of $TXA_2$. As therapeutic agents, $TXA_2$ synthetase inhibitors are more useful; see for example, Gorman, "Biological and Pharmacological Evaluation of Thromboxane Synthetase Inhibitors ", Advances in Prostaglandin and Thromboxane Research, 6:417 (1980), and references cited therein. Selective inhibitors of $TXA_2$ synthetase are particularly important.

A number of $TXA_2$ synthetase inhibitors are known, including the bi-heterocyclic 9,11-trideoxy-PGF-type compounds disclosed in US Patent Specification No 4,112,224; SQ 80,388, i.e. 1-(3-phenyl-2-propenyl)-1H-imidazole, disclosed by Harris et al., Advances in Prostaglandin and Thromboxane research 6:437 (1980); pyridine and its derivatives, as disclosed by Miyamoto et al., Advances in Prostaglandin and Thromboxane research, 6:443 (1980); and compounds disclosed in British Patent Specification No 2,039,903A. See also Tai et al., Advances in Prostaglandin and Thromboxane Research, 6:447 (1980). Other compounds which have been disclosed as thromboxane synthetase inhibitors include sodium p-benzyl-4-(1-oxo-2-(4-chlorobenzyl)-3-phenylpropyl)-phenylphosphate, imidazoles, nordihydroguaiaretic acid and 12L-hydroperoxy-5,8,10,14-eicosatetraenoic acid (HETE). As noted in the British Patent Specification given above, the inhibitory activity of these latter compounds on thromboxane synthetase is very weak, making them unsatisfactory as medicines.

Tetrahydropyridinyl- and piperidinyl-substituted benzofurans are disclosed in U.S. patent 4,259,338 as psychopharmaceuticals and antidepressants. Similar compounds are disclosed in German Offenleggunschrift 2,537,837.

SUMMARY OF THE INVENTION

Surprisingly and unexpectedly it has been found that selective thromboxane synthetase inhibition may be achieved by employing a compound of the formula F-I

wherein $Z_1$ is

(a) 4-pyridinyl,

(b) 3-pyridinyl,

(c) 3-pyridinyl substituted at the 4 position by

(1) methyl,

(2) $-OCH_3$,

(3) $-N(CH_3)_2$, or

(4) $-NH_2$, or

(5) at the 2, 4, 5, or 6 position by chlorine;

(d) imidazolyl, or

(e) imidazolyl substituted by $(C_1-C_3)$alkyl;

wherein $X_1$ is

(a) $-(CH_2)_n-$,

(b) -O-,

(c) -S-,

(d) -S(O)-,

(e) $-S(O)_2-$,

(f) $-CH_2-O-$,

(g) $-CH_2-N(R_3)-$,

(h) $-N(R_3)-CH_2-$,

(i) -CH(OH)-,

(j) -C(O)-, or

(k) $-O-CH_2-$;

with the proviso that when $X_1$ is (b), (c), (d), (e), (f), (g), (h), or (k), $Z_1$ is (a), (b), or (c), i.e., a pyridinyl substituent;

wherein $Y_1$ is

(a) -O- or

(b) -S-,

with the proviso that when $Y_1$ is -S-, $X_1$ is other than (d) or (e);

wherein $R_1$ is hydrogen, a pharmacologically acceptable cation, $(C_1-C_{12})$ alkyl, $(C_3-C_{10})$ cycloalkyl, $(C_7-C_{12})$ aralkyl, phenyl, phenyl mono-, di-, or trisubstituted by chloro, $(C_1-C_3)$ or alkyl, or phenyl para-substituted by

(a) $-NHCO-R_{25}$,

(b) $-O-CO-R_{26}$,

(c) $-CO-R_{24}$,

(d) $-O-CO-(p-Ph)-R_{27}$, or

(e) $-CH=N-NH-CO-NH_2$,

wherein $R_{24}$ is phenyl or acetamidophenyl, $R_{25}$ is methyl, phenyl, acetamidophenyl, benzamidophenyl, or amino, $R_{26}$ is methyl, phenyl, amino or methoxy; and $R_{27}$ is hydrogen or acetamido, and wherein -(p-Ph) is 1,4-phenylene;

wherein $R_2$ is

(a) hydrogen,

(b) $(C_1-C_3)$alkyl,

(c) phenyl, or

(d) $-CO_2R_1$;

wherein $R_3$ is

(a) hydrogen, or

(b) methyl;

wherein $R_4$ is

(a) hydrogen,

(b) $(C_1-C_4)$alkyl, or

(c) phenyl;

wherein $R_7$ is

(a) hydrogen,

(b) $-CH_2OH$,

(c) $-COOR_1$,

(d) $-CH_2N(R_4)_2$,

(e) $-CN$

(f) $-CON(R_4)_2$, or

(g) $-C(O)-R_4$;

wherein $R_9$ and $R_{12}$ are the same or different and are

(a) hydrogen,

(b) hydroxy,

(c) $(C_1-C_4)$alkyl,

(d) fluoro,

(e) chloro,

(f) bromo,

(g) $-OCH_3$, or,

(h) when taken together and attached to contiguous carbon atoms, $-O-CH_2-O-$;

wherein D represents a single or a double bond; and

wherein m and n are the same or different and are the integers 0 to 4, inclusive;

including, pharmacologically acceptable acid addition salts thereof; and

when D represents a single bond, an enantiomer or a racemic mixture of enantiomers thereof.

These compounds may be classified as set forth in Table 1. In Table 1, $Z_2$ is defined as

(a) 4-pyridinyl,

(b) 3-pyridinyl, or

(c) 3-pyridinyl substituted at the 4 position by

(1) methyl,

(2) $-OCH_3$,

(3) $-N(CH_3)_2$; or

(4) $-NH_2$, or

(5) at the 2, 4, 5, or 6 position by chlorine;

$Z_3$ is defined as

(a) imidazolyl, or

(b) imidazolyl substituted by $(C_1-C_3)$alkyl;

$X_2$ is defined as

(a) $-(CH_2)n-$,

(b) -O-,

(c) -S-,

(d) $-CH_2-O-$,

(e) $-CH_2-N(R_3)-$,

(f) $-N(R_3)-CH_2$,

(g) -CH(OH)-,

(h) -C(O)-, or

(i) $-O-CH_2-$;

$X_3$ is defined as

(a) $-(CH_2)n-$,

(b) -CH(OH)-, or

(c) -C(O)-;

and all other variables are as defined above. Thus, the Formula II compounds include the class "A" pyridyl-benzofurans; class "B" pyridyl-benzothiophenes; class "C" imidazolyl-benzofurans and class "D" imidazolyl-benzothiophenes.

Thus, the present invention particularly provides:

(I) a compound of the formula A-I wherein $Z_2$ is

(a) 4-pyridinyl,

(b) 3-pyridinyl, or

(c) 3-pyridinyl substituted at the 4 position by

(1) methyl,

(2) $-OCH_3$,

(3) $-N(CH_3)_2$, or

(4) $NH_2$, or

(5) at the 2, 4, 5, or 6 position by chlorine;

wherein $X_1$ is

(a) $-(CH_2)_n-$,

(b) -O-,

(c) -S-,

(d) -S(O)-,

(e) $-S(O)_2-$,

(f) $-CH_2-O-$,

(g) $-CH_2-N(R_3)-$,

(h) $-N(R_3)-CH_2-$,

(i) -CH(OH)-,

(j) -C(O)-, or

(k) $-O-CH_2-$;

wherein $R_1$ is hydrogen, a pharmacologically acceptable cation, $(C_1-C_{12})$ alkyl, $(C_3-C_{10})$ cycloalkyl, $(C_7-C_{12})$ aralkyl, phenyl, phenyl mono-, di-, or trisubstituted by chloro, $(C_1-C_3)$ or alkyl, or phenyl para-substituted by

(a) $-NHCO-R_{25}$,

(b) $-O-CO-R_{26}$,

(c) $-CO-R_{24}$,

(d) $-O-CO-(p-Ph)-R_{27}$, or

(e) $-CH=N-NH-CO-NH_2$,

wherein $R_{24}$ is phenyl or acetamidophenyl, $R_{25}$ is methyl, phenyl, acetamidophenyl, benzamidophenyl, or amino, $R_{26}$ is methyl, phenyl,

amino or methoxy; and $R_{27}$ is hydrogen or acetamido, and wherein -(p-Ph) is 1,4-phenylene;

wherein $R_2$ is

(a) hydrogen,

(b) $(C_1-C_3)$alkyl,

(c) phenyl, or

(d) $-CO_2R_1$;

wherein $R_3$ is

(a) hydrogen,

(b) methyl,

wherein $R_4$ is

(a) hydrogen,

(b) $(C_1-C_4)$alkyl, or

(c) phenyl;

wherein $R_7$ is

(a) hydrogen,

(b) $-CH_2OH$,

(c) $-COOR_1$,

(d) $-CH_2N(R_4)_2$,

(e) -CN

(f) $-CON(R_4)_2$, or

(g) $-C(O)-R_4$;

wherein $R_9$ and $R_{12}$ are the same or different and are

(a) hydrogen,

(b) $(C_1-C_4)$alkyl

(c) fluoro,

(d) chloro,

(e) bromo,

(f) $-OCH_3$, or,

(g) when taken together and attached to contiguous carbon atoms, $-O-CH_2-O-$;

wherein D represents a single or a double bond; and

wherein m and n are the same or different and are the integers 0 to 4, inclusive; including, pharmacologically acceptable acid addition salts thereof; and

when D represents a single bond, an enantiomer or a racemic mixture of enantiomers thereof;

(II) A compound of the formula B-I wherein $Z_2$ is

(a) 4-pyridinyl,

(b) 3-pyridinyl, or

(c) 3-pyridinyl substituted at the 4 position by

(1) methyl,

(2) $-OCH_3$,

(3) $-N(CH_3)_2$, or

(4) $-NH_2$, or

(5) at the 2, 4, 5, or 6 position by chlorine;

wherein $X_2$ is

(a) $-(CH_2)_n-$,

(b) -O-,

(c) -S-,

(d) $-CH_2-O-$,

(e) $-CH_2-NR_3-$,

(f) $-N(R_3)-CH_2-$,

(g) -C(OH)-,

(h) -C(O)-, or

(i) $-O-CH_2-$;

wherein $R_1$ is hydrogen, a pharmacologically acceptable cation, $(C_1-C_{12})$ alkyl, $(C_3-C_{10})$ cycloalkyl, $(C_7-C_{12})$ aralkyl, phenyl, phenyl mono-, di-, or trisubstituted by chloro, $(C_1-C_3)$ or alkyl, or phenyl para-substituted by

(a) $-NHCO-R_{25}$,

(b) $-O-CO-R_{26}$,

(c) $-CO-R_{24}$,

(d) $-O-CO-(p-Ph)-R_{27}$, or

(e) $-CH=N-NH-CO-NH_2$,

wherein $R_{24}$ is phenyl or acetamidophenyl, $R_{25}$ is methyl, phenyl, acetamidophenyl, benzamidophenyl, or amino, $R_{26}$ is methyl, phenyl, amino or methoxy; and $R_{27}$ is hydrogen or acetamido, and wherein -(p-Ph) is 1,4-phenylene;

wherein $R_2$ is

(a) hydrogen,

(b) $(C_1-C_3)$alkyl,

(c) phenyl, or

(d) $-CO_2R_1$;

wherein $R_3$ is

(a) hydrogen,

(b) $(C_1-C_3)$alkyl, or
(c) acyl;

wherein $R_4$ is
(a) hydrogen,
(b) $(C_1-C_4)$alkyl, or
(c) phenyl;

wherein $R_7$ is
(a) hydrogen,
(b) $-CH_2OH$,
(c) $-COOR_1$,
(d) $-CH_2N(R_4)_2$,
(e) -CN
(f) $-CON(R_4)_2$, or
(g) $-C(O)-R_4$;

wherein $R_9$ and $R_{12}$ are the same or different and are
(a) hydrogen,
(b) $(C_1-C_4)$alkyl
(c) fluoro,
(d) chloro,
(e) bromo,
(f) $-OCH_3$, or,
(g) when taken together and attached to contiguous carbon atoms, $-O-CH_2-O-$;

wherein D represents a single or a double bond; and

wherein m and n are the same or different and are the integers 0 to 4, inclusive; including, pharmacologically acceptable acid addition salts thereof; and

when D represents a single bond, an enantiomer or a racemic mixture of enantiomers thereof;

(III) A compound of the formula C-I wherein $Z_3$ is
(a) imidazolyl, or
(b) imidazolyl substituted by $(C_1-C_3)$alkyl;

wherein $X_3$ is
(a) $-(CH_2)_n-$,
(b) -C(OH)-, or
(c) -C(O)-;

wherein $R_1$ is hydrogen, a pharmacologically acceptable cation, $(C_1-C_{12})$ alkyl, $(C_3-C_{10})$ cycloalkyl, $(C_7-C_{12})$ aralkyl, phenyl, phenyl

mono-, di-, or trisubstituted by chloro, ($C_1$-$C_3$) or alkyl, or phenyl para-substituted by

(a) -NHCO-$R_{25}$,
(b) -O-CO-$R_{26}$,
(c) -CO-$R_{24}$,
(d) -O-CO-(p-Ph)-$R_{27}$, or
(e) -CH=N-NH-CO-$NH_2$,

wherein $R_{24}$ is phenyl or acetamidophenyl, $R_{25}$ is methyl, phenyl, acetamidophenyl, benzamidophenyl, or amino, $R_{26}$ is methyl, phenyl, amino or methoxy; and $R_{27}$ is hydrogen or acetamido, and wherein -(p-Ph) is 1,4-phenylene;

wherein $R_2$ is

(a) hydrogen,
(b) ($C_1$-$C_3$)alkyl,
(c) phenyl, or
(d) -$CO_2R_1$;

wherein $R_3$ is

(a) hydrogen,
(b) ($C_1$-$C_3$)alkyl, or
(c) acyl;

wherein $R_4$ is

(a) hydrogen,
(b) ($C_1$-$C_4$)alkyl, or
(c) phenyl;

wherein $R_7$ is

(a) hydrogen,
(b) -$CH_2OH$,
(c) -$COOR_1$,
(d) -$CH_2N(R_4)_2$,
(e) -CN
(f) -$CON(R_4)_2$, or
(g) -C(O)-$R_4$;

wherein $R_9$ and $R_{12}$ are the same or different and are

(a) hydrogen,
(b) ($C_1$-$C_4$)alkyl
(c) fluoro,
(d) chloro,
(e) bromo,

(f) $-OCH_3$, or,

(g) when taken together and attached to contiguous carbon atoms, $-O-CH_2-O-$;

wherein D represents a single or a double bond; and

wherein m and n are the same or different and are the integers 0 to 4, inclusive; including, pharmacologically acceptable acid addition salts thereof; and

when D represents a single bond, an enantiomer or a racemic mixture of enantiomers thereof; and

(IV) A compound of the formula D-I wherein $Z_4$ is

(a) imidazolyl, or

(b) imidazolyl substituted by $(C_1-C_3)$alkyl;

wherein $X_3$ is

(a) $-(CH_2)_n-$,

(b) -C(OH)-, or

(c) -C(O)-;

wherein $R_1$ is hydrogen, a pharmacologically acceptable cation, $(C_1-C_{12})$ alkyl, $(C_3-C_{10})$ cycloalkyl, $(C_7-C_{12})$ aralkyl, phenyl, phenyl mono-, di-, or trisubstituted by chloro, $(C_1-C_3)$ or alkyl, or phenyl para-substituted by

(a) $-NHCO-R_{25}$,

(b) $-O-CO-R_{26}$,

(c) $-CO-R_{24}$,

(d) $-O-CO-(p-Ph)-R_{27}$, or

(e) $-CH=N-NH-CO-NH_2$,

wherein $R_{24}$ is phenyl or acetamidophenyl, $R_{25}$ is methyl, phenyl, acetamidophenyl, benzamidophenyl, or amino, $R_{26}$ is methyl, phenyl, amino or methoxy; and $R_{27}$ is hydrogen or acetamido, and wherein -(p-Ph) is 1,4-phenylene;

wherein $R_2$ is

(a) hydrogen,

(b) $(C_1-C_3)$alkyl,

(c) phenyl, or

(d) $-CO_2R_1$;

wherein $R_3$ is

(a) hydrogen,

(b) $(C_1-C_3)$alkyl, or

(c) acyl;

wherein $R_4$ is

(a) hydrogen,

(b) $(C_1-C_4)$alkyl, or

(c) phenyl;

wherein $R_7$ is

(a) hydrogen,

(b) $-CH_2OH$,

(c) $-COOR_1$,

(d) $-CH_2N(R_4)_2$,

(e) -CN

(f) $-C(ON(R_4)_2$, or

(g) $-C(O)-R_4$;

wherein $R_9$ and $R_{12}$ are the same or different and are

(a) hydrogen,

(b) $(C_1-C_4)$alkyl

(c) fluoro,

(d) chloro,

(e) bromo,

(f) $-OCH_3$, or,

(g) when taken together and attached to contiguous carbon atoms, $-O-CH_2-O-$;

wherein D represents a single or a double bond; and

wherein m and n are the same or different and are the integers 0 to 4, inclusive; including, pharmacologically acceptable acid addition salts thereof; and

when D represents a single bond, an enantiomer or a racemic mixture of enantiomers thereof.

The carbon atom content of various hydrocarbon-containing moieties is indicated by a prefix designating the minimum and maximum number of carbon atoms in the moiety, i.e., the prefix $(C_i-C_j)$ indicates a moiety of the integer "i" to the integer "j" carbon atoms, inclusive. Thus $(C_1-C_3)$alkyl refers to alkyl of one to 3 carbon atoms, inclusive, or methyl, ethyl, propyl, and isopropyl.

Examples of alkyl of one to 12 carbon atoms, inclusive, are methyl, ethyl, propyl, butyl, pentyl, hexyl, heptyl, octyl, nonyl, decyl, undecyl, dodecyl, and isomeric forms thereof.

Examples of cycloalkyl of 3 to 10 carbon atoms, inclusive, which includes alkyl-substituted cycloalkyl, are cyclopropyl, 2-methylcyclopropyl, 2,2-dimethylcyclopropyl, 2,3-diethylcyclopropyl, 2-butylcyclopropyl, cyclobutyl, 2-methylcyclobutyl, 3-propylcyclobutyl, 2,3,4-triethylcyclobutyl, cyclopentyl, 2,2-dimethylcyclopentyl, 2-pentylcyclopentyl, 3-tert-butylcyclopentyl, cyclohexyl, 4-tert-butylcyclohexyl, 3-isopropylcyclohexyl, 2,2-dimethylcyclohexyl, cycloheptyl, cyclooctyl, cyclononyl and cyclodecyl.

Examples of aralkyl of 7 to 12 carbon atoms, inclusive, are benzyl, 2-phenethyl, 1-phenylethyl, 2-phenylpropyl, 4-phenylbutyl, 3-phenylbutyl, 2-(1-naphthylethyl), and 1-(2-naphthylmethyl).

Examples of phenyl substituted by one to 3 chloro or alkyl of one to 3 carbon atoms, inclusive, are p-chlorophenyl, m-chlorophenyl, 2,4-dichlorophenyl, 2,4,6-trichlorophenyl, p-tolyl, m-tolyl, o-tolyl, p-ethylphenyl, 2,5-dimethylphenyl, 4-chloro-2-methylphenyl, and 2,4-dichloro-3-methylphenyl.

The compounds of the present invention may be in the form of pharmacologically acceptable salts. These salts are formed when $R_1$ is a pharmacologically acceptable cation. Such cations include: pharmacologically acceptable metal cations, ammonium, amine cations, or quaternary ammonium cations.

Especially preferred metal cations are those derived from the alkali metals, e.g., lithium, sodium, and potassium, and from the alkaline earth metals, e.g., magnesium and calcium, although cationic forms of other metals, e.g., aluminum, zinc, and iron are within the scope of this invention.

Pharmacologically acceptable amine cations are those derived from primary, secondary, or tertiary amines. Examples of suitable amines are methylamine, dimethylamine, trimethylamine, ethylamine, dibutylamine, triisopropylamine, N-methylhexylamine, decylamine, dodecylamine, allylamine, crotylamine, cyclopentylamine, dicyclohexylamine, benzylamine, dibenzylamine, α-phenylethylamine, β-phenylethylamine, ethylenediamine, diethylenetriamine, and the like aliphatic, cycloaliphatic, araliphatic amines containing up to and including about 18 carbon atoms, as well as heterocyclic amines, e.g., piperidine, morpholine, pyrrolidine, piperazine, and lower-alkyl derivatives thereof, e.g.,

1-methylpiperidine,
4-ethylmorpholine,
1-isopropylpyrrolidine,
2-methylpyrrolidine,

1,4-dimethylpiperazine,
2-methylpiperidine,

and the like, as well as amines containing water-solubilizing or hydrophilic groups, e.g,

mono-, di-, and triethanolamine,
ethyldiethanolamine,
N-butylethanolamine,
2-amino-1-butanol,
2-amino-2-ethyl-1,3-propanediol,
2-amino-2-methyl-1-propanol,
tris(hydroxymethyl)aminomethane,
N-phenylethanolamine,
N-(p-tert-amylphenyl)diethanolamine,
glactamine,
N-methylglycamine,
N-methylglucosamine,
ephedrine,
phenylephrine,
epinephrine,
procaine,

and the like. Further useful amine salts are the basic amino acid salts, e.g.,

lysine and
arginine.

Examples of suitable pharmacologically acceptable quaternary ammonium cations are

tetramethylammonium,
tetraethylammonium,
benzyltrimethylammonium,
phenyltriethylammonium, and the like.

Pharmaceutically acceptable acid addition salts are formed at the heterocyclic amine moiety and are also useful for administering the compounds of this invention. These salts include hydrochloride, hydrobromide, hydroiodide, sulfate, phosphate, acetate, propionate, lactate, maleate, malate, succinate, tartrate, and the like. They are prepared by methods well known in the art, e.g., as depicted in Example 8.

The compounds of the present invention will be named herein as

benzofurans, using the Chemical Abstracts numbering system (see Naming and Indexing of Chemical Substances for Chemical Abstracts during the Ninth Collective Period (1972-1976), a reprint of section IV from the Volume 76 Index Guide.)

The compounds of the present invention were tested for $TXA_2$ inhibition as follows:

Rabbit aortic strips were superfused in series with Krebs solution. Thromboxane $A_2$ ($TXA_2$) was generated by mixing prostaglandin $H_2$ ($PGH_2$) with human platelet microsomes (HPM).

Potential inhibitors were tested by comparing the response of the rabbit aorta to the amount of $TXA_2$ produced by mixing $PGH_2$ and HPM without the test compound in the reaction medium and then the amount of $TXA_2$ produced when the test compound was added to the HPM 5 minutes before the HPM was mixed with $PGH_2$. By this means compounds which selectively inhibit $TXA_2$ synthetase are found. For a discussion of $TXA_2$ synthetase inhibition testing see, e.g., R. Gorman, supra.

Using this test system, two compounds, ethyl 5-(3'-pyridinylmethyl)benzofuran-2-carboxylic acid, sodium salt (Example A-2), and 5-(3-pyridinylmethyl)benzo[b]thiophene-2-carboxylic acid (Example B-7), have been shown to be the most effective in inhibiting $TXA_2$ formation. The former compound has an approximate $ED_{50}$ in this system of 10 ng/ml, and the latter has an approximate $ED_{50}$ of 1 ng/ml.

The novel compounds of this invention have thus been shown to be highly active as selective inhibitors of the thromboxane synthetase enzyme system. Accordingly, these novel compounds are useful for administration to mammals, including humans, whenever it is desirable medically to inhibit this enzyme system. For a discussion of the utility of $TXA_2$ inhibitors, see, e.g., Derwent Farmdoc Nos. 18399B; 72896B; 72897B; 63409B; 03755C; 03768C; and 50111C.

Thus, for example, these novel compounds are useful as antiinflammatory agents in mammals and especially humans, and for this purpose, are administered systemically and preferably orally. For oral administration, a dose range of 0.05 to 50 mg per kg of human body weight is used to give relief from pain associated with inflammatory disorders such as rheumatoid arthritis. They are also administered intravenously in aggravated cases of inflammation, preferably in a dose range 0.01 to 100 μg per kg per minute until relief from pain is attained. When used for these purposes, these

novel compounds cause fewer and lesser undesirable side effects than do the known synthetase inhibitors used to treat inflammation, for example, aspirin and indomethacin. When these novel compounds are administered orally, they are formulated as tablets, capsules, or as liquid preparations, with the usual pharmaceutical carriers, binders, and the like. For intravenous use, sterile isotonic solutions are preferred.

These compounds are useful whenever it is desired to inhibit platelet aggregation, reduce the adhesive character of platelets, and remove or prevent the formation of thrombi in mammals, including man, rabbits, dogs, and rats. For example, these compounds are useful in the treatment and prevention of myocardial infarcts, to treat and prevent post-operative thrombosis, to promote patency of vascular grafts following surgery, and to treat conditions such as atherosclerosis, arteriosclerosis, blood clotting defects due to lipemia, and other clinical conditions in which the underlying etiology is associated with lipid imbalance or hyperlipidemia. For these purposes, these compounds are administered systemically, e.g., intravenously, subcutaneously, intramuscularly, and in the form of sterile implants for prolonged action. For rapid response especially in emergency situations, the intravenous route of administration is preferred. Doses in the range about 0.005 to about 20 mg per kg of body weight per day are used, the exact dose depending on the age, weight, and condition of the patient or animal, and on the frequency and route of administration.

These compounds are further useful as additives to blood, blood products, blood substitutes, or other fluids which are used in artificial extracorporeal circulation or perfusion of isolated body portions, e.g., limbs and organs, whether attached to the original body, detached and being preserved or prepared for transplant, or attached to a new body. During these circulations and perfusions, aggregated platelets tend to block the blood vessels and portions of the circulation apparatus. This blocking is avoided by the presence of these compounds. For this purpose, the compound is added gradually or in single or multiple portions to the circulating blood, to the blood of the donor animal, to the perfused body portion, attached or detached, to the recipient, or to two or all of these at a total steady state dose of about 0.001 to 10 mg per liter of circulating

fluid. It is especially useful to use these compounds in laboratory animals, e.g., cats, dogs, rabbits, monkeys, and rats, for these purposes in order to develop new methods and techniques for organ and limb transplants.

The compounds of the present invention are useful in mammals, including humans and certain useful animals, e.g., dogs and pigs, to reduce or avoid gastrointestinal ulcer formation, and accelerate the healing of such ulcers already present in the gastrointestinal tract. For this purpose, these compounds are injected or infused intravenously, subcutaneously, or intramuscularly in an infusion dose range about 0.1 μg to about 500 μg/kg of body weight per minute, or in a total daily dose by injection or infusion in the range about 0.1 to about 20 mg/kg of body weight per day, the exact dose depending on the age, weight, and condition of the patient or animal, and on the frequency and route of administration.

The novel compounds are used for the purposes described above in the free acid form, in ester form, and in the pharmacologically acceptable salt form. When the ester form is used, the ester is any of those within the above definition of $R_1$. However, it is preferred that the ester be alkyl of one to 12 carbon atoms, inclusive. Of the alkyl esters, methyl and ethyl are especially preferred for optimum absorption of the compound by the body or experimental animal system; and straight-chain octyl, nonyl, decyl, undecyl, and dodecyl are especially preferred for prolonged activity in the body or experimental animal.

Thromboxane synthetase converts $PGH_2$ (prostaglandin endoperoxide) into $TXA_2$. $PGH_2$ is also converted to prostacyclin, $PGD_2$, and other compounds by other enzymes. Thus, because the compounds of this invention inhibit thromboxane $A_2$ synthetase, they increase the $PGH_2$ substrate and thus increase the amount of endogenous prostacyclin. Therefore, they are also useful for many of the pharmacological purposes for which prostacyclin is employed.

Prostacyclin and a thromboxane synthetase inhibitor have both been shown to be effective in controlling tumor cell metastasis, see, e.g., K. Honn, et al., "Thromboxane Synthetase Inhibitors and Prostacyclin Can Control Tumor Cell Metastasis," an Abstract of the Twentieth Annual Meeting of the American Society for Cell Biology, in the Journal of Cell Biology, 87:64 (1980).

Similarly, prostacyclin has been shown to be an effective antihypertensive agent. The compounds of the present invention are also used for this purpose. (See, e.g., British patent specification 2,039,903A).

For a general discussion of the utility of $TXA_2$ synthetase inhibitors which increase endogenous prostacyclin, see, Aiken, et al. J. Pharmacol. Exp. Ther., 219:299 (1981).

The compounds of the present invention are prepared by the methods depicted in Chart A-R.

Thus, the compounds of the present invention wherein m is zero and $R_2$ is hydrogen are prepared by the method of Chart A. In Chart A, $R_{10}$ is all substituents within the scope of $R_1$ excluding the pharmacologically acceptable cations. All other variables in Chart A are defined as above. A hydroxybenzaldehyde of the Formula X is cyclized into the compounds of the present invention by methods known in the art. See, e.g., S.Tanaka, J. Am. Chem. Soc., 73:872 (1951). Thus, the compound may be reacted with diethyl bromomalonate in the presence of potassium carbonate to yield the desired benzofuran-2-carboxylic acid ester. See, e.g., D. T. Witiak, et al., J. Med. Chem. 21:833 (1978). Higher yields are obtained when the reaction conditions are changed so that the compound is reacted in the presence of sodium hydride in toluene (solubilized with dicyclohexyl-18-crown-6). Both of these procedures are set out more fully in Example 1. Conversion of the ester of the Formula XV to the desired pharmacologically acceptable salts or free acid is accomplished by known methods.

The compounds of the Formula X are well known and readily available compounds, and may be prepared from known benzylpyridines of the Formula XX as depicted in Chart B. (See, also, British patent application 2,039,903A.)

Referring to Chart B, a compound of the Formula XX, wherein all variables are defined as above, is nitrated by methods well known in the art, for example, treatment with nitric acid. (While the para nitro compound is the predominant product, the meta and ortho nitro compounds are also formed in smaller quantities. The desired isomer is separated by known methods.) The nitro function is easily reduced by treatment with hydrogen over a 5% palladium-on-carbon catalyst, to form a Formula XXII amine. This amino group is replaced by a hydroxyl

moiety via diazotization followed by decomposition of the diazonium salt in hot aqueous acid. Formylation of this phenol to obtain the hydroxybenzaldehyde of the Formula XXIV is accomplished by modification of the Duff reaction (see, J. Duff, J. Chem. Soc. 547 (1941)), by the use of hexamethyltetramine in trifluoracetic acid, see, W.E. Smith, J. Org. Chem., 37:3972 (1972).

Compounds of the Formula XX when $X_1$ is $-CH_2N-$ may not be readily available. However, compounds of the Formula XXIII when $X_1$ is $-CH_2N-$ are disclosed in G. Walker, et al., J. Med. Chem. 9:624 (1966). Formula XX compounds when $X_1$ is -O- are disclosed in F. Villani, et al., J. Med. Chem. 18:1 (1975). Formula XX compounds when $X_1$ is $(CH_2)_n$ and n is zero are disclosed in A. Katritzky, Chem. Soc. (London) p. 1511 (1960). Formula XX compounds when $X_1$ is $-CH_2O-$ are disclosed in British patent 1,203,149.

Compounds of the Formula XXIII when $X_1$ is $-(CH_2)_n-$ and n is 2, 3, or 4 are prepared according to Chart G. A hydroxybenzaldehyde of the Formula LXXI (wherein P is 0, 1, or 2) is reacted with an appropriate pyridinylalkyltriphenylphosphonium chloride of the Formula LXXII in the presence of n-butyllithium to yield the unsaturated hydroxyphenylalkylpyridine of the Formula LXXIII. Catalytic reduction of the olefinic bonds yields the alkylene bridged compounds of the formula LXXIV. Related compounds of the Formula XX wherein m is three are disclosed in F. Villani, et al., J. of Pharm. Sciences, 60:1586-1587 (1971). Formula XX compounds wherein m is four are disclosed in B. Baker, et al., J. Med. Chem. 14:793-799 (1971).

Compounds of the present invention wherein $R_2$ is other than hydrogen are prepared by the method depicted in Chart C. Referring to Chart C, a solution of a hydroxybenzyl pyridine of the Formula XXX in a solvent such as methylene chloride and an acid chloride of the Formula $R_{20}COCl$, wherein $R_{20}$ is $C_1$-$C_3$ alkyl or phenyl, is cooled in an ice bath. Anhydrous aluminum chloride is added and the mixture is stirred at room temperature for 20 hr to yield a compound of the Formula XXXI, which is worked up successively with a base such as sodium hydroxide, followed by an neutralization with acid to yield the acylhydroxybenzyl pyridine of the Formula XXXII. The corresponding benzofuran of the Formula XXXIII is prepared as described in Chart A.

For compounds wherein m is one, the method of Chart D is used. An ester of the Formula XL is reduced with lithium aluminum hydride in

ether or tetrahydrofuran to yield the corresponding alcohol after workup. This alcohol is tosylated or mesylated using p-toluenesulfonyl chloride or methanesulfonyl chloride in pyridine to yield the Formula XLII product. (Ts indicates the tosylated compound, but the compound could also be mesylated). This compound is treated with excess sodium cyanide in dimethylformamide (DMF) and stirred under nitrogen at room temperature for 5 hr to yield the Formula XLIII cyano compound. This compound is dissolved in ethanol and treated with 25% aqueous potassium hydroxide to yield the corresponding acid. This compound is esterified by means well known in the art, e.g., treatment with diazomethane in methanol for the methyl ester. Pharmacologically acceptable salts are also prepared by means well known in the art.

Chart E depicts the synthesis of compounds of the present invention wherein m is 2, 3, or 4. In Chart E, q is zero, one, or 2. An ester of the Formula L is reduced with diisobutylaluminum hydride (DIBAL) in toluene or methylene chloride at low temperature to yield, after workup, the Formula LI aldehyde. Reaction of this aldehyde with an alkoxy alkylene-triphenylphosphorane of the formula $Ph_3P{=}CHCH_2$-$(CH_2)_qCOOR_{10}$ (wherein Ph is phenyl) yields the unsaturated ester of the Formula LII after workup. Careful reduction of this unsaturated ester by reaction with one equivalent of hydrogen over palladium-on-carbon in alcohol yields the saturated ester of the Formula LIII. The free acid or a pharmacologically acceptable salt of this ester is prepared by means well known in the art. The corresponding amides, phenacyl esters, and the like are prepared by the methods depicted in e.g., U.S. patents 4,292,445 and 4,172,206.

Example A-11 also depicts the preparation of amides. The dihydrobenzofurans are prepared as depicted in Chart F. A solution of a formula LX benzofuran in water is stirred with excess sodium amalgam (NaHg) for 24 hr. After workup there is obtained the corresponding Formula LXI dihydrobenzofuran. (See, e.g., D.T. Witiak, et al., J. Med. Chem. 14, 754 (1971).)

Reduction of the corresponding acid or ester of the formula $COOR_{10}$ with lithium aluminum hydride as depicted in Chart D, (XL to XLI) is used to prepare all of the corresponding alcohols within the scope of Formula I. This is seen in Example 7. Conversion of the alcohol to a corresponding acid addition salt is accomplished by known means as seen in Example 8.

The compounds of this invention wherein m is zero and $R_7$ is hydrogen are prepared by the method of Chart H, which is described more fully in Preparation A-25 and Example A-26. A formula LXXV aldehyde is reacted with an appropriate Wittig reagent (prepared by reacting sodium hydride and dimethylsulfoxide with an alkoxyalkyltriphenyl phosphonium halide) to yield the formula LXXVI enol ether. This compound is treated with perchloric acid to yield the formula LXXVII benzofuran.

Compounds of this invention wherein $X_1$ is -O- or -S- are prepared by the method of Chart I. In Chart I, $X_5$ is -S- or -O-, and is attached at the 3 or 4 position of the pyridine ring. This procedure is described more fully in Preparations A-30 through A-34 and Examples A-35-38. A compound of the formula LXXX (e.g., 3-hydroxypyridine) is treated with potassium hydroxide, and reacted with a compound of the formula LXXXI (e.g., p-bromoanisole) in the presence of activated copper bronze to yield the formula LXXXII product. The formula LXXXII compound is then reacted with hydrogen bromide to yield the formula LXXXIII product, which is then transformed to the corresponding benzofuran final products by the methods of Charts A, B, C, D, E, F, and H. The LXXXIII compounds wherein $X_5$ is -S- are similarly prepared by reaction of a 3 or 4-bromopyridine (LXXX') with p-methoxythiophenol (LXXXI) to yield the formula LXXXII compound.

The benzothiophenes of the present invention are prepared by the method of Chart J, as described more fully in Preparations B-1, B-2, B-3, B-4 and B-5, and Examples B-5, B-7, and B-8. Conversion of the formula LXXXV thiophenol to the formula LXXXVII benzothiophene using known methods as described, e.g., in Y. Matsuki, et al., Nippon Kugaku Zasshi 87:186 (1966) and Chapman, et al., J. Chem. Soc. 514 (1968). The formula LXXXVII benzothiophene thus prepared is reacted with an appropriate 3- or 4- lithiopyridine compound (prepared by reacting the corresponding 3- or 4-bromopyridine compound with n-butyllithium) to yield the corresponding formula LXXVIII compound wherein $X_1$ is -CH(OH)-. Reaction with thionyl chloride yields the formula LXXXIX compound. The chloro group is removed by treatment of this compound with zinc dust in the presence of acid (e.g., propionic acid), to yield the formula XC compound, which is carboxylated by known means (e.g., pouring a solution of the XC compound in an inert solvent in the presence of n-butyllithium over crushed dry ice), to yield the

formula XCI compound, which is converted to other compounds of this invention by the methods of the previous charts. Alternatively, as set forth in the Preparations and Examples described below, the formula LXXXVII compound is converted to the corresponding bromomethyl compound by known means (see, e.g., Matsuki, supra). This XCII compound is converted to the corresponding aldehyde of the formula XCIII, which is then reacted with 3-lithiopyridine (as described in Example B-5) to yield the formula LXXXVIII compound which is then reacted as described above.

Chart K depicts a method for preparing the imidazolyl compounds of this invention. An ethyl-benzofuran-2-carboxylate of the formula XCVI is prepared from the corresponding XCV aldehyde by the methods described above (and set forth more fully in Preparation C-1). This compound is alkyl chlorinated by treatment with paraformaldehyde and zinc chloride to yield the formula XCVII compound. This compound is formylated by known methods, (e.g., reaction of sodium metal with ethanol followed by the addition of the XCVII compound) to yield the formula XCVIII compound. Alternatively, the XCVII compound is reacted with imidazole or an alkyl substituted imidazole to yield the formula XCIX product. As an alternative, the formula XCVIII compound may be reacted with 3-amino pyridine to yield the compound of the formula C wherein $X_1$ is -NH-. This is described in Example A-31. Still another alternative is the reaction of the formula XCVIII compound with 3-lithiopyridine (prepared, e.g., by reaction of 3-bromopyridine with n-butyllithium) to yield the formula CI compound wherein $X_1$ is -C(OH)-. (Preparation A-13 describes a similar method using 3-methoxy-4-benzyloxybenzaldehyde.) The formula XCVII compound is also treated with a mixture of sodium hydride, dimethylformamide, and 3-hydroxypyridine to obtain the formula CII compound wherein $X_1$ is -O-, as described more fully in Example C-13.

Chart L depicts a method for preparing chloro-pyridinyl compounds of this invention. This method is described more fully in Preparation A-11 and Example A-13. The CV pyridinyl derivative is treated with m-chloroperbenzoic acid to yield the corresponding CVI N-oxide. The N-oxide is treated with phosphorous oxychloride to yield the corresponding chloropyridyl isomers of the formula CVII.

Substituted benzofurans and benzothiophenes (i.e. compounds wherein $R_9$ and $R_{12}$ are other than hydrogen) are prepared by the

methods depicted in Charts M, N, O, P, and Q.

Chart M depicts a method for preparing brominated derivatives. An aldehyde of the formula CX (prepared by the method of Chart B, see formula XXIV) is treated with bromine to yield the corresponding brominated compound of the formula CXI, which is then converted to the compounds of the present invention by the method of Chart A. This is more fully described in Preparation A-15 and Example A-21.

Chart N depicts a method for preparing methyl or methoxy substituted benzofurans or benzothiophenes. In Chart N, $R_{19}$ is methyl or methoxy. The formula CXV ether is hydrolyzed (using hydrobromic acid for example) to yield the formula CXVI alcohol. Similarly, the formula CXV' ether is hydrogenolyzed with hydrogen over palladium on carbon catalyst to yield the formula CXVI alcohol. This alcohol is treated with trifluoroacetic acid in the presence of hexamethylenetetramine to yield the formula CXVII aldehyde, which is converted to the compounds of this invention by the method of Chart A.

Chart O depicts a general method for preparing the substituted benzothiophenes by the method of Chart J. The $R_9$ and $R_{12}$ substituted para-methylthiophenol starting materials of the formula CXXXI are thus by conversion of an appropriate formula CXXX substituted phenol into a thiophenol using the well established Newman-Kwart rearrangement (see, e.g., Org. Syn., 51:139 (1971)) and other related procedures. See also the Schönberg rearrangement by J.L. Wardell in "The Chemistry of the Thiol Group", ed. S. Patai, Wiley, New York, 1974, p. 163 and the Kawata-Harano-Taguchi rearrangement, Chem. Pharm. Bull. (Japan), 21, 604 (1973). The para-methylphenols are either commercially available or can be prepared by methods known in the art.

Similarly, various substituted hydroxy benzaldehydes are available commercially or may be prepared by methods known in the art. The hydroxybenzaldehydes are thus converted to the claimed benzofurans by the method of Chart A.

Chart P depicts a method for preparing compounds wherein $X_1$ is -C(O)-, as described more fully in Example A-12. A compound of the formula CXX is treated with potassium superoxide to yield the formula CXXI compound.

Chart Q depicts a method for preparing compounds wherein $R_2$ is $-COOR_1$. The formula CL compound of Preparation A-10 (XXIII of Chart B) is converted by the method of D.J. Zwanenburg and W.A.P. Reynen,

Synthesis, 624 (1976) to the formula CLI 5-(3-pyridylmethyl)-2,3-dioxo-2,3-dihydrobenzofuran and thence, by the method of V. Titoff, H. Müller, and T. Richstein, Helv. Chim. Acta, 20:883 (1937) to 5-(3-pyridylmethyl)-2,3-benzofurandicarboxylic acid (formula CLIII) and 5-(3-pyridylmethyl)-3-benzofurancarboxylic acid (CLIV).

Hydrogenolysis of compounds wherein $X_1$ is -C(OH)- is depicted in Chart R. A compound of the formula CLV is hydrogenated using, e.g., a Parr apparatus and a palladium-on-carbon catalyst. This is depicted in Preparations A-13, A-19, and A-23.

Compounds where $Z_1$ is 4-methylpyridine are prepared by converting the corresponding 4-chloropyridine of Chart L with methyl magnesium halides to the 4-methyl pyridine derivative according to the procedure described in K. Thomas and D. Jerchel, in "Newer Methods of Organic Chemistry," Vol. III., W. Foerst, ed., Academic Press, N.Y. 1964, pp 74-75.

The 4-methoxy, 4-amino, and 4-N,N-demethylamino derivatives are prepared from the corresponding 4-methoxy-3-bromopyridine (see T. Talik, Roczniki Chem, 36:1465 (1962)), 3-bromo-4-aminopyridine (see T. Talik, Roczniki Chem., 37:69 (1963)) and 3-bromo-4-dimethylaminopyridine (see J.M. Essery and K. Schofield, J. Chem. Soc., 4953 (1960)), respectively, using the procedure of Chart K (conversion of XCVIII to CI).

Preparation of various other benzofuran and benzothiophene derivatives within the scope of this invention are prepared by analogous procedures well known in the art. In general, the procedures used for benzofurans are useful for benzothiophenes and vice-versa.

Certain compounds of the present invention are preferred. Thus, compounds of the formula F-I, wherein D denotes a double bond, $X_1$ is $-(CH_2)_n-$ (wherein n is zero or one, more preferably one), $Z_1$ is 3-pyridinyl, m is zero, $R_7$ is $COOR_1$, $R_1$ is Na or H and $R_2$, $R_9$ and $R_{12}$ are hydrogen are preferred. Compounds having all these preferences are more preferred. Thus, 5-(3'-pyridinylmethyl)benzofuran-2-carboxylate and 5-(3-pyridinylmethyl)-benzo[b]thiophene-2-carboxylic acid are preferred compounds of this invention. Sodium 5-(3'-pyridinylmethyl)benzofuran-2-carboxylate is the most preferred compound of this invention.

DESCRIPTION OF THE PREFERRED EMBODIMENTS

The present invention is seen more fully by the Examples given below.

PREPARATION OF CLASS "A" COMPOUNDS"

Preparation A-1 3-(3'-Formyl-4'-hydroxybenzyl)pyridine

Refer to Chart B. (Conversion of XXIII to XXIV).

A solution of 3-(4'-hydroxybenzyl)pyridine (2.038 g., 0.011 mole) and hexamethylenetetramine (1.61 g., 0.0115 mole) in trifluoroacetic acid (20 ml) is stirred and heated in an 80° oil bath for 4 hrs. Excess trifluoroacetic acid is removed under reduced pressure. Water (35 ml) is added to the residue and the resulting solution is allowed to stand for 20 min. at room temperature. The solution is adjusted to pH ~ 7 by the addition of solid sodium bicarbonate. A gummy oil precipitates. The mixture is extracted with ethyl acetate (3x), the extract solution is dried over $MgSO_4$, filtered, and concentrated under reduced pressure. The crude product is chromatographed over silica gel (130 g) packed as a slurry in 1:1 acetone-hexane. Elution is with the same solvent and fractions of 150 ml volume are collected. The titled product (1.263 g, 0.00593 mole, 54% yield) is obtained in fractions 5-7 as a colorless, crystalline solid. Recrystallizationn from acetone-hexane gives colorless crystals, mp 129-132° C. A second recrystallization gives 3-(3'-formyl-4'-hydroxybenzyl)pyridine, mp 130-132° C; NMR ($CDCl_3$) peaks are observed at δ 9.89, 8.55, 6.70-7.66, and 3.98. The IR spectrum (mull) reveals peaks at 2560. 1670, 1610, 1595, 1580, 1505, and 1480 $cm^{-1}$.

The Carbon:Hydrogen:Nitrogen (C:H:N) ratio is 73.54:5.27:6.19.

Preparation A-2 (E,Z)-1-(4-Hydroxyphenyl)-2-(3-pyridinyl)-ethylene

Refer to Chart G (Conversion of Formulas LXXI and LXXII to Formula LXXIII).

A flask under a dry nitrogen atmosphere is charged with 170 ml of dry tetrahydrofuran and 80 ml (0.128 mol) of 1.6 M n-butyllithium in hexane. The flask is cooled in an ice water bath and 3-pyridinylmethyltriphenylphosphonium chloride hydrochloride (17.04 g, 0.04 mol) is added in portions as a solid. A very dark redish-brown slurry results. The ice bath is removed and the mixture is stirred at room temperature for 30 min A solution of 4-hydroxybenzaldehyde (4.0 g, 0.033 mol) in tetrahydrofuran (4.0 g, 0.033 mol) is added dropwise over a 15 min period. The reaction is stirred overnight at room temperature.

The reaction mixture is poured into cold 2N HCl solution in ether (pH ~ 2). The mixture is shaken and then the layers are separated. The aqueous layer is extracted one more time with ether. The aqueous layer is then made basic with saturated $NaHCO_3$ solution and extracted 4 times with ethyl acetate. The pooled ethyl acetate layers are washed with water, dried over $Na_2SO_4$, filtered, and evaporated to give a red oil. The oil is chromatographed on 500 g of silica gel. Elution is with 50% ethyl acetate-hexane. 1.50 g of the titled product is obtained as an oil.

The NMR ($CDCl_3$, δ) peaks observed are 8.53, 8.40,, 7.78, and 7.01.

Preparation A-3 1-(4-Hydroxyphenyl)-2-(3-pyridinyl)ethane

Refer to Chart G. (Conversion of Formula LXXIII to LXXIV).

The product of Preparation A-2 (1.96, 9.9 mmol) is reduced by adding the compound to 100 ml of methanol solution and shaking this mixture with 5% Pd on carbon and hydrogen in a Parr apparatus. The catalyst is removed by filtration through sintered glass. 613 mg of the titled product are obtained. The solid is recrystallized from acetone-hexane twice to give 376 mg of crystals with a melting point of 180-181.5° C.

High resolution mass spectrum reveals the following

Calcd for $C_{13}H_{13}NO$: 199.0997

Found: 199.0996.

IR analysis (nujol) shows peaks observed at 2749, 2684, 2614, 1611, 1593, 1579, 1515, 824, and 803 $cm^{-1}$.

Preparation A-4 2-Hydroxy-3-[2-(3'-pyridinyl)ethyl]benzaldehyde

Refer to Chart B. (Conversion of Formula XXIII to XXIV).

Using the method described previously, the phenol of Preparation A-3 (757 mg, 3.80 mmol) is reacted with hexamethylenetetramine (546 mg, 3.9 mmol) to form the corresponding aldehyde. A total of 454 mg (53% of theory) of the titled product is obtained after chromatography. This solid is recrystallized from ethyl-pentane to give 370 mg of crystals with a melting point of 63-64.5° C. The C:H:N ratio is: 73.71:5.92:5.88.

High resolution mass spectrum reveals the following:

Calcd. for $C_{14}H_{13}NO_2$: 227.0946

Found: 227.0941.

Other peaks are observed at m/e 199, 135, and 93.

The IR (nujol) spectrum reveals peaks at 2765, 2677, 2603, 2502, 1676, 1612, 1597, 1581, 1502, 1482, 1277, 1246, 908, 826, 801, and 705 $cm^{-1}$.

The NMR (δ, $CDCl_3$) peaks observed are 9.82, 8.41, 7.10, and 2.92.

Preparation A-5 (E,Z)-1-(3-Hydroxyphenyl)-2-(3-pyridinyl)ethylene

Refer to Chart G. (Conversion of Formulas LXXI and LXXII to Formula LXXIII).

A solution of n-butyllithium in hexane (1.6 M) (35.5 ml, 60 mmol) is added to 150 ml of benzene in a flask under a dry nitrogen atmosphere. The flask is cooled with an ice-water bath and then 3-pyridinylmethyltriphenylphosphonium chloride hydrochloride (8.52 g 0.020 mol) is added as a solid in several portions. The ice-water bath is removed and the mixture is stirred at room temperature for one hour. 3-Hydroxybenzaldehyde (2.0 g, 0.016 mol) is dissolved in benzene (80 ml, warming is required to achieve solution) and added to the ylid dropwise. The reaction is stirred overnight at room temperature. The reaction is heated at 50° C for 7.5 hrs. After cooling the contents of the flask are poured into a separatory funnel containing 2 N HCl and ice. The pH is made basic (pH approximately 8) with saturated sodium bicarbonate solution. After shaking, the layers are separated. The aqueous layer is then extracted well with ether (4x). All organic layers are combined and washed with water, dried ($Na_2SO_4$), filtered and evaporated to give 8.5 g of a dark red oil. The oil is chromatographed on 400 g of silica gel packed as a slurry with 25% acetone-hexane followed with 35% acetone hexane. The mixture was eluted in 350 ml fractions. Fractions 31 to 36 yield the minor isomer (0.41 g). The NMR ($CDCl_3$, δ) showed peaks observed at 8.34 and 8.17.

Fraction 37 yielded a total of 0.59 of mixed products. Fractions 38 to 40 yielded the major isomer (1.27 g).

The NMR ($D_2O$-acetone, δ) peaks observed are 8.80 and 8.48.

Preparation A-6 1-(3-Hydroxyphenyl)-2-(3-pyridinyl)ethane

Refer to Chart G. (Conversion of Formula LXXII to LXXIV).

The major isomer obtained from the Wittig reaction of Preparation A-5, (1.27 g, 6.4 mmol) is dissolved in absolute ethanol (50 ml) followed by the addition of 10% palladium/carbon (120 mg). The mixture is hydrogenated at atmospheric pressure for 3 hrs (a total of 175 ml of $H_2$ is consumed). The reaction mixture is filtered and the

solvent is removed at reduced pressure. The crude product is chromatographed on two Merck size B columns with 500 ml of 50% ethyl acetate-hexane followed by 75% ethylacetate-hexane. 0.885 g of the titled product are obtained. The solid is recrystallized from acetone-hexane to give 699 mg of white crystals having a melting point of 107.5-108.5° C.

The C:H:N ratio is 78.55:6.57:6.66.

High resolution mass spectroscopy reveals the following:

Calcd. for $C_{13}H_{13}NO$: 199.0997

Found: 199.0988

Other peaks are observed at m/e 107, 92, 77, and 65.

The IR spectrum (nujol) reveals peaks at 3060, 2720, 2620, 1620, 1596, 1580, 1525, 1505, 1485, 1285, 1250, 1160, 875, 810, 785, 710, and 700 $cm^{-1}$.

The NMR ($CDCl_3$, δ) spectrum reveals peaks at 8.40, 6.53-7.67, and 2.85.

The minor isomer (0.41 g, 2.1 mmol) is reduced in a similar manner to give, after chromatography, 134 mg of a solid. The solid is recrystallized from acetone-hexane to give white crystals (63 mg) MP 107-108° C. NMR and IR (mineral oil mull) spectra are identical to those of the product obtained from the reduction of the major isomer.

Preparation A-7 2-Hydroxy-4-[2-(3'-pyridinyl)ethyl]benzaldehyde

Refer to Chart B. (Conversion of Formula XXIII to XXIV).

1.69 g (8.49 mmol) of the phenol prepared in Preparation A-6 in a flask with hexamethylenetetramine (1.26 g, 9 mmol) is dissolved in trifluoroacetic acid (10 ml) and heated in an 80° C oil. TLC analysis after 45 min reveals that no starting material remains. The reaction is cooled to room temperature and the trifluoroacetic acid is removed under reduced pressure. The residue is stirred for 20 min with 15 ml of water. The mixture is made slightly basic with saturated $NaHCO_3$ and solid $NaHCO_3$ and extracted well with ethyl acetate (5x). The ethyl acetate layers are pooled, washed twice with water, dried ($Na_2SO_4$), filtered and evaporated to give 2.00 g of a yellow oil. The oil is chromatographed on 130 g of silica gel packed as a slurry with 50% acetone-hexane. Elution is with 50% acetone-hexane followed by 75% acetone-hexane with 1% triethylamine added. 0.170 g of the titled product are obtained.

The solid is recrystallized twice from ether-pentane to yield 91

mg of white crystals with a melting point of 93-94° C.

Anal. Calcd. for $C_{14}H_{13}NO_2$: C, 73.99; H, 5.77; N, 6.16
Found: C, 73.57; H, 5.81; N, 5.28
Repeat: C, 73.34; H, 5.84; N, 6.94

High resolution mass spectroscopy reveals the following:
Calcd. for $C_{14}H_{13}NO_2$: 227.0946
Found: 227.0950

Other peaks at m/e 199, 135, 107, and 92.

The NMR ($CDCl_3$, δ) peaks observed are 9.85, 8.45, 7.66-6.68, and 2.94.

Preparation A-8 3-(4'-Nitrobenzyl)pyridine

Refer to Chart B (conversion of Formula XX to XXI).

3-Benzylpyridine (13.52 g, 0.080 mole) and nitric acid (70%, 100 ml) are stirred at 50° C for 6 hr. The solution is poured into ice-water (1500 ml). The mixture is made alkaline by the careful addition of 50% aqueous sodium hydroxide and then extracted 4 times with 300 ml of ether. The extracts are washed with brine, dried ($MgSO_4$), filtered, and concentrated. The crude product is crystallized from acetone-hexane giving 5.67 g of 3-(4'-nitrobenzyl)pyridine. The filtrate is concentrated and the residue is chromatographed in two equal portions (4.66 g each) over two Merck size C Lobar columns. The columns are eluted with 50% ethyl acetate-hexane. A less polar product [1.83 g, 3-(2'-nitrobenzyl)pyridine], mixed fractions (1.67 g), and additional 3-(4'-nitrobenzyl)pyridine (2.55 g) after crystallization from acetone-hexane, total 7.92 g, 0.037 mole, 46%). Two recrystallizations from acetone-hexane gives the titled product as colorless needles, having a melting point of 87-88° C.

The C:H:N ratio is 67.33:4.77:13.23.

From the mixed fractions, 3-(3'-nitrobenzyl)-pyridine is also obtained.

Preparation A-9 3-(4'-Aminobenzyl)pyridine

Refer to Chart B (conversion of Formula XXI to XXII).

A solution of 3-(4'-nitrobenzyl)pyridine of Preparation A-8 (7.924 g, 0.0370 mole) in methanol (100 ml) is shaken with 5% Pd on carbon and hydrogen in a Parr apparatus. Hydrogen uptake is complete within 45 min. The catalyst is removed by filtration through sintered glass. The solvent is removed under reduced pressure. The crystalline residue is recrystallized from methylene chloride-hexane with a

first crop of 5.229 g; a second crop of 0.492 g; a third crop of 0.480 g; and a fourth crop of 0.137 g (total 6.338 g, 0.0347 mole, 94%). Recrystallization from $CH_2Cl_2$-hexane gives colorless crystals of the titled product with a melting point of 121-123° C; IR (nujol) peaks are observed at 3400, 3300, 3200, 1640, 1610, 1590, 1575, 1515, 1480, 845, 800, and 710 $cm^{-1}$.

The C:H:N ratio is 77.91:6.53:15.02.

Preparation A-10 3-(4'-Hydroxybenzyl)pyridine

Refer to Chart B (conversion of Formula XXII to XXIII).

A solution of sodium nitrite (0.350 g) in water (1.5 ml) is cooled in ice, and added, with stirring, to a cold (ice-bath) solution of 3-(4'-aminobenzyl)pyridine (Preparation A-10, 0.920 g, 0.0050 mole) in water (3.75 ml), concentrated sulfuric acid (2.5 ml), and ice (7 g). This solution in turn is added dropwise to a third solution of water (5 ml) and sulfuric acid (6.25 ml) that is maintained at 160° C in an oil bath. The resulting solution is kept at 160° C for 10 min and then cooled to room temperature. The pH of the solution is adjusted to 7.0 by addition of aqueous 50% sodium hydroxide. The resulting mixture is extracted 4 times with 25 ml of ether. The aqueous portion is further extracted continuously with ether for 40 hr. The ether extracts are washed with brine, dried over $MgSO_4$, filtered, and concentrated under reduced pressure. The crystalline residue is decolorized with activated charcoal and is recrystallized from acetone-hexane, giving a first crop (0.426 g) of the titled crystals, with a melting point of 182-185° C. A second crop (0.034 g) and third crop (0.050 g, total 0.510 g, 0.00276 mole, 55%) of titled crystals are also obtained. Recrystallization of the first crop from acetone-hexane gives the titled product as colorless crystals with a melting point of 184-186° C. IR (nujol) peaks are observed at 2900, 2800, 2720, 2680, 2600, 1615, 1590, 1580, 1515, 1485, 1280, 1250, 845, 800, 710, and 640 $cm^{-1}$.

The C:H:N ratio is 77.97:6.12:7.66.

Example A-1 Ethyl 5-(3'-pyridinylmethyl)benzofuran-2-carboxylate (Formula A-I, $Z_1$ is 3-pyridinyl, $X_1$ is $-CH_2-$ and is para to the oxygen, $R_2$, $R_9$, and $R_{12}$ are hydrogen, m is zero, D denotes a double bond , $R_7$ is $COOR_1$, and $R_1$ is ethyl)

Refer to Chart A.

Method 1

A mixture of Preparation A-1 (1.26 g, 0.00592 mole), ethyl bromomalonate (1.577 g, 0.00660 mole), and potassium carbonate (1.66 g, 0.012 mole) in methyl ethyl ketone is stirred at reflux temperature for 18 hrs. The reaction is worked up by removing the solvent under reduced pressure, water is added, and the aqueous mixture is extracted 4 times with ethyl acetate. The combined extracts are washed with brine, dried over $MgSO_4$, filtered, and concentrated. The crude product is chromatographed over one Merck size B Lobar silica gel column. Acetone-hexane (1:3) is used to develop the column ( a higher acetone-hexane ratio is used to dissolve the crude product for application to the column). Fractions of 25 ml volume were collected. Fractions 22-28 contain the titled product (0.464 g., 0.00165 mole, 28%), which crystallizes. Recrystallization from acetone-hexane gives a first crop of 0.367 g, having a melting point of 73-74° C. Another recrystallization gives an analytical sample of crystals with a melting point of 73-74° C, the NMR ($CDCl_3$, δ) peaks observed are 8.54, 7.67-7.00, 4.45, 4.10, and 1.41.

The C:H:N ratio is C, 72.52:5.47:4.71.

Method 2

A mixture of sodium hydride (1.25 g, 0.052 mole, 2.113 of a 59.6% dispersion of NaH in mineral oil, washed with hexane) in dry toluene (20 ml) is prepared. To this is added slowly a solution of aldehyde of Preparation A-1 (4.26, 0.020 mole) in toluene (300 ml). Then a solution of diethyl bromomalonate (5.26 g, 0.022 mole) in toluene (20 ml) is added dropwise. Dicyclohexyl-18-crown-6 (36 drops) is added and the resulting mixture is stirred at room temperature. After stirring 20 hours, a thin film of solid that has caked on the walls of the flask is carefully scraped loose with a spatula. Stirring at room temperature is continued as TLC examination reveals that the reaction is incomplete. After 48 hrs, TLC (1:1 acetone-hexane) shows the reaction is complete with the desired less polar product the only significant material in the reaction. The reaction solution is poured onto a mixture of ice (100 ml), saturated sodium bicarbonate solution (100 ml), and brine (200 ml). The mixture is extracted three times with ethyl acetate. The combined organic extracts are washed (2 x 200 ml) with brine, dried over $Na_2SO_4$, filtered, and evaporated to give 6.57 g of a yellow oil. The oil is chromatographed over a column of

silica gel (200 g) packed as a slurry in 30% acetone-hexane. The column is eluted with 30% acetone-hexane and fractions of about 200 ml volume are collected. The product is eluted in fractions 7-12 and after appropriate recrystallizations from acetone-hexane, a total of 4.62 g (0.0165 mole, 82% yield) of crystalline titled compound is obtained.

Example A-2 Sodium 5-(3'-pyridinylmethyl)benzofuran-2-carboxylate (Formula A-I, $Z_1$ is 3-pyridinyl, $X_1$ is $-CH_2-$ and is para to the oxygen, $R_2$, $R_9$ and $R_{12}$ are hydrogen, m is zero, D denotes a double bond, $R_7$ is $COOR_1$, and $R_1$ is a sodium cation)

A solution of ethyl 5-(3'-pyridinylmethyl)benzofuran-2-carboxylate (0.425 g, 0.00151 mole) in methanol (15 ml) and aqueous 0.10 N sodium hydroxide (15.1 ml, 0.00151 mole) is stirred at room temperature for 16 hrs. The solution is concentrated under reduced pressure giving a crystalline solid. Recrystallization from a small quantity of water and a large amount of acetone gives a first crop (0.370 g) and a second crop (0.011 g, total 0.381 g, 0.00138 mole, 92%) of the titled product as glistening colorless crystals, with a melting point greater than 300° C.

NMR ($D_2O$, δ) peaks are observed at 8.28, 7.53-6.90, and 3.62.

Example A-3 Ethyl 5-[2-(3'-pyridinyl)ethyl]benzofuran-2-carboxylate (Formula A-I, $Z_1$ is 3-pyridinyl, $X_1$ is $-CH_2-CH_2-$ and is para to the oxygen, $R_2$, $R_9$ and $R_{12}$ are hydrogen, m is zero, the D denotes a double bond, $R_7$ is $COOR_1$, and $R_1$ is ethyl)

Refer to Chart A.

Following the procedure of Example A-1, the aldehyde of Preparation A-4 (441 mg, 1.94 mmol), methylethylketone (15 ml), diethylbromomalonate (509 mg, 2.13 mmol) and anhydrous potassium carbonate (442 mg, 3.20 mmol) are reacted to obtain 109 mg (19% ofPreparation theory) of the titled product as a solid. The solid is recrystallized from ether-pentane to give 43 mg of a white solid 91.5-92.5° C.

High resolution mass spectroscopy reveals the following:

Calcd. for $C_{18}H_{17}NO_3$: 295.1208

Found: 295.1194

Other peaks are observed at m/e 250, 203, and 175.

The C:H:N ratio is 72.84:6.22:4.65.

The NMR ($CDCl_3$, δ) peaks observed are 8.38, 7.29, 4.39, and 1.41.

Example A-4 Sodium 5-[2-(3'-pyridinyl)ethyl]benzofuran-2-carboxylate (Formula A-I, $Z_1$ is 3-pyridinyl, $X_1$ is $-CH_2-CH_2-$ and is para to the oxygen, $R_2$, $R_9$ and $R_{12}$ are hydrogen, m is zero, the D denotes a double bond, $R_7$ is $COOR_1$, and $R_1$ is Na)

The ester prepared in Example A-3 (92 mg, 0.31 mmol) is hydrolyzed as described in Example A-2 to give a white powder. 90 mg of the titled product are obtained.

IR (nujol) peaks observed are at 1620, 1575, 1480, 935, and 790 $cm^{-1}$.

Example A-5 Ethyl 6-[2(3-pyridinyl)ethyl]-benzofuran-2-carboxylate (Formula A-I, $Z_1$ is 3-pyridinyl, $X_1$ is $-CH_2-CH_2-$ and is meta to the oxygen, $R_2$, $R_9$ and $R_{12}$ are hydrogen, m is zero, the D denotes a double bond, $R_7$ is $COOR_1$, and $R_1$ is ethyl)

A solution of the aldehyde of Preparation A-7 (155 mg, 0.68 mmol) is treated with anhydrous potassium carbonate (155 mg, 1.13 mmol) and diethyl bromomalonate (130 ml, 179 mg, 0.75 mmol). The mixture is heated at reflux temperature for 23 hrs. The reaction is then cooled to room temperature and poured into water and ethyl acetate. The mixture is shaken and the layers are separated. The aqueous layer is extracted four times with ethyl acetate. The pooled ethyl acetate layers are washed with water, dried over $Na_2SO_4$, filtered and evaporated to give 208 mg of an amber colored oil. The oil is chromatographed on one Merck size A column using 20% acetone-hexane to elute. 60 mg (30% of theory) of the titled product are obtained as a solid.

The product is rechromatographed. 129 mg are obtained as a white solid. A 65 mg portion is recrystallized from ether-pentane to give white crystals (36 mg) with a melting point of 81-83° C.

The C:H:N ratio is 72.84:6.22:4.65.

High resolution mass spectroscopy reveals the following:

Calcd for $C_{18}H_{17}NO_3$: 295.1208

Found: 295.1203

Other peaks are observed at m/e 250, 203, 175, and 149.

IR (nujol) peaks are observed at 1720, 1678, 1621, 933, 885, 858,

814, 767, and 715 $cm^{-1}$.

NMR ($CDCl_3$, δ) peaks are observed at 8.38, 7.67-6.90, 4.39, 2.98, and 1.39.

Example A-6 Sodium 6-[2-(3'-pyridinyl)ethyl]benzofuran-2-carboxylate (Formula A-I, $Z_1$ is 3-pyridinyl, $X_1$ is $-CH_2-CH_2-$ and is meta to the oxygen, $R_2$, $R_9$ and $R_{12}$ are hydrogen, m is zero, the D denotes a double bond, $R_7$ is $COOR_1$, and $R_1$ is Na)

The ester of Example A-5 (124 mg, 0.42 mmol) is dissolved in methanol (4 ml). Sodium hydroxide solution (4.2 ml of 0.1 N NaOH) is added with stirring. The reaction is complete after 5 hrs. The methanol is partially removed under reduced pressure. The aqueous residue is frozen in a dry ice-acetone bath and then lyophilized. A white powder is obtained (116 mg).

IR peaks (nujol) are observed at 1610, 1560, 840, 810, 790, and 715 $cm^{-1}$.

Example A-7 2-Hydroxymethyl-5-(3'-pyridinylmethyl)benzofuran (Formula A-I, $Z_1$ is 3-pyridinyl, $X_1$ is $-CH_2-$, and is para to the oxygen, $R_2$, $R_9$ and $R_{12}$ are hydrogen, m is zero, D denotes a double bond, and $R_7$ is $CH_2OH$)

To a mixture of lithium aluminum hydride (0.439 g) in tetrahydrofuran (20 ml), cooled under $N_2$ in an ice bath, is added dropwise a solution of ethyl 5-(3'-pyridinylmethyl)benzofuran-2-carboxylate (3.10 g) (Example A-6) in tetrahydrofuran (40 ml). The ice bath is removed after 15 min and after one hr the reaction is quenched with ethyl acetate followed tetrahydrofuran-water (3:1). The mixture is filtered through Celite. The filtrate is washed with saturated aqueous sodium bicarbonate and with brine. The organic solution is dried over sodium sulfate, filtered, and evaporated giving 2.67 g. of a pale yellow oil. Following chromatography over silica gel using 50-70% acetone-hexane as an eluent, 2.491 g of pure 2-hydroxymethyl-5-(3'-pyridinylmethyl)benzofuran is obtained. The compound crystallizes and after two recrystallizations from acetone-hexane, colorless crystals, having a melting point of 89-89.5° C, are obtained. NMR ($CDCl_3$, δ) reveals peaks at 8.40, 6.90-7.55, 6.53, 4.72, and 3.97; IR (Nujol) reveals peaks at 3189, 1616, 1599, 1579, 1470, 1450, 1442, 1263, 1234, 1189, 1114, 1073, 950, 810, and 794 $cm^{-1}$. High resolution mass spectoscopy reveals the following

peaks: m/e 239.0948 (calcd for $C_{15}H_{13}NO_2$: 239.0946), 222, 210, 180, 161, and 147.

The C:H:N ratio is 75.25:5.44:5.85.

Example A-8 2-Hydroxymethyl-5-(3'-pyridinylmethyl)benzofuran Hydrochloride

Hydrogen chloride gas is bubbled through a solution of 2-hydroxymethyl-5-(3'-pyridinylmethyl)benzofuran (0.263 g) (Example A-7) in dry ether. A white precipitate forms which is recrystallized from methanol-ether, giving the product (0.262 g) as colorless crystals with a melting point of 163-166° C.

Example A-9 5-(3-Pyridinylmethyl)-2-benzofurancarboxylic Acid (Formula A-I, $Z_1$ is 3-pyridinyl, $X_1$ is $-CH_2-$ and is para to the oxygen, $R_2$, $R_9$ and $R_{12}$ are hydrogen, D represents a double bond, $R_2$ is hydrogen, m is 0, and $R_7$ is COOH) (The free acid of Example A-1)

Aqueous 1.0 N hydrochloric acid (2.0 ml, 0.0020 mole) is added to a solution of the compound of Example A-2 (0.550 g., 0.0020 mole) in water (1 ml). A white solid separates immediately and, after 15 min, is collected by filtration using an extra milliliter of water to aid the transfer of the mixture. The solid is washed with water (4 ml) and dried, giving 0.458 g (0.0081 mole, 91%) of crystalline solid, with a melting point of 227-228° C. The solid can be recrystallized from hot water or from acetone. From acetone, white crystals, melting point 229-230° C are obtained having the properties: IR (Nujol) reveals peaks at 1722, 1567, 1332, 1199, 1143, 1060, 804, 775, 764, and 712 $cm^{-1}$.

The C:H:N ratio is 71.11:4.32:5.49.

Example A-10 5-(3-Pyridinylmethyl)-2-benzofurancarboxylic Acid Hydrochloride (The hydrochloric acid addition salt of Example A-9)

A hydrogen chloride saturated ether solution (20 ml) is added to a solution of the compound of Example A-9 (0.210 g, 0.000830 mole) in acetone (375 ml). The volume of the resulting solution is reduced by removing solvent on the steam bath. When the volume reaches about 250 ml, crystals are seen. The solution is cooled on ice for two hrs and the crystals (0.165 g, 0.000571 mole, 69%) are collected and are found to have a melting point of 216-248° C. The crystals are placed in acetone (400 ml), and another portion (20 ml) of ether saturated with

hydrochloride is added. The mixture is left at room temperature several days after which 0.132 g of the titled crystals are collected, having a melting point 215-240° C. IR (Nujol) reveals peaks at 2575, 1716, 1633, 1611, 1566, 1555, 1278, 1265, 1193, 1137, 938, 833, 819, 807, 791, 767, 761, 745, 715, and 690 $cm^{-1}$.

Anal. Calcd. for $C_{15}H_{12}ClNO_3$: C, 62.18; H, 4.18; N, 4.84.

The C:H:N ratio is 62.05:4.22:4.88.

The crystalline product dissolves in water (at room temperature) but after standing several minutes, a crystalline precipitate forms. These crystals are collected by filtration and have a melting point of 227-229° C.

Example A-11 5-(3-Pyridylmethyl)-2-benzofurancarboxylic Acid Amide (Formula A-I, $Z_1$ is 3-pyridinyl, $X_1$ is $-CH_2-CH_2-$ and is para to the oxygen, $R_2$ is hydrogen, m is zero, the D denotes a double bond, $R_7$ is $-CONH_2$, and $R_1$ is ethyl) (The amide of Example A-3)

A solution of the compound of Example A-1 (2.0 g, 0.0071 mole) in absolute ethanol (240 ml) saturated with anhydrous ammonia is stirred intermitently at room temperature (for a total of 100 hrs) and at reflux temperature (twice for a total of 12 hrs). The solution is re-saturated with ammonia following each reflux period. The product: starting material ratio now is about 3:1. Ethanol is removed under reduced pressure and the crystalline residue is recrystallized from acetonitrile to give 1.396 g (0.00553 mole, 78%) of titled product as a white crystalline solid, with a melting point of 151.5-152.5° C, containing no starting ester as determined by TLC. The compound is recrystallized again from acetonitrile, giving colorless crystals, with melting point 151.5-152.5°C. The IR (Nujol) spectrum reveals peaks at 3249, 3085, 1695, 1635, 1585, 1477, 1425, 1266, 1193, 1125, 1042, 1027, 944, 937, 875, 835, 830, 770, 714 and 704 $cm^{-1}$. The NMR ($CDCl_3$, δ) spectrum reveals peaks at 8.55, 7.64-7.16, and 4.10. The mass spectrum reveals ions at m/e 252.0891, 236, 208, and 180.

The C:H:N ratio is 71:18:4.87:11.00.

Example A-12 5-(3-Pyridinoyl)-2-benzofurancarboxylic Acid (Formula A-I, $Z_1$ is 3-pyridinyl, $X_1$ is -C(O)- and is para to the oxygen, $R_2$ is hydrogen, D represents a double bond, $R_2$ is hydrogen, m is 0, and $R_7$ is COOH)

A mixture of potassium superoxide (0.20 g, 0.00282 mole) in

dimethylsulfoxide (10 ml) containing dicyclohexyl-18-crown-6 (0.10 g) is stirred 30 min at room temperature. The compound of Example A-1 (0.140 g, 0.00050 mole) is added directly to the mixture and stirring is continued. The reaction is followed by TLC (1:1 acetone-hexane or 75% ethyl acetate-hexane) until no starting material remains in the reaction mixture. After four hours the reaction is poured into water (75 ml) and the pH of the resulting solution is adjusted to 4-5 by addition of 1N aqueous hydrochloric acid. A white precipitate gradually forms. The solution is cooled on ice and after several hours the solid is collected by filtration. The dry solid weighs 0.049 g (0.000183 mole, 36%) and did not melt below 300°C. The solid may be crystallized from either acetic acid-water or methanol. A sample dissolved in hot glacial acetic acid and crystallized by the addition of water was submitted for analysis; IR (Nujol) reveals peaks at 1731, 1655, 1613, 1598, 1584, 1572, 1321, 1254, 1180, 1143, 1125, 1116, 1097, 1053, 754 $cm^{-1}$, mass spectrum reveals ions at 267, 222, 189, and 161 m/e.

Anal. Calcd. for $C_{15}H_9NO_4$ (267.23): C, 67.41; H, 3.39; N, 5.26. The C:H:N ratio is 67.07:3.40:5.09.

<u>Preparation A-11</u> Ethyl 5-(3-Pyridinylmethyl)-2-benzofurancarboxylate, N-Oxide (The N-oxide of the compound of Example A-1)

Refer to Chart L (conversion of CV to CVI).

A solution of the benzofuran ester of Example A-1 (563 mg, 2.0 mmol) in methylene chloride (50 ml) is treated with m-chloroperbenzoic acid (447 mg of 85% m-chloroperbenzoic acid, 2.2 mmol) and stirred at room temperature. The reaction is found to be complete after two hrs by thin-layer chromatography on silica gel. The reaction is worked up by dilution with methylene chloride. The methylene chloride solution is washed with saturated $HaHCO_3$ solution (2x), followed by water and thin dried ($Na_2So_4$). After filtration, the solvent is removed to give 621 mg of a solid. The product is recrystallized twice from acetone-hexane to give 445 mg of white crystals having a melting point of 120-121° C.

TLC: silica gel, 10% methanol-chloroform starting material $R_f$ = 0.66, product $R_f$ = 0.42.

Anal. Calcd for $C_{17}H_{15}NO_4$: C, 68.67; H, 5.08; N, 4.71. The C:H:N ratio is 68.64:5.46:4.62.

The high resolution mass spectrum reveals: Calcd for $C_{17}H_{15}HO_4$: 297.1001 Found: 297.0994.

The IR (mull) spectrum reveals peaks at 1739, 1602, 1568, 1480, 1319, 1296, 1265, 1215, 1198, 1143, 970, 938, 895, 851, 841, 832, 805, and 758 $cm^{-1}$.

NMR ($CDCl_3$, δ) spectrum reveals peaks at 8.09, 7.27, 4.45 and 4.03.

Example A-13 5-[3-(2-Chloropyridyl)methyl]-2-benzofurancarboxylic Acid, Ethyl Ester; 5-[3-(4-Chloropyridyl)methyl]-2-benzofurancarboxylic Acid, Ethyl Ester; 5-[3-(5-Chloropyridyl)methyl-2-benzofurancarboxylic Acid, Ethyl Ester; and 5-[3-(6-Chloropyridyl)methyl]-2-benzofurancarboxylic Acid, Ethyl Ester (Formula A-I, $Z_1$ is 3-(2,4,5, or 6-chloropyridyl), $X_1$ is $-CH_2-$ and is para to the oxygen, $R_2$ is hydrogen, D is a double bond, m is zero, and $R_7$ is $COOCH_2CH_3$)

Refer to Chart L (conversion of CVI to CVII).

A solution of the Preparation A-11 (0.878 g, 0.00295 mole) in 10 ml phosphorous oxychloride ($POCl_3$) is stirred and heated to the reflux temperature of $POCl_3$ for 30 mins. At this time TLC indicates that the desired reaction is complete. The reaction solution is poured into ice-water and ether and the pH of the aqueous phase is adjusted to approximately 8 by the addition of solid potassium carbonate. The ether layer is separated and the aqueous phase is extracted three more times with ether. The ether extracts are pooled, washed with water, dried over sodium sulfate, filtered, and concentrated under reduced pressure. The residual oil is chromatographed over two Merck size B Lobar silica gel columns, using 20% ethyl acetate-hexane followed at fraction 113 by 40% ethylacetate-hexane to elute the column. Fractions of 25 ml are collected and the products elute in the following order of increasing polarity: the 6-chloropyridyl isomer (0.295 g, 0.00093 mole, 31%) is obtained in fractions 31-43; the 2-chloropyridyl isomer, (0.271 g, 0.00086 mole, 29%) is obtained in fractions 47-56; the 5-chloropyridinyl isomer, (0.017 g, 0.000054 mole, 2%) is obtained in fractions 79-87; and the 4-chloropyridyl isomer (0.114 g, 0.00036 mole, 12%) is obtained in fractions 145-154. After pooling the appropriate fractions and removing solvent, all the products crystallize. The individual isomers are recrystallized and

analyzed as follows:

Recrystallization of fractions 31-43 from acetone-hexane gives 0.272 g of 5-[3-(6-chloropyridyl)methyl]-2-benzofurancarboxylic acid ethyl ester as colorless crystals, melting point 112-112.5° C; IR (Nujol) reveals peaks at 1734, 1723, 1563, 1439, 1320, 1304, 1288, 1214, 1194, 1145, 1140, 1097, 1025, 836, 812, 767, 746 $cm^{-1}$; 'N NMR ($CDCl_3$, δ) spectrum reveals peaks at 8.36, 7.18-7.65, 4.45, 4.07 and 1.42; mass spectrum reveals ions at 315.0649, (calcd for $C_{17}H_{14}$ $^{35}ClNO_3$: 315.0662) 270, 242, and 214 m/e.

Anal. Calcd. for $C_{17}H_{14}ClNO_3$: C, 64.66; H, 4.47; N, 4.44. The C:H:N ratio is 64.30:4.54:4.36.

Recrystallization of fractions 47-56 from hexane gives 0.243 g of 5-[3-(2-chloropyridyl)methyl]-2-benzofurancarboxylic acid ethyl ester, as colorless crystals, melting point 82-83° C; IR (Nujol) reveals peaks at 1727, 1568, 1564, 1409, 1322, 1311, 1298, 1214, 1200, 1193, 1146, 1098, 1063, 1020, 843, 811, 786, 767 $cm^{-1}$; NMR ($CDCl_3$, δ) spectrum reveals peaks at 8.32, 7.10-7.68, 4.43, 4.17, and 1.40. Mass spectrum reveals ions at 315.0658, 270, 242) and 214 m/e.

Anal. Calcd. for $C_{17}H_{14}ClNO_3$: C, 64.66; H, 4.47; N, 4.44. The C:H:N ratio is 64.53:4.49:4.35.

The crystalline fractions 79-87 of 5-[3-(5-chloropyridyl)methyl]-2-benzofurancarboxylic acid ethyl ester are used for analysis without recrystallization and have melting point 90-91°C; NMR ($CDCl_3$, δ) spectrum reveals peaks at 8.48, 7.23-7.73, 4.48, 4.09, and 1.43. Mass spectrum reveals ions at 315.0661, 271, 242 and 214 m/e.

Recrystallization of fractions 145-154 from hexane gives 0.098 g of 5-[3-(4-chloropyridyl)methyl]-2-benzofurancarboxylic acid ethyl ester as colorless crytals, melting point 103-104° C; IR (Nujol) reveals peaks at 1714, 1578, 1556, 1436, 1408, 1347, 1318, 1302, 1267, 1221, 1199, 1134, 1125, 1084, 1012, 943, 882, 833, 825, 820, 804, 782, 762, 741, 695 $cm^{-1}$; NMR ($CDCl_3$, δ) spectrum reveals peaks at 8.52, 8.47, 7.23-7.73, 4.48, 4.09, and 1.43; mass spectrum reveals ions at 315.0655, 270, 242, and 214 m/e.

Anal. Calcd. for $C_{17}H_{14}ClNO_3$: C, 64.66; H, 4.47; N, 4.44. The C:H:N ratio is 64.46:4.44:4.61.

<u>Example A-14</u> 5-[3-(6-Chloropyridyl)methyl]-2-benzofurancarboxylic Acid, Sodium Salt (Formula A-I, $Z_1$ is 6-chloropyridyl $X_1$ is $-CH_2-$ and is para to the oxygen, $R_2$ is hydrogen,

D is a double bond, m is zero, and $R_7$ is COONa)

A solution of the corresponding ethyl ester (0.221 g, 0.00070 mole) in methanol (15 ml) and aqueous 0.10 N sodium hydroxide (7.3 ml, 0.00073 mole) is stirred 16 hrs at room temperature after which TLC indicates complete conversion of starting material to product. The solvent is removed under reduced pressure, leaving a white solid.

The 'N NMR ($D_2O$ + $CD_3OD$, δ) spectrum reveals peaks at 8.13, 7.05-7.63 and 3.94.

Example A-15 5-[3-(2-Chloropyridyl)methyl]-2-benzofurancarboxylic Acid, Sodium Salt (Formula A-I, $Z_1$ is 2-chloropyridyl $X_1$ is $-CH_2-$ and is para to the oxygen, $R_2$ is hydrogen, D is a double bond, m is zero, and $R_7$ is COONa)

A solution of the corresponding ethyl ester (0.200 g, 0.00063 mole) in methanol (11 ml) and aqueous 0.10 N sodium hydroxide (6.50 ml, 0.00065 mole) is stirred 16 hrs at room temperature. TLC (A-9) shows reaction is complete. Solvent is removed under reduced pressure, leaving a white solid.

'N NMR ($D_2O$ + $CD_3OD$ , δ) spectrum reveals peaks at 8.29, 7.87, 7.18-7.64 and 4.16.

Example A-16 5-[3-(4-Chloropyridyl)methyl]-2-benzofurancarboxylic Acid, Sodium Salt (Formula A-I, $Z_1$ is 4-chloropyridyl $X_1$ is $-CH_2-$ and is para to the oxygen, $R_2$ is hydrogen, D is a double bond, m is zero, and $R_7$ is COONa)

A solution of the corresponding ethyl ester (0.063 g, 0.00020 mole) in methanol (4 ml) and aqueous 0.10 N sodium hydroxide (2.1 ml, 0.00021 mole) is stirred 16 hr at room temperature. TLC (A-9) shows no remaining starting material. Solvent is removed under reduced pressure leaving a white solid.

The NMR ($D_2O$ + $CD_3OD$, δ) spectrum reveals peaks at 8.44, 8.33, 7.10-7.60 and 4.13.

Preparation A-12 3-[(3'-Methoxy-4'-benzyloxyphenyl)hydroxymethyl]-pyridine

Refer to Chart K (analogous to conversion of XCVIII to CI).

The procedure described previously is followed using 3-bromopyridine (15.8 g, 9.64 ml, 0.10 mole), n-butyllithium (75.5 ml of a 1.6 M solution 0.12 mole), and 3-methoxy-4-benzyloxybenzaldehyde (24.2 g, 0.10 mole, Aldrich Chem. Co.) in ether-THF under a nitrogen atmosphere at -78° C. After the addition of aldehyde is complete, the

dry ice-acetone bath is removed and the reaction is allowed to warm. After 3 hrs, the reaction is quenched by the careful addition of a 3:1 mixture of THF-water. Additional water is added and the mixture is extracted 3 times with ether. The combined ether extracts are wased with water, with brine, and are dried over sodium sulfate. The ether solution is filtered and concentrated under reduced pressure. The residue (34 g) is combined with the crude product (33 g) from a second, identical preparation and chromatographed over silica gel (1.2 kg) packed as a slurry in 40% acetone-hexane. The crude product is applied to the column as a solution in 1:1 acetone-methylene chloride. The column is eluted with 40% acetone-hexane (25 fractions), 50% acetone-hexane (23) fractions, and then with 60% acetone-hexane. Fractions of 350 ml volume are collected. The desired product is eluted in fractions 33-59 and was obtained as a crystalline material weighing 43.14 g (0.131 mole, 65%). Recrystallization of a 2.0 g sample from acetone-hexane gives 1.88 g of the titled product as white crystals, with a melting point of 107.5-108.5° C. A second recrystallization gives 1.80 g of white crystals, with a melting point of 107.5-108.5° C. IR (Nujol) spectral analysis reveals peaks at 3150, 1607, 1592, 1583, 1514, 1261, 1222, 1132, 1077, 1030, 1008, 921, 862, 806, 752, 710, 701 and 670 $cm^{-1}$; the NMR ($CDCl_3$, δ) spectrum reveals peaks at 8.44, 8.25, 7.66, 6.70-7.53, 5.68, 5.08, and 3.73. The mass spectrum reveals ions at m/e 321 ($M^+$), 230, 106 and 91.

Anal. Calcd for $C_{20}H_{19}NO_3$: C, 74.74; H, 5.96; N, 4.36. The C:H:N ratio is 74.49:6.12:4.68.

Preparation A-13 2-Methoxy-4-(3-pyridylmethyl)phenol

Refer to Chart Q (conversion of CLV to CLVI).

A solution of the compound of Preparation A-13, (18.13 g, 0.056 mole) in absolute ethanol (200 ml) and 70% perchloric acid (5.1 ml, 0.056 mole) is shaken with 10% palladium on carbon (7.0 g) and hydrogen in a Parr apparatus. Uptake of hydrogen is complete after 4 hours. The catalyst is removed by filtration. Sodium bicarbonate (4.70 g, 0.056 mole) is added to the solution which then is left standing overnight. The ethanol is then removed under reduced pressure and the residue is taken up in ethyl acetate and half saturated aqueous sodium bicarbonate (40 ml). Additional water is added to dissolve a precipitated solid. The layers are separated and the aqueous layer is extracted with additional ethyl acetate. The

combined ethyl acetate layers are washed with brine twice, dried over sodium sulfate, filtered, and concentrated under reduced pressure. The residue (12.03 g) is a dark red oil which crystallizes. From this and from a second reduction (of 18.53 g, 0.058 mole) there is obtained upon crystallization from ethanol 7.52 g of crystals with a melting point of 138-141° C.

Recrystallization from acetone gives 6.36 g of titled product with a melting point of 141-142° C. The IR (Nujol) spectrum reveals peaks at 2785-2431, 1607, 1592, 1579, 1510, 1427, 1277, 1244, 1193, 1155, 1129, 1047, 1039, 879, 819, 805, 742, and 709 $cm^{-1}$. The NMR ($d_6$-acetone) spectrum reveals peaks at 8.57δ, 8.47, 7.65, 7.30, 6.63-7.00, 3.92, and 3.80. The mass spectrum reveals ions at m/e 215.0937, 200, 184, 172, and 154.

Anal. Calcd. for $C_{13}H_{13}NO_2$: C, 72.54; H, 6.09, N, 6.51.

The C:H:N ratio is 72.61:6.17:6.51.

The filtrates and residues from the above are pooled and chromatographed over silica gel (1 kg) packed as a slurry is 35%-acetone hexane. The material is applied to the column in an acetone solution and the column is eluted with 35% acetone-hexane, collecting 350 ml fractions. From fractions 32-50, there is obtained 6.35 g (total 12.71 g, 0.059 mole, 51%) of titled product, melting point of 142-143° C.

<u>Preparation A-14</u> 2-Hydroxy-3-methoxy-5-(3-pyridylmethyl)benzaldehyde

Refer to Chart B (conversion of XXIII to XXIV).

The procedure described previously is followed using the compound of Preparation 13 (12.82 g, 0.059 mole), hexamethylenetetramine (9.11 g, 0.064 mole), and trifluoroacetic acid (125 ml). Following workup and chromatography over silica gel (RP2, 370 q, 30-40% acetone-hexane), there is obtained crystalline titled product (6.33 g, 0.026 mole, 43%) with a melting point of 114-115° C, from acetone-hexane.

Recrystallization from acetone-hexane give colorless crystals with a melting point of 114.5-115.5° C.; IR (Nujol) spectrum reveals peaks at 2416-2776, 1670, 1607, 1601, 1583, 1503, 1272, 1218, 1192, 1148, 1085, 987, 931, 815, 800, and 711 $cm^{-1}$. The NMR ($CDCl_3$, δ) spectrum reveals peaks at 9.98, 8.58, 7.57, 7.30, 7.02, 4.01, and 3.90. The mass spectrum reveals ions at m/e 243.0881, 215, and 214.

Anal. Calcd. for $C_{14}H_{13}NO_3$: C, 69.12; H, 5.39; N, 5.76.

The C:H:N ratio is 69.02:5.41:5.71.

Preparation A-15 3-Hydroxy-7-methoxy-5-(3-pyridylmethyl)-2,3-dihydrobenzofuran-2,2-dicarboxylic Acid, Diethyl Ester

Refer to Chart A (conversion of X to XIII).

Using the procedure of preceeding Examples the following quantities of reagents are used: sodium hydride (0.624 g, 0.026 mole, 1.05 g of a 59.6% suspension in mineral oil) in toluene (10 ml), the above aldehyde of Preparation 14 (2.43 g, 0.010 mole) in toluene (150 ml), diethyl bromomalonate (2.63 g, 0.011 mole) in toluene (10 ml), and dicyclohexyl-18-crown-6(0.415 g). The reaction is followed by quenching aliquots in aqueous $NaHCO_3$ and ethyl acetate with TLC examination of the ethyl acetate layer. After 48 hr, the reaction appears to contain about 1:1 starting material and a single product and is not progressing further. The reaction is worked up as previously described and the crude extract is chromatographed over two Merck size B Lobar silica gel columns. The sample is applied to the columns in methylene chloride solution and the column is eluted with ethyl acetate, collecting 25 ml fractions. Fractions 22-30 contained the new product (1.21 g, 0.00302 mole, 30%), fractions 31-34 contained 0.184 g of mixed products and fractions 34-54 contain 0.410 g starting materials. Recrystallization of fractions 22-30 from acetone-hexane gives colorless crystals with melting point of 140.5-142° C. A second recrystallization gives an analytical sampe of the title crystals, melting point of 140.5-142° C. The IR (Nujol) spectrum reveals peaks at 3004-3093, 2728, 1744, 1722, 162, 1608, 1596, 1582, 1503, 1309, 1303, 1260, 1222, 1144, 1092, 1068, 1043, 1032, 1023, 856 and 728 $cm^{-1}$. The NMR ($CDCl_3$, δ) spectrum reveals peaks at 8.42, 7.50, 7.24, 6.85, 6.70, 5.98, 4.30, 3.90, 3.83, and 1.27. The mass spectrum reveals ions at m/e 401.1483, 311, 282, 266, 254, and 241.

Anal. Calcd. for $C_{21}H_{23}NO_7$: C, 62.83; H, 5.78; N, 3.49.

The C:H:N ratio is 62.65:5.94:3.47.

Preparation A-16 3-Hydroxy-7-methoxy-5-(3-pyridylmethyl)-2,3-dihydrobenzofuran-2,2-dicarboxylic Acid, Disodium Salt (Converstion of Preparation A-15 to its sodium salt)

A solution of the compound of Preparation A-15 (0.100 g, 0.00025 mole) in methanol (4 ml) and aqueous 0.10 N sodium hydroxide (5.5 ml,

0.00055 mole) is stirred 2 hr at room temperature after which time no remaining starting material is detected by TLC. The solvent is removed under reduced pressure giving a white solid. The NMR ($D_2O$, δ) spectrum reveals peaks at 8.32, 7.64, 7.30, 6.91, 6.86, 5.63, 3.92, and 3.84. A 54 mg sample is recrystallized (or repreceipitated) from methanol-water, giving 30 mg of titled product. The IR (Nujol) spectrum reveals peaks at 3271, 1645, 1624, 1574, 1503, 1354, 1322, 1291, 1237, 1142, 1066, 1042, 1027, 1015, 878, 797, 738 and 718 $cm^{-1}$.

<u>Example A-17</u> 7-Methoxy-5-(3-pyridinylmethyl)-2-benzofurancarboxylic Acid Ethyl Ester (Formula A-I: $Z_2$ is 3-pyridinyl, $X_1$ is $-CH_2-$ and is para to the oxygen, $R_9$ is 7-methoxy, $R_{12}$ and $R_2$ are hydrogen, D is a double bond, m is zero, and $R_7$ is $-COOCH_2CH_3$)

Refer to Chart A (conversion of XIII to XV).

A solution of the compound of Preparation A-15 (0.818 g, 0.00204 mole) in dry tetrahydrofuran (THF, 20 ml) is added dropwise to a stirred mixture of sodium hydride (0.049 g, 0.00204 mole, 0.082 g of a 59% dispersion in oil) in THF (2 ml) kept under an atmosphere of nitrogen. The reaction is stirred for 1 hr. After one hr, additional sodium hydride (0.049 g) and THF (20 ml) are added. Dicyclohexyl-18-crown-6 (0.10 g) is also added at this point. After about two hr following the initial additions of reactants, TLC shows roughly equal amounts of starting material and a new material assumed to be product. Sixteen hrs later, no further change is observed in the ratio of starting material and final product so the reaction is diluted with an additional 150 ml of THF. This had little effect on the ratio of compounds. After 72 hr, the reaction is again diluted with THF (200 ml). The reaction is complete within one hr after this final dilution. The reaction mixture is poured into a solution made up of brine (100 ml) and saturated aqueous sodium bicarbonate (100 ml) together with some ice. The layers are separated and the aqueous phase is extracted four times with ethyl acetate. All organic extracts are pooled, washed twice with 50% brine, dried ($Na_2SO_4$), filtered, and concentrated under reduced pressure. A pale yellow oil (0.569 g) was obtained and is chromatographed over one Merck size B silica gel Lobar column. The sample is applied to the column as a solution in acetone-methylene chloride. The column is eluted with 30% acetone-hexane and fractions of 25 ml volume are collected. The

desired material (0.253 g, 0.00081 mole, 40%) is eluted in fractions 31-36 and crystallized. One recrystallization from acetone-hexane gives 0.225 g of titled product as colorless crystals, with a melting point of 98.5-99.5° C. The IR (Nujol) spectrum reveals peaks at 1728, 1600, 1569, 1321, 1295, 1273, 1201, 1153, 1146, 1095, 1031, 1021, 991, 951, 944, 891, 842, 787, 769, 745, and 719 $cm^{-1}$. The NMR ($CDCl_3$, δ) spectrum reveals peaks at 8.60, 7.54, 7.51, 7.27, 7.08, 6.80, 4.42, 4.04, 3.96, and 1.38.

The mass spectrum reveals ions at m/e 311.1169, 296, 282, and 266.

Anal. Calcd. for $C_{18}H_{17}NO_4$: C, 69.44; H, 5.50; N, 4.50.

The C:H:N ratio is 69.43:5.61:4.43.

Example A-18 7-Methoxy-5-(3-pyridylmethyl)-2-benzofurancarboxylic Acid Sodium Salt (Formula A-I: $Z_1$ is 3-pyridinyl, $X_1$ is $-CH_2-$ and is para to the oxygen, $R_9$ is 7-bromo, $R_{12}$ and $R_2$ are hydrogen, m is zero, D is a double bond, and $R_7$ is -COONa)

A solution of the compound of Example A-17 (0.175 g, 0.00056 mole) in methanol (6 ml) and aqueous 0.10 N sodium hydroxide (6.2 ml) is stirred at room temperature 4 hr. The methano is removed under reduced pressure and the aqueous solution remaining is lyophilized, giving 0.169 g of titled product as a white powder. The IR (Nujol) spectrum reveals peaks at 3379, 1617, 1600, 1574, 1479, 1330, 1268, 1210, 1143, 1028, 993, 938, 845, 815, 791, 786, 744, and 716 $cm^{-1}$. The NMR ($D_2O$, δ) spectrum reveals peaks at 8.27, 7.46, 7.2, 6.78, 6.62, 3.86, and 3.76.

Preparation A-17 3-Bromo-2-hydroxy-5-(3-pyridylmethyl)benzaldehyde

Refer to Chart M (conversion of CX to CXI).

Bromine (0.54 ml, 1.59 g, 0.010 mole) is added dropwise over a period of 1-2 min to a stirred solution of 2-hydroxy-5-(3-pyridinylmethyl)benzaldehyde (2.13 g, 0.010 mole) in glacial acetic acid (50 ml). As the addition of bromine is completed, orange cyrstals are seen to be forming in the reaction mixture. The mixture is stirred 30 min at room temperature and then filtered to allow collection of the crystalline solid. (1.865 g are obtained after air drying on the filter). The filtrate is concentrated, giving a partially crystalline residue. TLC (75% ethyl acetate in hexane) shows unreacted starting material remaining in the residue (a sample

is quenched in aq. $NaHCO_3$ and ethyl acetate for TLC). The residue is mixed with acetic acid (25 ml) and stirred. Bromine (0.2 ml) is added. After stirring 30 min, the additional orange crystals that had formed are collected (2.179 g). The crystals dissolve in acetone with loss of their orange color. Crystallization is attempted by addition of hexane but only gives a greenish solid mass. Finally, the solvent is removed, the residue is taken up in water, and the pH of the aqueous solution is adjusted to approximately 7 by addition of sodium carbonate. The resulting mixture is extracted once with ether and 4 times with ethyl acetate. The pooled organic extract solutions are washed with brine, dried ($MgSo_4$), filtered, and concentrated under reduced pressure. The residue is taken up in acetone (in which it is not completely soluble) and chromatographed over silica gel (65 g) packed as a slurry in 1:1 acetone-hexane. Fractions of 25 ml volume are collected. The desired product is found in fractions 12-22 and is a crystalline solid. Recrystallization from acetone-hexane gives a first crop of yellow crystals (0.919 g) with a melting point of 87-88° C. A second crop of pale yellow crystals (0.212 g, total crystals, 1.131 g, 0.0045 mole, 45%), melting point of 114-115° C, is obtained. Upon recrystallization from acetone-hexane, the crystals from the first crop gave pale yellow crystals with a melting point of 115-116° C. The IR (Nujol) spectrum reveals peaks at 1650, 1575, 1476, 1458, 1440, 1311, 1302, 1239, 1136, 1027, 835, 739, 720, and 695 $cm^{-1}$. UV (EtOH) at 0.01856 g/ℓ reveals 221 mμ (ε = 21,600), 258.5 (11,550), 263 (12,350), 342 (3,000), and 400 (1,750) at 0.00742 g/ℓ 220 (20,250), 263 (12,250), 268 sh (10,600), 345 (2.750), and 399 (3,250). The mass spectrum reveals ions at m/e 293, 291, 265, 263, 212, 184, and 154.

Anal. Calcd. for $C_{13}H_{10}BrNO_2$: C, 53.45; H, 3.45; N, 4.80; Br, 27.36.

The C:H:N:Br ratio is 53.42:3.67:4.72:27.41.

Example A-19 7-Bromo-5-(3-pyridylmethyl)-2-benzofurancarboxylic Acid Ethyl Ester (Formula A-I: $Z_1$ is 3-pyridinyl, $X_1$ is $-CH_2-$ and is para to the oxygen, $R_9$ is 7-bromo, $R_{12}$ and $R_2$ are hydrogen, m is zero, D is a double bond, and $R_7$ is $COOCH_2CH_3$)

Refer to Chart A.

A solution of potassium t-butoxide (0.6 ml, 1.6-1.8 M in THF) is added in portions to a stirred solution of Preparation 17 (0.292 g,

0.0010 mole) in THF (8 ml). A pale yellow precipitate forms over a period of several min. The mixture is stirred 20 min and then a solution of diethyl bromomalonate (0.247 g, 0.00103 mole) in THF (5 ml) is added dropwise. The mixture is stirred one hr after the addition is completed and after this time conversion of the starting phenol to a more polar material is nearly complete as detected by TLC. Sodium hydride (0.045 g of a 59% suspension in oil, 0.026 g, 0.0010 mole) is added to the stirred mixture. No significant change in the reaction is observed and after 4 hr additional sodium hydride (0.045 g of the suspension) is added. This, and additional THF (10 ml), has little effect on the reaction during a period of 12 hr and only after addition of potassium t-butoxide (0.6 ml of THF) does the polar intermediate change to the less polar, desired product. The reaction is quenched one hr after addition of the K-t-OBu by pouring into cold brine and saturated sodium bicarbonate. The aqueous phase is extracted with ethyl acetate 4 times. The combined extracts are washed with brine, dried over sodium sulfate, filtered, and concentrated under reduced pressure. The crude product (0.357 g) is chromatographed over one Merck size B Lobar silica gel column using 25% acetone-hexane to elute the column. Fractions of 25 ml volume are collected. A minor component (0.019 g, 0.000049 mole, 5%) is obtained in fractions 23-26. This material crystallizes and from the NMR spectrum is assigned the structure of 7-bromo-5-(3-pyridylmethyl)-2-benzofurancarboxylic acid, t-butyl ester. The NMR ($CDCl_3$, δ) spectrum reveals peaks at 8.58, 7.17-7.60, 4.05, and 1.62.

The desired product is eluted in fractions 30-35, giving 0.201 g (0.000556 mole, 55%) of crystalline material. Recrystallization from acetone-hexane gives 0.169 g of 7-bromo-5-(3-pyridylmethyl)-2-benzofurancarboxylic acid, ethyl ester as colorless crystals, with a melting point of 109-110° C. The IR (Nujol) spectrum reveals peaks at 1702, 1582, 1574, 1424, 1366, 1303, 1215, 1134, 1088, 1018, 762, 735, and 715 $cm^{-1}$. The NMR ($CDCl_3$, δ) spectrum reveals peaks at 8.56, 7.13-7.60, 4.44, 4.05 and 1.40. The mass spectrum reveals ions at m/e 361, 359, 333, 332, 331, 330, 316, 314, 280, 252, 207, and 178.

Anal. Calcd. for $C_{17}H_{14}BRNO_3$: C, 56.68; H, 3.92; N, 3.89; Br, 22.19. The C:H:N:Br ratio is 56.52:3.90:3.79:22.33.

Example A-20 7-Bromo-5-(3-pyridylmethyl)-2-benzofurancarboxylic Acid Sodium Salt (Formula A-I: $Z_1$ is 3-pyridinyl, $X_1$ is

$-CH_2-$ and is para to the oxygen, $R_9$ is 7-methyl, $R_2$ and $R_{12}$ are hydrogen, m is zero, D is a double bond, and $R_7$ is -COONa)

A solution of the compound of Example A-19 (0.108 g, 0.00030 mole) in methanol (5 ml) and aqueous 0.10 N sodium hydroxide (3.0 ml, 0.00030 mole) i s stirred at room temperature overnight. TLC evidence (1:1 acetone-hexane) indicates complete consumption of the starting ester. The solvent is removed under pressure. The glassy residue crystallizes. The product is scraped from the flask and used as such.

The IR (Nujol) spectrum reveals peaks at 3359, 1634, 1620, 1614, 1602, 1577, 1572, 1337, 1198, 1128, 1028, 939, 871, 798, 743, and 709 $cm^{-1}$.

Preparation A-18 3-(4-Methoxy-3-methyl)phenylhydroxymethylpyridine

Refer to Chart K (analogous to the conversion of XCVIII to CI).

The procedure described in Example A-34 is followed using 3-bromopyridine (24.2 g, 14.7 ml, 0.15 mole), n-butyllithium (93.8 ml of a 1.6 M solution in hexane, 0.15 mole), and 3-methyl-p-anisaldehyde (25.0 g of 90% purity, 22.5 g, 0.15 mole) in ether under a nitrogen atmosphere at -78° C. The reaction is allowed to warm to room temperature over a 3 hr period and the product is washed as previously described. The crude product is chromatographed over silica gel (1.5 kg) packed as a slurry in 35% acetone-hexane. The column is eluted with 40% acetone-hexane (22 fractions) and 50% acetone-hexane (to end) with 350 ml fractions collected. The product (26.26 g, 0.114 mole, 76%) is obtained in fractios 23-40 and is crystalline. Two recrystallizations from acetone-hexane give the title product as colorless crystals with a melting point of 92.5-95° C. The IR (Nujol) spectrum reveals peaks at 3194, 1607, 1592, 1578, 1505, 1448, 1425, 1318, 1256, 1213, 1186, 1127, 1056, 1034, 1027, 825, 814, 745, 713, and 671 $cm^{-1}$.

The NMR ($CDCl_3$, $\delta$) spectrum reveals peaks at 8.47, 8.30, 7.74, 6.68-7.33, 5.72, 3.78, and 2.17.

The mass spectrum reveals ions at m/e 229.1115, 212, 198, 151, 123, and 106.

Anal. Calcd. for $C_{14}H_{15}NO_2$: C, 73.34; H, 6.59; N, 6.11. The C:H:N ratio is 73.51:6.68:5.96.

Preparation A-19 3-(4-Methoxy-3-methyl)benzylpyridine

Refer to Chart R (conversion of CLV to CLVI).

The procedure for hydrogenolysis is followed using 3-(4-methoxy-3-methyl)phenylhydroxymethylpyridine (23.54 g, 0.103 mole) in ethanol (200 ml), perchloric acid (9.23 ml of a 70% aqueous solution), and 10% palladium on carbon (7 g). Following reduction and work-up, 22.38 g of an amber oil is obtained. TLC indicates that this material is of satisfactory purity for use in synthetic transformations. For analysis, a sample of the product is chromatographed over two Merck size B Lobar silica gel columns using 40% ethyl acetate-hexane for elution. The pure titled product is a colorless oil that fails to crystallize. The IR (neat) spectrum reveals peaks at 3027, 2997, 2950, 2920, 2912, 2835, 1612, 1575, 1506, 1479, 1465, 1455, 1441, 1424, 1297, 1289, 1254, 1225, 1183, 1134, 1034, 1027, 803, 741, and 712 $cm^{-1}$.

The NMR ($CDCl_3$, δ) spectrum reveals peaks at 8.50, 8.43, 7.19, 6.64-7.07, 3.84, 3.78, and 2.18. The mass spectrum reveals ions at m/e 213.1164, 198, 182, and 135.

Anal. Calcd. for $C_{14}H_{15}NO$: C, 78.84; H, 7.09; N, 6.57. The C:H:N ratio is 78.63:7.06::6.33.

<u>Preparation A-20</u> 2-Methyl-4-(3-pyridylmethyl)phenol

Refer to Chart N (conversion of CXV to CXVI).

A solution of Preparation A-19 (18.83 g, 0.088 mole) in aqueous 48% hydrobromic acid (50 ml) is heated for 18 hr in an 110° C oil bath. The reaction solution is poured into ice water and the pH of the resulting solution is adjusted to about 7 by careful addition of 50% aqueous sodium hydroxide. The mixture is extracted 4 times with ethyl acetate and the pooled extracts are washed with brine, dried over sodium sulfate, filtered, and concentrated under reduced pressure. The residue (15.62 g) is a solid which is recrystallized from acetone-hexane, giving 12.29 g (0.0618 mole, 70%) of the titled product with a melting point of 165-168° C (capillary). Two recrystallizations from ethyl acetate-hexane give crystals having melting point of 166.5-168.5° C (capillary). The IR (Nujol) spectrum reveals peaks at 2713, 2676, 2579, 1740, 1611, 1592, 1588, 1512, 1480, 1435, 1425, 1273, 1262, 1245, 1213, 1186, 1143, 1120, 1046, 1033, 810, 739, 710, 645, and 630 $cm^{-1}$.

The NMR ($d_6$ acetone, δ) spectrum reveals peaks at 8.52, 8.42, 7.60, 7.27, 6.67-7.05, 3.88, and 2.16.

The mass spectrum reveals ions at m/e 199.0995, 184, 170, 154,

and 121.

Preparation A-21 2-Formyl-6-methyl-4-(3-pyridylmethyl)phenol

Refer to Chart N (conversion of CXVI to CXVII).

The procedure described previously is followed using the compound of Preparation A-20 (11.0 g, 0.055 mole), hexamethylenetetramine (8.55 g, 0.061 mole), and trifluoroacetic acid (105 ml, 157 g, 1.38 mole). Following workup and chromatography of silica gel (600 g) in 30% acetone-hexane, there is obtained 5.63 g (0.0248 mole, 45%) of titled product as a crystalline solid. Two recrystallizations from acetone-hexane give colorless titled crystals having a melting point of 49-50° C.

The IR (Nujol) spectrum reveals peaks at 1645, 1619, 1608, 1576, 1479, 1442, 1326, 1278, 1162, 1035, 1027, 834, 814, 744, 721 and 711 $cm^{-1}$.

The NMR ($CDCl_3$, δ) spectrum reveals peaks at 9.88, 8.58, 8.55, 7.55, 7.13-7.34, 3.93, and 2.24.

The mass spectrum reveals ions at m/e 227.0943 and 199.

Anal. Calcd. for $C_{14}H_{13}NO_2$: C, 73.99; H, 5.77; N, 6.16. The C:H:N ratio is 74.21:5.76:6.05.

Example A-21 7-Methyl-5-(3-pyridylmethyl)-2-benzofurancarboxylic Acid, Ethyl Ester (Formula Z-I: $Z_1$ is 3-pyridinyl, $X_1$ is $-CH_2-$ and is para to the oxygen, $R_9$ is 7-methyl, $R_2$ and $R_{12}$ are hydrogen, m is zero, D is a double bond, and $R_7$ is $-COOCH_2CH_3$)

Refer to Chart A (conversion of X to XV).

The previously described procedure is followed using the compound of Preparation A-21 (2.27 g, 0.010 mole), sodium hydride (0.264 g, 0.011 mole), diethyl bromomalonate (2.63 g, 0.011 mole), and dicyclohexyl-18-crown-6 (0.415 g) in a total of 210 of dry toluene. The reaction does not go to completion (TLC, 1:1 acetone-hexane) and, at various times, additional reagents (0.48 g of sodium hydride, 0.526 g of diethyl bromomalonate, 0.20 g of dicyclohexyl-18-crown-6, and 200 ml of toluene) are added without significantly affecting the reaction. After 4 days, the reaction is worked up as previously described, giving 4.43 g of an oil.

The oil (4.43 g) is dissolved in THF (300 ml) and sodium hydride (0.304 g of dispersion, 0.181 g, 0.00754 mole) is added as the dispersion in oil. Additional sodium hydride (total 0.608 g of

dispersion) and THF (300 ml) are added over a period of 2 days. At this point, the desired product has formed in part and the reaction mixture is worked up with the intent of obtaining this material. The previously described workup is followed giving 4.06 g of an amber oil. The oil is chromatographed over two Merck size B Lobar silica gel columns using acetone-methylene chloride to apply the sample and 30% acetone-hexane to elute the column. Fractions of 25 ml volume are collected. The desired material (0.834 g, 0.00282 mole, 28%) is obtained in fractions 34-39 and is a crystalline solid. Two recrystallizations from hexane give colorless crystals of the titled product with melting point 100-101° C.

The NMR ($CDCl_3$, δ) spectrum reveals peaks at 8.58, 8.53, 7.04-7.63, 4.44, 4.04, 2.54, and 1.42. The mass spectrum reveals ions at m/e 295.1200, 280, 266, 250, 222, and 194.

Anal. Calcd. for $C_{18}H_{17}NO_3$: C, 73.20; H, 5.80; N, 4.74. The C:H:N ratio is 73.07:5.93:4.68.

Example A-22 7-Methyl-5-(3-pyridylmethyl)-2-benzofurancarboxylic Acid, Sodium Salt (Formula Z-I: $Z_2$ is 3-pyridyl, $X_1$ is $-CH_2-$, and is meta to the oxygen, $R_9$, $R_{12}$, $R_2$ are hydrogen, m is zero, and $R_7$ is -COONa)

A solution of the compound of Example 23 (0.500 g, 0.00169 mole) in methanol (20 ml) and aqueous 0.10 N sodium hydroxide (17.5 ml, 0.00175 mole) are stirred at room temperature for six hours. Removal of solvent under reduced pressure give a pale yellow, solid residue (0.433 g recovered from flask).

The NMR ($D_2O$, δ) spectrum reveals peaks at 8.27, 6.70-7.42, 3.57, and 2.37.

Preparation A-22 3-[(3'-Benzyloxyphenyl)hydroxymethyl]pyridine

Refer to Chart K (analogous to the conversion of XCVIII to CI).

A solution of n-butyllithium (62.5 ml of a 1.6 M solution of THF, 0.10 mole) in ether (125 ml) is stirred and cooled to -78° C. To this is added dropwise over a 45-min time period a solution of 3-bromopyridine (15.8 g, 9.64 ml, 0.10 mole) in ether (125 ml). A light yellow solid precipitates from solution. The mixture is stirred and the temperature kept at -78° while adding dropwise over a period of 20 min a solution of 3-benzyloxybenzaldehyde (21.22 g., 0.10 mole) in ether (250 ml, incompletely dissolved), and 25 ml THF. Stirring is continued while allowing the bath and reaction mixture to warm to room

temperature. Thin layer chromatography (30% acetone-hexane, 40% ethyl acetate-hexane) of a sample quenched in water and extracted with ether shows starting aldehyde remaining at 15 min and 2 hr but completely gone after the reaction is stirred at room temperature overnight. The rection mixture is poured into water and extracted twice with ether (500 ml). The combined ether layers are washed with brine, dried ($mgSO_4$), filtered, and concentrated. The residue is chromatographed over silica gel (500 g) packed as a slurry in 25% acetone-hexane. The column is eluted with acetone-hexane in proportions increasing from 25% to 50% of acetone. Fractions of 350 ml are collected and those containing the product (detected by TLC) are pooled. The crystalline product is recrystallized from acetone-hexane, giving a first crop of 17.785 g, melting point of 86-88° C, a second crop of 2.025 g, melting point of 87-88° C, and a third crop of 1.525 g (total 21.335 g, 0.073 mole, 73%). A sample of the first crop is recrystallized for analysis. The IR (Nujol) spectrum reveals peaks at 3167, 1600, 1593, 1579, 1258, 1082, 1070, 1023, 799, 740, 718, 699, and 694 $cm^{-1}$.

The NMR ($CDCl_3$, δ) spectrum reveals peaks at 8.53, 8.37, 6.8-7.9, 5.78 and 5.03.

The C:H:N ratio is 77.89:5.96:4.68.

Preparation A-23 3-(3-Pyridylmethyl)phenol

Refer to Chart R (conversion of CLV to CLVI).

A solution of Preparation A-22 (2.91 g, 0.010 mole) in absolute ethanol (150 ml) containing 5.75 ml of 2 N HCl and 10% palladium on carbon (2.91 g) is shaken on a parr apparatus with hydrogen for 5-1/4 hr. TLC (75% ethyl acetate-hexane) shows the starting compound to be completely consumed. Catalyst is removed by filtration through a sintered glass funnel and sodium bicarbonate (solid, 1.25 g) is added to the filtrate. Solvent is removed under reduced pressure. The residue is worked up with ethyl acetate (200 ml) and water (15 ml). The aqueous layer is separated and extracted again with ethyl acetate. The combined ethyl acetate layers are washed with water, dried ($Na_2SO_4$), filtered, and concentrated, giving an oil (1.55 g). The oil is chromatographed over silica gel (70 g) packed as a slurry in 40% acetone-hexane. The oil is dissolved in $CH_2Cl_2$ for application to the column and the column is eluted with 40% acetone-hexane. Fractions of 125 ml volume are collected. The desired phenol (0.782 g, 0.042 mole, 42%) is obtained in fractions 4-6. The crystalline product is

recrystallized twice from acetone-hexane, giving the titled product as colorless crystals, melting point of 117.5-118.5° C. The IR (Nujol) spectrum reveals peaks at 3038, 2716, 2623, 1586, 1485, 1480, 1427, 1283, 1192, 1152, 1047, 873, 778, and 748 $cm^{-1}$. The NMR ($CDCl_3$, δ) spectrum reveals peaks at 8.41, 7.58, 6.60-7.25, and 3.90. The mass spectrum reveals ions at m/e 185. The C:H:N ratio is 77.86:6.33:7.55.

Preparation A-24 2-Formyl-5-(3-pyridylmethyl)phenol and 4-Formyl-3-(3-pyridylmethyl)phenol

Refer to Chart B (conversion of XXIII to XXIV).

Using the conditions described above, the phenol of Preparation A-23 (2.75 g, 0.0148 mole) is reacted with hexamethylenetetramine (2.18 g, 0.0155 mole) dissolved in trifluoroacetic acid (20 ml). Following work-up, there is obtained 2.75 g of a yellow oil which is chromatographed over slica gel (two Merck size B Lobar columns). The material is applied to the columns in $CH_2Cl_2$ solution and the columns are eluted with 30% acetone-hexane. Fractions of 25 ml volume are collected. Eluted first in fractions 41-42 was 4-formyl-3-(3-pyridylmethyl)phenol, (0.054 g, 0.00025 mole, 1.7%). The NMR ($CDCl_3$, δ) spectrum reveals peaks at 10.31, 8.54, 6.75-7.70, and 4.34.

The desired titled product (0.677 g, 0.00313 mole, 21%) is obtained in fractions 44-59. The crystalline material is recrystallized twice from acetone-hexane, giving the titled product as colorless crystals, melting point of 75-76° C. The NMR ($CDCl_3$, δ) spectrum reveals peaks at 9.97, 8.58, 6.70-7.65, and 4.00. The IR (Nujol) spectrum reveals peaks at 2400-3100, 1680, 1612, 1595, 1583, 1315, 1253, 1033, 821, 748, and 710 $cm^{-1}$. The mass spectrum reveals ions at m/e 213. The C:H:N ratio is 72.81:5.51:6.44.

Example A-23 Ethyl 6-(3-pyridylmethyl)benzofuran-2-carboxylate (Formula A-I: $Z_2$ is 3-pyridyl, $X_1$ is $-CH_2-$, and is meta to the oxygen, $R_9$, $R_{12}$, $R_2$ are hydrogen, m is zero, D is a double bond, and $R_7$ is $-COOCH_2CH_3$)

Refer to Chart A (conversion of X to XV).

Using the procedure described above a solution of the phenol-aldehyde of Preparation A-24 (0.776 g, 3.64 mmol) in toluene (60 ml), second, is added to a solution of diethyl bromomalonate in toluene (5 ml) and, third, dicyclohexyl-18-crown-6 (8 drops) to a stirred mixture of sodium hydride (0.393 g of a 59.6% suspension of NaH in mineral oil or 0.234 g of NaH, 9.75 mmol) in toluene (5 ml). The reaction is

complete after stirring 90 hr at room temperature and, after work-up, there is obtained 1.17 g of crude product. This material is chromatographed over silica gel (one Merck size B Lobar column) using methylene chloride for application to the column and 25% acetone-hexane for elution. Fractions of 25 ml are collected and the desired titled product (0.664 g, 2.36 mmol, 65%) is obtained in fractions 27-33. The crystalline product is recrystallized twice from acetone-hexane, giving an analytical sample as colorless crystals, melting point of 90-91.5°C. The NMR ($CDCl_3$, δ) spectrum reveals peaks at 8.54, 7.10-7.70, 4.44, 4.10, and 1.40. The IR (Nujol) spectrum reveals peaks at 1726, 1621, 1563, 1480, 1371, 1325, 1299, 1219, 1190, 1147, 1098, 768, 756, 747, and 717 $cm^{-1}$. The mass spectrum reveals ions at m/e 281.1061. The C:H:N ratio is 72.21:5.36:4.85.

Example A-24 6-(3-Pyridylmethyl)benzofuran-2-carboxylic Acid, Sodium Salt (Formula A-I: $Z_2$ is 3-pyridyl, $X_1$ is $-CH_2-$, and is meta to the oxygen, $R_9$, $R_{12}$, $R_2$ are hydrogen, m is zero, and $R_7$ is COONa) (Sodium salt of Example A-23)

A solution of ester of Example A-23 (0.397 g, 1.41 mmol) in methanol (15 ml) and 0.10 N sodium hydroxide (14.1 ml, 1.41 mmol) is stirred at room temperature for 4 hr. TLC (50% acetone-hexane) indicates complete saponification of the ester. The solvent is removed under reduced pressure leaving a white solid. The solid is dissolved in distilled water (4 ml) and reprecipitated with the addition of acetone (250 ml). The solid, melting point greater than 280° C (10, 0.0297 g, 1.08 mmol, 76%) is collected by filtration and dried over $P_2O_5$. The IR (Nujol) spectrum reveals peaks at 1601, 1587, 1560, 1255, 1191, 846, 803, 789, and 715 $cm^{-1}$. The NMR ($D_2O$, δ) spectrum reveals peaks at 8.22, 6.75-7.65 and 3.80.

Example A-25 6-(3-Pyridylmethyl)benzofuran-2-carboxylic Acid (Formula A-I: $Z_2$ is 3-pyridyl, $X_1$ is $-CH_2-$, and is meta to the oxygen, $R_9$, $R_{12}$, $R_2$ are hydrogen, m is zero, and $R_7$ is COOH) (Free acid of Example A-23)

A sample of the compound of Example A-24 (0.055 g, 0.00020 mole) is dissolved in water (0.7 ml). Aqueous 1.0 N hydrochloric acid (0.20 ml, 0.00020 mole) is added and the resulting white precipitate is collected by filtration, washed with water (1 ml) on the filter, and dried overnight in a vacuum dessicator, yielding 0.044 g (0.000174 mole, 87%), of product with a melting point of 222-225° C. Recrystallization from acetone yields crystals as colorless prisms, melting point of 224-226° C. The IR (Nujol) spectrum reveals peaks at 2382, 1713, 1294, 1230, 1187, 1098, 1051, 830, 795 $cm^{-1}$. The C:H:N ratio is 70.91:4.31:5.59.

<u>Preparation A-25</u> 2-(2-Methoxyethenyl)-4-(3-pyridinylmethyl)-phenol

Refer to Chart H (conversion of LXXV to LXXVI).

Sodium hydride (395 mg of the 60% dispersion, 9.87 mmoles) is washed twice with 5 ml of dry hexane under nitrogen to remove the mineral oil. Then 8.5 ml of dry dimethylsulfoxide is added, and the stirred suspension is heated at 65° for 2 hr. The grey, homogeneous solution is cooled to 25° and treated, in one portion, with 3.38 g (9.87 mmoles) of methoxymethyltriphenylphosphonium chloride. The dark red solution is stirred for 30 min at 25°, then treated dropwise (via addition funnel) with a solution of 195 mg (0.92 mmole) of the aldehyde corresponding to the titled product in 2 ml of dimethylsulfoxide. The reaction mixture is stirred for 30 min at 25°, then poured with ice/brine/pH 7 buffer and extracted with ethyl acetate. The extracts are washed with brine, dried over anhydrous sodium sulfate, and evaporated in vacuo.

The crude product is chromatographed on an 80 g column of silica gel, packed and eluted (7 ml fractions) with ethyl acetate. Fractions 42-50 are homogeneous by TLC, and, upon combination, yield 163 mg (74% of theory) of pure enol ether. The product, which crystallizes spontaneously, is triturated with cold ethyl acetate, filtered, and dried under vacuum at 25° for 3 hr. The recrystallized enol ether weighed 95 mg and exhibited melting point 133-135° C. (The mother liquors, 65 mg, crystallize readily and are still completely clean by TLC.) Later fractions (51-55) contain a small amount of additional enol ether, contaminated with an unknown, more polar, UV-absorbing impurity.

The IR ($\nu$max, mull) spectrum of the title compound reveals peaks at 1647, 1604, 1595, 1580, 1506, 1440, 1428, 1406, 1377, 1259, 1250, 1212, 1189, 1148, 1115, 1090, 1049, 805, 707, and 635 $cm^{-1}$.

The NMR ($CDCl_3$, $\delta$) spectrum reveals peaks at 8.65-8.25, 7.70-6.70, 6.08, 6.00, 5.36, 5.27, 3.88, and 3.82.

The mass spectrum reveals ions at m/e 241.1129, 226, 210, 198, 180, 167, 154, 131, 115, and 92.

<u>Example A-26</u> 5-(3'-Pyridinylmethyl)benzofuran (Formula A-I: $Z_2$ is

3-pyridinyl, $X_1$ is $-CH_2-$ and is para to the oxygen, $R_9$, $R_{12}$, $R_2$, and $R_7$ are hydrogen, D is a double bond, and m is zero)

Refer to Chart H (conversion of LXXVI to LXXVII).

A solution of 310 mg of enol ether in 10 ml of tetrahydrofuran is diluted with 150 ml of ether, and the resulting cloudy suspension is treated with 1 ml of concentrated perchloric acid. The vigorously stirred reaction mixture remains a suspension for a short time; then a yellow gum precipitates onto the walls of the flask. The mixture is partitioned between brine/sodium bicarbonate and ethyl acetate. The ethyl acetate layer is washed with brine, dried over anhydrous sodium sulfate and evaporated, yielding 325 mg of a viscous, pale yellow oil. TLC of the crude product indicates a 1:1 mixture of enol ether and the corresponding aldehyde. The crude product is re-subjected to the same reaction conditions as described above. This time, after 1 hr, TLC shows that both the enol ether and the aldehyde have been largely converted to the titled benzofuran. An additional 30 ml tetrahydrofuran and 10 ml of 2M perchloric acid were added and stirring is continued at 25° for 90 min. TLC analysis of an aliquot (1:1 acetone/hexane) shows that the benzofuran is still the major product. Workup under conditions described above yields 300 mg of crude benzofuran, which is chromatographed on a column containing 20 g of silica gel. The column is packed and eluted (2 ml fractions) with 1:1 acetone/hexane.

Fractions 32-42 are combined and give 125 mg of pure titled benzofuran as a viscous, colorless oil which crystallizes in the refrigerator and melts just below room temperature.

The IR (νmax, neat) spectrum reveals peaks at 1576, 1536, 1469, 1423, 1331, 1264, 1197, 1126, 1110, 1028, 884, 812, 769, 753, 735, and 713 $cm^{-1}$.

The NMR ($CDCl_3$, TMS, δ) spectrum reveals peaks at 8.60-8.25, 7.65-6.90, 6.70-6.55, and 4.06.

The mass spectrum reveals ions at m/e 209, 180, 166, 152, 131, 102, 90, 77, and 63.

<u>Preparation A-26</u> 3-(4-Nitrophenyl)pyridine

Refer to Chart B (conversion of XX to XXI).

3-Phenylpyridine (25.0 g, 0.161 mole) is added carefully and with cooling (ice-bath) to 70% nitric acid (140 ml). The resulting

solution is stirred and, when TLC (1:1 acetone-hexane) shows no reaction after 24 hr, it is heated in a 70° C oil bath. Some reaction is observed by TLC but it is very slow and so, after 4 days, concentrated sulfuric acid (50 ml) is added. The mixture is stirred over several days at room temperature after which the starting material is entirely consumed. The solution is poured onto ice and the mixture is made alkaline by the cautious addition of 50% aqueous NaOH. The mixture is extracted 3 times with ethyl acetate. (650 ml), the combined organic layers are washed with brine, dried over $MgSO_4$, filtered, and reduced in volume to 400 ml. Crystallization occurred, giving a first crop of 13.139 g of titled product, melting point of 145-148° C and, from the concentrated filtrate, a second crop of 4.16 g (total 17.299 g, 0.0865 mole, 53%) of product, melting point 143-147° C.

Preparation A-27 3-(4-Aminophenyl)pyridine

Refer to Chart B (conversion of XXI to XXII).

A mixture of nitropyridine, Preparation A-26 (16.799 g, 0.084 mole) with 5% palladium on carbon (1 g) in absolute ethanol (500 ml) is shaken with hydrogen on a Parr hydrogenation apparatus for 1 hr during which time the hydrogen uptake ceased. TLC (1:1 acetone-hexane) shows only a new, more polar spot. The catalyst is removed by filtration, the solvent is removed under reduced pressure, and the crystalline residue is recrystallized from methylene chloride-hexane, giving the titled product as a first crop, 8.470 g, melting point 119-120° C; a second crop, 4.635 g, melting point 117-119° C; and a third crop, 0.980 g (total 14.085 g, 0.0827 mole, 98%), melting point 113-117° C.

Preparation A-28 4-(3-Pyridyl)phenol

Refer to Chart B (conversion of XXII to XXIII).

Using the procedure described above, 3-(4'-aminophenyl)pyridine (10.0 g,0.059 mol) is converted to the phenol. A total of 7.66 g of product is obtained, after chromatography, which is then recrystallized from acetone-hexane to give 6.52 g (65% of theory) of off-white crystals which have as melting point of 202-203° C.

The C:H:N ratio is 76.71:5.42:8.13; when repeated, it is 76.76:5.30:8.18.

The IR (mull) spectrum reveals peaks at 2688, 2652, 2624, 2455, 1608, 1595, 1583, 1525, 1292, 1274, 1251, 1180, 842, 830, and 812

$cm^{-1}$.

The high resolution mass spectrum yields ions at m/e 171.0690, 154, 142, and 115.

Preparation A-29 2-Formyl-4-(3-pyridyl)phenol

Refer to Chart B (conversion of XXIII to XXIV).

The formylation procedure described above is followed using the phenol-pyridine of Preparation A-28 (5.844 g, 0.0342 mole) with hexamethylenetetramine (5.3 g) in trifluoroacetic acid (60 ml). Following work-up, the crude product is chromatographed over silica gel (200 g) packed as a slurry in 40% acetone-hexane. The crude product is applied to the column in 75% acetone-hexane and the column is eluted with 40% acetone-hexane. Fractions of 200 ml volume are collected. Fractions 4-10 contain the major product and are pooled. Crystallization from acetone-hexane gives a first crop of 2.090 g, melting point 111-113° C, and a second crop of 0.533 g, melting point of 111-120° C. TLC (1:1 acetone-hexane) of the second crop shows the presence of two components. The first crop is dissolved in acetone and after addition of hexane was filtered to remove a cloudy precipitate. The filtrate is cooled in a freezer, giving light yellow crystals, melting point 122-123° C. An additional recrystallization from acetone-hexane gives an analytical sample of the titled product as very pale yellow crystals with a melting point of 123-124° C. The IR (Nujol) spectrum reveals peaks at 2567, 1674, 1608, 1308, 1290, 1259, 1241, 1196, 1166, 1117, 1034, and 810 $cm^{-1}$. The NMR ($CDCl_3$, $\delta$) spectrum reveals peaks at 10.03, 8.86, 8.64, and 7.95-7.05.

The C:H:N ratio is 72.27:4.59:703.

Example A-27 Ethyl 5-(3-pyridyl)-2-benzofurancarboxylate (Formula A-I, $Z_2$ is 3-pyridyl, $X_1$ is a valence bond (i.e., n is zero) and is para to the oxygen, $R_9$, $R_{12}$, and $R_2$ are hydrogen, D is a double bond, and $R_7$ is $-COOCH_2CH_3$)

Refer to Chart A (conversion of X to XV).

Using sodium hydride (1.01 g of 59.6% sodium hydride dispersion, 25 mmol NaH), diethylbromomalonate (2.53 g, 10.6 mmol), and dicyclohexyl-18-crown-6 (24 drops), the phenol aldehyde of Preparation A-29 (1.80 g, 9.6 mmol) is converted to the benzofuran by the procedure described above. A total of 1.58 g (61% of theory) of the product is obtained as a solid after chromatography on silica gel using 20% acetone-hexane. The product is recrystallized from acetone-

hexane twice to give 1.33 g, melting point of 91-92° C.

Anal. Calcd. for $C_{16}H_{13}NO_3$: C, 71.90; H, 4.90; N, 5.24. The C:H:N ratio is 71.91:4.88:5.27.

The mass spectrum reveals ions at m/e 267.0888, 239, 222, 195, and 166.

The IR (Nujol) spectrum reveals peaks at 1723, 1567, 1312, 1221, 1173, 950, 882, 844, 831, and 812 $cm^{-1}$.

The NMR ($CDCl_3$, δ) spectrum reveals peaks at 8.87, 8.63, 7.67. 4/46. amd 1.44.

<u>Example A-28</u> Sodium 5-(3-pyridyl)-2-benzofurancarboxylate (formula A-I, $Z_2$ is 3-pyridyl, $X_1$ is a valence bond and is para to the oxygen, $R_9$, $R_{12}$, and $R_2$ are hydrogen, D is a double bond, and $R_7$ is -COONa) (The sodium salt of Example A-27)

A solution of ethyl 5-(3-pyridyl)-2-benzofuran carboxylate (1.21 g, 4.53 mmol) in methanol (15 ml) is treated with a solution of 5 ml of 1N NaOH and 10 ml of water. The reaction is stirred at room temperature for 16 hr and found to be complete by TLC evidence. The methanol-water is removed under reduced pressure to give a white solid. The solid is recrystallized twice from water-acetone to give 0.928 g, melting point of greater 290° C.

The IR (mull) spectrum reveals peaks at 3493, 3380, 1633, 1609, 1513, 1574, 1256, 1168, 1131, 944, 880, and 809 $cm^{-1}$.

The mass spectrum reveals ions at m/e 195 and 44.

The NMR ($D_2O$, δ) spectrum reveals peaks at 8.25 and 7.30.

The C:H:N ratio is 58.03:3.07:4.84; when repeated it is 58.60:3.33:4.73.

<u>Example A-29</u> Ethyl 5-[(3'-pyridinyl)aminomethyl]-benzofuran-2-carboxylate and Ethyl 4-[(3'-pyridinyl)aminomethyl]-benzofuran-2-carboxylate (Formula A-I: $Z_1$ is 3-pyridinyl, $X_1$ is $-NHCH_2-$ and is para to the oxygen, $R_2$, $R_9$ and $R_{12}$ are hydrogen, D is a double bond, m is zero, $Y_1$ is -O-)

Refer to Chart K (conversion of XCVIII to C).

Part A.

A 200 ml 1-neck (24/40) round bottomed flask is charged with 1.75 g (8.02 mmol) of the aldehyde mixture of Preparation C-3, 3-aminopyridine (0.93 g, 9.8 mmol), p-toluenesulfonic acid (0.092 g,

0.48 mmol) and benzene (50 ml). The flask is fitted with a Dean-Stark trap and a condenser and the mixture is heated to reflux and stirred fro 4 hr. Little change is observed by TLC after 2 hr. The mixture is cooled, filtered through a Celite pad and the filtrate is concentrated under reduced pressure to give a yellow oil.

Part B.

The oil is dissolved in 25 ml of mthanol, placed under a positive nitrogen atmosphere, cooled to -25° C and treated with 0.36 g (9.5 mmol) of sodium borohydride. After stirring at -25° C for 20 min the reaction is quenched by the addition of saturated aqueous ammonium chloride and the methanol is removed under reduced pressure. The concentrate is diluted with 25 ml of saturated aqueous sodium bicarbonate and extracted with 100 ml of chloroform. The organic phase is washed with brine, (25 ml) dried ($MgSO_4$), filtered, and concentrated to give 2.60 g of crude product as a yellow oil.

This material is chromatographed on 192 g of HPLC grade silica gel, eluting with hexane-ethyl acetate (7:3) (+5% ethanol) and collecting 45 ml fractions. Fractions 37-46 are homogeneous by TLC and are combined and concentrated to give 0.48 g of the 7-pyridinylaminomethyl product as a pale yellow oil which solidifies. This material is recrystallized from chloroform/hexane to give a white grannular solid with melting point of 128.5-129. Fractions 47-51 contain a mixture of both isomers (0.22 g) and fractions 52-80 afford 1.46 g of the 5-pyridinylaminomethyl product as a white solid. This material is recrystallized from chloroform/hexane to give white needles with a melting point of 116-117° C. The combined yield is 91%. For the 4-pyridinylamino compound, spectral data is as follows:

The NMR ($CDCl_3$; TMS, δ) spectrum reveals peaks at 8.27-6.80, 5.19, 4.53, 4.37, and 1.38.

The IR (Nujol, νmax) spectrum reveals peaks at 3251, 1736, 1613, 1591, 1559, 1536, 1484, 1422, 1329, 1292, 1251, 1237, 1184, 1157, 1105, 1020, 846, 792, 763, 743, and 702 $cm^{-1}$.

The C:H:N ratio is 68.29:5.42:9.65.

The mass spectrum reveals ions at m/e 291.1170, 203 and 175.

For the 5-pyridinylamine product, spectral data is as follows:

The NMR ($CDCl_3$, δ) spectrum reveals peaks at 8.20-6.82, 4.60-4.30, and 1.42.

The IR (Nujol, νmax) spectrum reveals peaks at 3239, 1726, 1714,

1591, 1581, 1531, 1420, 1346, 1295, 1231, 1220, 1200, 1141, 1123, 1089, 1026, 952, 896, 845, 799, 791, 764, 748, 705, 660 and 627 $cm^{-1}$.

The C:H:N ratio is 68.75:5.41:9.27.

The mass spectrum reveals ions at m/e 296.1156, 203, and 175.

Example A-30 Sodium 5-[(3'-pyridinyl)aminomethyl]-benzofuran-2-carboxylate (Formula A-I: $Z_1$ is 3-pyridinyl, $X_1$ is $-NHCH_2-$ and is para to oxygen, $R_2$, $R_9$ and $R_{12}$ are hydrogen, D is a double bond, m is zero, $Y_1$ is -O-, and $R_7$ is -COONa)

The ethyl ester staring material (Example A-29) (0.927 g, 3.11 mmol) is dissolved in 15 ml of absolute ethanol. Sodium hydroxide reagent (1.0 N, 3.11 ml, 3.11 mmol) is added and the resulting solution is stirred at room temperature for 2 hr at which time TLC analysis indicates no remaining starting material. Ethanol is removed under reduced pressure and the concentrate is diluted with 100 ml of water, filtered through a cotton plug, frozen and lyophilized to give 0.776 g of product as a pale yellow granular solid.

Example A-31 Sodium 4-[(3'-pyridinyl)aminomethyl]-benzofuran-2-carboxylate (Formula A-I: $Z_1$ is 3-pyridinyl, $X_1$ is $-NHCH_2-$ and is meta to the oxygen, $R_2$, $R_9$ and $R_{12}$ are hydrogen, D is a double bond,m is zero, $Y_1$ is -O-, and $R_7$ is -COONa)

The ethyl ester starting material (Example A-29) (0.208 g, 0.7 mmol) is dissolved in 10 ml, 0.700 ml, 0.7 mmol) is added and the resulting solution is stirred at room temperature for 1.5 hr. After this time TLC analysis still indicates unreacted starting material. Another 0.070 ml of sodium hydroxide reagent is added and the mixture (a precipitate had formed approximately 1 hr after the start of the hydrolysis) is stirred at room temperature for 65 hr a which time TLC analysis confirms the hydrolysis is complete. Ethanol is removed under reduced pressure and the concentrate is diluted with 50 ml of water, lyophilized to give 0.169 g of product as a white fluffy powder.

Example A-32 Ethyl 5-[(3'-pyridinyl)hydroxymethyl]-benzofuran-2-carboxylate and Ethyl 4-[(3'-pyridinyl)-hydroxymethyl]-benzofuran-2-carboxylate (Formula A-I: $Z_1$ is 3-pyridinyl, $X_1$ is -CH(OH)- and is para or meta to the oxygen, $R_2$, $R_9$ and $R_{12}$ are hydrogen, D is a double

bond, m is zero, and $R_7$ is $-COOCH_2CH_3$)

Refer to Chart K (conversion of XCVIII to CI).

A 500 ml 3-neck (24/40) round bottomed flask, equipped with a nitrogen inlet and a mechanical stirer, is charged with 2.12 ml (22.1 mmol) of 3-bromopyridine ethyl ether (200 ml, dried over molecular sieves) is added and the resulting solution was cooled to -78° C under a nitrogen atmosphere. n-Butyllithium (15 ml, 23 mmol) is added to the stirred solution via syringe over 2-3 min to give a pale yellow milky suspension. After stirring for 30 min, a solution of the aldehdye mixture (Preparation C-3) in 25 ml of anhydrous ether is added in one portion (approximately 2 ml of tetrahydrofuran is added initially to the aldehyde mixture to get it to dissolve). A dull tan colored milky suspension results and is stirred at -78° C for one hr. Saturated aqueous ammonium chloride (25 ml) is added to quench the reaction. the mixture is allowed to warm to room temperature and ether is removed under reduced pressure. the concentrate is diluted with chloroform and washed twice with brine (100 ml), dried ($MgSO_4$), filtered and concentrated to give 2.77 g of crude product as an orange colored oil.

This material is chromatographed on 192 g of HPLC grade silica gel, eluting with hexane-ethyl acetate (7:3) (+5% ethanol), collecting 40 ml fractions. Fractions 53-62 are homogeneous by TLC and are combined and concentrated to give 0.194 g of the 7-pyridinyl product as a yellow oil. Similarly fractions 63-67 afford 0.071 g of a mixture and fractions 68-103 afford 0.837 g of the 5-pyridinyl product as a yellow oil. The NMR spectrum of the title product indicates some impurities present and the material is rechromatographed over 6.7 g HPLC grade silica gel, eluting with hexane-acetone (4:1) and collecting 10 ml fractions. The fractions are combined and concentrated to give 0.180 g of pure 4-pyridinyl product, with a combined yield of 51%. Spectral data for this product is as follows:

The NMR ($CDCl_3$; TMS, $\delta$) spectrum reveals peaks at 8.57-7.10, 6.11, 5.70, 4.39 and 1.38.

The IR (film, $\nu$max) spectrum reveals peaks at 3152, 2984, 1724, 1611, 1594, 1571, 1562, 1478, 1425, 1393, 1372, 1329, 1309, 1261, 1183, 1154, 1104, 1070, 1040, 1028, 1020, 979, 951, 856, 791, 763, 713, 666 and 633 $cm^{-1}$.

The mass spectrum reveals ions at m/e 297.1020, 280, 252, 224,

196, 191, 167, 145, 119, 106, 91, and 79.

For the 5-pyridinyl product, spectral data is as follows:

The NMR ($CDCl_3$; TMS, δ) spectrum reveals peaks at 8.55-7.04, 6.0, 5.90, 4.39, and 1.39.

The IR (film, νmax) spectrum reveals peaks at 2923, 2856, 1720, 1576, 1562, 1464, 1456, 1373, 1347, 1322, 1294, 1268, 1229, 1192, 1140, 1120, 1094, 1063, 1040, 1027, 1019, 945, 937, 893, 842, 810, 766, 748, 716, 690, 679, 649, 632, and 610 $cm^{-1}$.

The mass spectrum reveals ions at m/e 297.1014, 280, 268, 252, 224, 219, 191, 119, 106, and 91.

The C:H:N ratio is 68.43:5.12:4.83.

Example A-33 Sodium 5-[(3'-pyridinyl)hydroxymthyl]-benzofuran-2-carboyxlate (Formula A-I: $Z_1$ is 3-pyridinyl, $X_1$ is -CH(OH)- and is para to oxygen, $R_2$, $R_9$ and $R_{12}$ are hydrogen, D is a double bond, m is zero, $Y_1$ si -O-, and $R_7$ is -COONa)

The ethyl ester starting material (Example A-32) (0.345 g, 1.16 mmol) is dissolved in 10 ml of absolute ethanol. Water (2 ml) and 1.15 ml (1.15 mml) of 1.00 N NaOH reagent are added and the resulting solution is stirred at room temperature for 18 hr at which time no starting material is evident by TLC. Ethanol is removed under reduced pressure and the concentrate is diluted with 150 ml of water, washed with ethyl ether, filtered through a cotton plug, frozen and lyophilized to give 0.303 g of product as a tan colored powder.

Example A-34 Sodium 4-[(3'-pyridinyl)hydroxymethyl]-benzofuran-2-carboxylate (Formula A-I: $Z_1$ is 3-pyridinyl, $X_1$ is -CH(OH)- and is meta to oxygen, $R_2$, $R_9$ and $R_{12}$ are hydrogen, D is a double bond, m is zero, $Y_1$ is -O-, and $R_7$ is -COONa)

The ethyl esters starting material (Example A-32) (0.117 g, 0.396 mmol) is dissolved in 5 ml of absolute ethanol. Water (0.5 ml) and 400 µl (0.400 mmol) of 1.0 N NaOH reagent are added and the resulting solution is stirred at room temperature for 6.5 hr at which time TLC analysis shows no remaining starting material. Ethanol is removed under reduced pressure and the concentrate is diluted with 50 ml of water, filtered through a cotton plug, frozen and lyophilized to give 0.113 g of product as a white fluffy powder.

Preparation A-30 3-(p-Methoxy)phenoxypyridine

Refer to Chart I (conversion of LXXX to LXXXII).

Following the procedure of Renshaw and Conn, J. Am. Chem. Soc. 59:297 (1937), a mixture of 10.95 g (0.166 mmol) of 85% potassium hydroxide pellets and 15.78 g (0.166 mmol) of 3-hydroxypyridine is heated to 200° C in a three-necked round bottom flask equipped with a thermometer, distillation apparatus and air stirrer. The mixture first liquifies, then forms green crystals, presumably the potassium salt of 3-hydroxypyridine. The temperature is reduced to 150° C and another 10 g (0.105 mmol) of 3-hydroxypyridine is added followed by 20.78 ml (0.166 mmol) of p-bromoanisole and 0.33 g of activated copper bronze (see below). The resulting mixture is heated to 200° C and stirred for 2 hr. The brown solution is then cooled to 100° C and poured into 300 ml of water. The solution becomes homogeneous (and black) after adding 39 g of 45% aqueous potassium hydroxide. The solution is transferred to a separatory funnel and the pH is adjusted to approximately 13. The final volume is 600 ml. Ethyl ether (8 L) is used to "extract" the compound: 500 ml of ether is added to the water layer and shaken, which results in an emulsion. The top 500 ml of the emulsion is extracted with ether until an organic layer can be discerned. The water layer after the ether extractions is then extracted with methylene chloride (4 L). The combined methylene chloride layers are washed with 500 ml of a pH = 14 potassium hydroxide solution. The methylene chloride extracts and concentrate from the ether layers are combined and filtered through 3 inches of silica gel, thereby removing the maroon color. The organic layer is dried over magnesium sulfate and concentrated in vacuo to yield 13.3 g of residue which is distilled at 112° C and 0.1-0.3 mm hg (lit. top 163-164°/10 mm) to yield 10.05 g of product. The 3-hydroxypyridine, distills at approximately 60° C and 0.1-0.3 mm Hg, and is therefore a slight contaminant (5%) in the final product.

The IR (νmax, mull) spectrum reveals peaks at 1230, 831, 1506, 1477, 1426, 1028, 709, 1261, 1103, 810, 824, 1191, 1199, 1574, and 1179 $cm^{-1}$. [Note: Continuous extraction of the water layer with methylene chloride would be much easier and more efficient than the laborious extraction procedure give above.]

The NMR ($CDCl_3$; TMS, δ) spectrum reveals peaks at 8.53-8.32, 7.74-6.82, and 3.80.

The mass spectrum reveals ions at m/e 201.0784, 202, 200, 188,

186, 173, 143, 123, 78, 77, 64, and 63.

TLC (silica gel GF) yields an $R_f$ of 0.11 (20% ethyl acetate/hexane).

Activation of Copper Bronze: A suspension is prepared by adding 10 g of copper bronze to 120 ml of a 2% (by weight) iodine/acetone solution. The combination is stirred for 5-10 min, filtered, and the residue resuspended in 75 ml of a 1:1 concentrated hydrochloric acid/acetone solution. After stirring for 5 min the solution is filtered and the residue is washed with 25 ml of the 1:1 concentrated hydrochloric acid/acetone solution followed by acetone (3 x 100 ml). The resulting solid is dried in a dessicator under a vacuum for 1 hr.

Preparation A-31 3-(p-Hydroxy)phenoxypyridine

Refer to Chart I (conversion of LXXXII to LXXXIII).

A solution of 9.0 g (44.7 mmol) of 3-(p-methoxy)phenoxypyridine and 145 ml of 48% of hydrogen bromide is heated to reflux under a nitrogen atmosphere for 1.5 hr. The solution is cooled and concentrated in vacuo to dryness after which water is added (approximately 80 ml) and the resulting solution slowly added to saturated sodium bicarbonate (300 ml). The light pink, pH approximately equal to 9 solution is extracted with ethyl acetate (6 x 250 ml). The pH is adjusted to approximately 7 and again extracted twice with ethyl acetate (250 ml). The combined ethyl acetate layers are dried over magnesium sulfate; filtered, and concentrated in vacuo to yield a pink residue which is recrystallized in ethyl acetate/hexane to yield 6.82 g of product (82% of theory) with a metling point range of 131-134° C.

The IR (νmax, mull) spectrum reveals peaks at 1232, 1507, 1427, 1260, 1482, 1198, 705, 843, 1575, 1457, 804, 627, 2677, 1110, and 3056 $cm^{-1}$.

The NMR ($CDCl_3$ and $d_6$-acetone; TMS) spectrum reveals peaks at $\delta$ 9.03-8.03, 8.52-8.27, 7.48-7.27, and 6.97.

The mass spectrum reveals ions at m/e 187.0633, 188, 186, 120, 109, 81, 79, 78, 65, and 51.

TLC (silica gel GF) yields an $R_f$ of 0.20 (40% ethyl acetate/hexane).

Preparation A-32 2-Hydroxy-5-(3-Pyridinyloxy)benzaldehyde

Refer to Chart B (conversion of XXIII to XXIV).

A mixture of 1.00 g (5.34 mmol) of 3-(p-hydroxy)phenoxypyridine

and 0.79 g (5.61 mmol) of hexamethylenetetramine in 10 ml of trifluoroacetic acid is heated to 80° C and is stirred under a nitrogen atmosphere for 4 hr. The reaction mixture is cooled to room temperature and the pH is adjusted to 7.5 with 50% aqueous sodium hydroxide, which results in an oily yellow precipitate. The solution is extracted with chloroform (3 x 250 ml, 1 x 100 ml) and the combined chloroform layers are dried over magnesium sulfate, filtered and concentrated in vacuo to yield 1.7 g of residue. The residue is chromatographed on 400 g of silica gel. The column is wet-packed and eluted with 6% acetone/methylene chloride and fractions of 20 ml are collected. Fractions 54-83 are combined and concentrated in vacuo to yield 0.51 g of product. The product is recyrstallized in ethyl acetate/hexane to give 0.31 g (27 % of theory) with a melting point of 122-124° C.

The IR (νmax (mull)) spectrum reveals peaks at 1672, 1232, 1446, 1441, 1246, 1430, 1479, 1276, 1267, 902, 1148, 806, 699, 1578, and 1382 $cm^{-1}$.

The NMR ($CDCl_3$; TMS, δ) spectrum reveals peaks at 9.97, 8.60-8.30, and 7.50-6.80.

The mass spectrum reveals ions at m/e 215.0582, 216, 214, 169, 137, 79, 78, 53, 51, and 39.

TLC (silica gel GF) yields an $R_f$ of 0.20 (6% acetone/methylene chloride).

Example A-35 5-(3-Pyridinyloxy)benzofuran-2-carboxylic acid, ethyl ester (Formula A-I: $Z_1$ is 3-pyridinyl, $X_1$ is -O- and is para to the oxygen, $R_9$, $R_{12}$, and $R_2$ are hydrogen, D is a double bond, m is zero, and $R_7$ is $-COOCH_2CH_3$)

To a mechanically stirred solution of 0.74 g (18.6 mmol) of sodium hydride (60% oil dispersion) in 60 ml of dry benzene under a nitrogen atmosphere is added 1.0 g (4.65 mmol) of hydroxy aldehyde of Preparation A-31 in one portion. The mixture is stirred for 15 min before adding 1.31 ml of diethyl bromomalonate in one portion. A spatula tip of dicyclohexano-18-crown-6-ether is added and the reaction is stirred at room temperature under a nitrogen atmosphere for 40 hr. The color changes from grey-green to dark yellow. The mixture is poured into 300 ml of 1:1 saturated brine/sodium bicarbonate solution, shaken, then extracted with ethyl acetate (3 x 250 ml). The ethyl acetate layers are dried over magnesium sulfate,

filtered, and concentrated in vacuo to yield 2.2 g of residue. The material is chromatographed on 220 g of silica gel. The column is wet-packed and eluted with 10% isopropanol/hexane and fractions of 20 ml were collected. Fractions 54-89 are combined and concentrated to yield 0.59 g of product. This product is recrystallized in ethyl ether/hexane at 0° C. The crystals are filtered and washed with cold hexane and dried in vacuo to yield 0.38 g of product (29% of theory) with a melting point pf 69-70° C.

The IR (νmax (mull)) spectrum reveals peaks at 1725, 1195, 1145, 1233, 1426, 1318, 1273, 1565, 1184, 1265, 1575, 1220, 1474, 1209, and 1102 $cm^{-1}$.

The NMR ($CDCl_3$; TMS, δ) spectrum reveals peaks at 8.63-8.30, 7.80-6.97, 4.67-4.23, and 1.67-1.20.

The mass spectrum reveals ions at m/e 283.0844, 284, 255, 254, 239, 238, 211, 182, 154, and 78.

TLC (silica gel GF) yields an $R_f$ of 0.24 (10% isopropanol/hexane).

<u>Example A-36</u> 5-(3-Pyridinyloxy)benzofuran-2-carboxylic acid, sodium salt (Formula A-I: $Z_1$ is 3-pyridinyl, $X_1$ is -O- and is para to the oxygen, $R_9$, $R_{12}$, and $R_2$ are hydrogen, D is a double bond, m is zero, and $R_7$ is -COONa)

A solution of 0.10 g (0.33 mmol) of ethyl ester of Example A-35 in 7 ml of methanol and 3.53 mol of 0.100 M sodium hydroxide is stirred at room temperature under a nitrogen atmosphere for 16 hr. The reaction mixture is filtered through a fine porosity funnel and the filtrate is concentrated in vacuo to dryness. The resulting residue is triturated with acetonitrile for 0.5 hr, filtered and the solids washed with cold acetonitrile. The solids are dried in vacuo to yield 63 mgs of product (68% of theory) with a melting point greater than 300° C.

The IR (νmax (mull)) spectrum reveals peaks at 1601, 1409, 1565, 1378, 1451, 1253, 1229, 1190, 791, 1476, 1334, 1272, 3029, 1145, 3056 $cm^{-1}$.

<u>Preparation A-33</u> 4-(3-Pyridinylthio)phenol and the corresponding methyl ether

Refer to Chart I (conversion of LXXX' to LXXXII' and LXXXIII).

A 1000 ml, 3-necked, round-bottomed flask, fitted with air stirrer, a condenser and nitrogen inlet, is flame-dried, then recooled in an atmosphere of nitrogen. A stirred, cooled (0°) solution of 14.0

g (100 mmol) of p-methoxythiophenol (freshly distilled) in 200 ml of hexamethylphosphoramide is treated with 17.62 g of a 22.7% oil dispersion of potassium hydride (100 mmol), added in portions over 10 min via a wide-mouthed pipet (with vigorous hydrogen evolution). After the potassium hydride addition is complete, the cooling bath is removed, and the orange mixture is stirred for 15 min at 25° (hydrogen evolution has ceased). 3-Bromopyridine (15.8 g, 100 mmol) is added without solvent, and the homogeneous brown mixture is stirred at 80° for 24 hr in a nitrogen atmosphere. The reaction mixture (very dark) is cooled to 5°, poured into 1:1 brine/water (1 ℓ), adjusted to pH 7 with 2M hydrochloric acid and extracted with ethyl acetate (5 x 400 ml). The extracts are washed with water (5 x 300 ml) and brine, dried over anhydrous magnesium sulfate and concentrated in vacuo.

The crude product (36 g) is chromatographed on 3 kg of silica gel. The column is packed with 10% acetone/methylene chloride, then eluted with 20 ℓ of 10%, 8ℓ of 20% and 8ℓ of 40% acetone/methylene chloride.

Fractions 30-69, combined based on their TLC homogenity, affords 6.81 g of title compound methyl ether (31% of theory), a pale yellow oil.

The IR (νmax, neat) spectrum reveals peaks at 3000, 2940, 2830, 1590, 1550, 1490, 1460, 1440, 1400, 1285, 1245, 1170, 1100, 1025, 1015, 825, 795, and 700 $cm^{-1}$.

The NMR ($CDCl_3$; TMS, δ) spectrum reveals peaks at 8.43-8.34, 7.48-6.84 and 3.82.

The mass spectrum reveals ions at m/e 217.0579, 202, 173, 131, 130, 96, 78, 63, 51, and 39.

TLC (silica gel GF) yields an $R_f$ of 0.40 (10% acetone/methylene chloride).

Fractions 109-128 from the above chromatogram are combined and yield 5.3 g of 4-(3-pyridinylthio)phenol (26% of theory). This material crystallizes readily and, after trituration with ether, affords white crystals with melting point 149-151°.

The IR (νmax, (mull)) spectrum reveals peaks at 3083, 3068, 2791, 2766, 2726, 2672, 1601, 1582, 1557, 1498, 1471, 1460, 1455, 1410, 1285, 1248, 1237, 1171, 1038, 1025, 836, 798, and 702 $cm^{-1}$.

The NMR ($CDCl_3$; TMS, δ) spectrum reveals peaks at 9.40, 8.35-8.06, and 7.41-6.75.

The mass spectrum reveals ions at m/e 203.0416, 186, 174, 159, 142, 125, 115, 97, 78, and 51.

TLC (silica gel GF) yields an $R_f$ of 0.19 (10% acetone/methylene chloride).

Demethylation of title compound methyl ether

A solution of 6.6 g of methyl ether (fractions 30-69 above) in 80 ml of concentrated hydrobromic acid is heated at reflux for one hr. The hydrobromic acid is then removed on the rotary evaporator (high vacuum pump, 40°). The residue was dissolved in 1:1 brine/water, adjusted to pH 7.0-7.2, and extracted thoroughly with ethyl acetate. The extracts were washed with brine, dried over anhydrous magnesium sulfate and concentrated. The crude product crystallizes spontaneously and is about 95% pure 4-(3-pyridinylthio)phenol by TLC. Recrystallization from acetone/methylene chloride, or from ether, does not substantially decrease the amount of two minor move polar impurities. The crude product is sufficiently clean to be used in subsequent reactions.

Preparation A-34 2-Hydroxy-5-(3-pyridinylthio)benzaldehyde

Refer to Chart B (conversion XXIII to XXIV).

A solution of 4.06 g (20 mmol) of 4-(3-pyridinylthio)phenol and 2.94 g (21 mmol) of hexamethylenetetramine in 40 ml of trifluoroacetic acid is heated at 80° under a nitrogen atmosphere for 5.5 hr. The mixture is cooled to 35° and concentrated in vacuo (rotovac- high vacuum pump). The residue is diluted with 150 ml of water and allowed to stand at 25° for 30 min. The pH of the aqueous mixture is then adjusted to 7.0, and the product is isolated by extraction with ethyl acetate. The extracts are washed with brine, dried over magnesium sulfate, and evaporated.

The crude product (6.5 g), mostly two components in comparable amounts, is chromatographed on a column containing 900 g of silica gel. The column is packed with 10% acetone/methylene chloride and eluted (1 x 300 ml, then 25 ml fractions) with 4 ℓ of 10%, 4 ℓ of 20% and 6 ℓ of 30% acetone/methylene chloride.

Fractions 98-169 were combined and yielded 1.99 g of the title aldehyde, homogeneous by TLC (43 % of theory). Recrystallization of a 200 mg sample from ethyl acetate/hexane gives 160 mg of a white solid with melting point of 115-117° C.

The IR (νmax (mull)) spectrum reveals peaks at 1680, 1673, 1592,

1579, 1560, 1491, 1475, 1425, 1413, 1393, 1301, 1234, 1175, 167, 1111, 1043, 800, 787, 697, and 638.

The NMR ($CDCl_3$; TMS, $\delta$) spectrum reveals peaks at 11.08, 9.98, 8.65-8.40 and 7.90-7.03. The mass spectrum reveals ions at m/e 231.0366, 203, 185, 174, 153, 142, 121, 111, 97, 79, 63, 51, and 39.

TLC (silica gel GF) yields an $R_f$ of 0.39 (15% acetone/methylene chloride).

Fractions 202-226 yield 180 mg of clean starting material (4%).

Fractions 346-400 from the above chromatogram are combined and afford 1.70 g of a colorless oil, whose identify remains uncertain. This by-product is completely stable to 5% trifluoroacetic acid in water and hence is likely not the methyl imine corresponding to the title aldehyde. (Such an intermediate can be isolated if hydrolysis conditions are too mild.) This material possesses no carbonyl band in the IR but has an unidentified singlet worth 2-3H at 4.05 ppm. (Still a phenol.)

<u>Example A-37</u> 5-(3-Pyridinylthio)-benzofuran-2-carboxylic acid, ethyl ester (Formula A-I: $Z_2$ is 3-pyridinyl, $X_1$ is -S- and is para to the oxygen, $R_9$, $R_{12}$, $R_2$ are hydrogen, D is a double bond, m is zero, and $R_7$ is $-COOCH_2CH_3$)

To a solution of 1.23 g (30.8 mmol) of sodium hydride (60% oil dispersion) in 80 ml of benzene under a nitrogen atmosphere with mechanical stirring is added 1.78 g (7.7 mmol) of hydroxyaldehyde from the preceding experiment in one portion, followed by 1.23 ml (7.2 mmol) of diethyl bromomalonate. A spatula tip of dicyclohexane-18-crown-6 is added and the reaction is stirred for 24 hr. After this time, another 1.23 ml (7.2 mmol) of diethyl bromomalonate is added and after 20 hr another 0.25 g (6.25 mmol) of sodium hydride (60% oil dispersion) is added to the reaction mixture. The reaction progress is periodically monitored by thin layer chromatography. After 3 days of stirring under nitrogen another 0.25 g (6.25 mmol) of sodium hydride (60% oil dispersion) is again added and after 30 hr the reaction mixture is poured into 250 ml of dry tetrahydrofuran and the resulting solution is stirred at room temperature under a nitrogen atmosphere for 36 hr. Another 0.2 g (5.0 mmol) of sodium hydride (60% oil dispersion) is added again and after 40 hr stirring under a nitrogen atmosphere the solution is concentrated in vacuo to remove 1/2 of the tetrahydrofuran. The resulting solution is poured into a

stirred brine/ice misture and the pH is continuously monitored and adjusted with dilute hydrochloric acid so as to keep it below 10. After complete addition of the reaction mixture the pH is adjusted with ethyl acetate (3 x 250 ml). The pH is changed to 7 and the solution is extracted with chloroform (250 ml). The combined ethyl acetate extracts are dried over magnesium sulfate, filtered and concentrated in vacuo. The residue is dissolved in the chloroform wash which is dried over magnesium sulfate, filtered and concentrated in vacuo to yield 4.7 g of residue. The residue is chromatographed on 325 g of silica gel. The column is wet-packed and eluted with 50% ethyl acetate/hexane and fractions of 30 ml were collected. Fractions 31-41 were combined and concentrated in vacuo to yield 0.67 g of oil products (29% of theory). The oil is dried under a vacuum for 16 hr. Part of the product which crystallized slowly on standing was recrystallized in diethyl ether/hexane to yield yellow crystals with a melting point range of 59-61° C.

The NMR ($CDCl_3$; TMS, δ) spectrum reveals peaks at 8.55-8.30, 7.85-7.00, 4.58-4.31, and 1.51-1.33.

The mass spectrum reveals ions at m/e 299.0616, 300, 271, 270, 254, 244, 226, 215, 198, 176, 148, 130, 120, 102, 92, 74, and 47.

TLC (silica gel GF) yields an $R_f$ of 0.36 (50% ethyl acetate/hexane).

Example A-38 5-(3-Pyridinylthio)-benzofuran-2-carboxylic acid, sodium salt (Formula A-I: $Z_1$ is 3-pyridinyl, $X_1$ is -CH(OH)- and is para or ortho to the oxygen, $R_2$, $R_9$ and $R_{12}$ are hydrogen, D is a double bond, m is zero, and $R_7$ is -COONa)

To a solution of 0.200 g (0.63 mmol, assumed 95% purity) of the ester of Example A-37 in 8 ml of tetrahydrofuran is added 6.34 ml of 0.700 M sodium hydroxide. The reaction mixture stirred under a nitrogen atmosphere for 3 days. The solution is concentrated to dryness using a vacuum pump and the resulting residue is triturated with 8 ml of acetonitrile for 4 hr. The crystals are filtered and washed with acetonitrile and then dried under vacuum to yield 0.144 g of product (77% of theory) with a melting point greater than 300° C.

Example A-39 5-[(3-Pyridinyloxy)methyl]-benzofuran-2-carboxylic acid, ethyl ester and 4-[(3-pyridinyloxy)methyl]-benzofuran-2-carboxylic acid, ethyl ester

Refer to Chart K (conversion of XCVII to CII).

A 50 ml two-necked, round-bottomed flask, fitted with nitrogen inlet and a magnetic stirrer, is flame-dried, then recooled, in a nitrogen atmosphere. The flask is charged with 220 mg (5.5 mmol) of sodium hydride (60% dispension in oil), and the oil is removed with two 5 ml hexane washes. Anhydrous dimethylformamide (10 ml) is added, and the stirred suspension is treated with a solution of 475 mg (5.0 mmol) of 3-hydroxypyridine in 2 ml of dimethylformamide, added over about 5 min (hydrogen evolution). The reaction mixture is heated to 80° for 10 min, then recooled to 25° and treated with a solution of 1.2 g (5.0 mmol) of a 3:1 mixture of 5-(chloromethyl)-benzofuran-2-carboxylic acid, ethyl ester and the corresponding 4-(chloromethyl) isomer in 2 ml of dimethylformamide. The reaction mixture is stirred for 3 hr at 25° (sodium chloride begins precipitating almost immediately). The reaction mixture is poured into 1:1 brine/water and extracted with methylene chloride (3 x 300 ml). The extracts are washed with water (three times), and brine, dried over anhydrous magnesium sulfate and filtered. Prior to concentration, the light yellow solution is allowed to stand overnight at 25° in a stoppered flask, during which time the solution becomes dark purple. TLC analysis of the dark product shows no difference from the yellow solution 18 hr earlier, with the exception of a purple spot which remains at the origin.

The crude product (1.6 g) is chromatographed on a column containing 285 g of 40-60 μ silica gel. The column is eluted with 2 ℓ of 10% isopropyl alcohol/hexane, then with 2 ℓ of 15% isopropyl alcohol/hexane (25 ml fractions).

Fractions 83-98 are combined and afford 67 mg of a pale yellow oil:

The IR (νmax (neat)) spectrum reveals peaks at 1720, 1570, 1475, 1420, 1370, 1320, 1295, 1220, 1180, 1095, 1015, 940, 850, 800, 790, 750 and 710 $cm^{-1}$.

The NMR ($CDCl_3$; TMS, δ) spectrum reveals peaks at 8.46-8.21, 7.72-7.22, 5.50, 4.57-4.31, and 1.33.

The mass spectrum reveals ions at m/e 297.0988, 252, 225, 203, 175, 158, 130, 102, 77, and 66.

TLC (silica gel GF) yields an $R_f$ of 0.30 (15% isopropyl alcohol/hexane).

The identity of this isomer is uncertain but probably represents attachment of the pyridinyloxymethyl moiety to either C-4 or C-6 of the benzofurancarboxylic acid.

Fractions 103-136 from the above chromatogram afford 808 mg of a crystalline solid. Trituration with 1:4 ether/pentane gave material with melting point 45-75°. Very careful TLC analysis of this product shows that it contains the expected 3:1 mixture of 5- and 7-substituted isomers.

The 808 mg product from the preceding paragraph is rechromatographed on 285 g of 40-60 μ silica gel. The column is equilibrated and eluted with 50% ethyl acetate/hexane.

Fraction 99-111 are combined and afford 203 mg of pure 7-substituted title product. This material crystallizes on standing, and is triturated with ether, filtered and dried. The melting point is 119-120° C.

The IR (νmax (mull)) spectrum reveals peaks at 1738, 1615, 1602, 1559, 1458, 1430, 1372, 1327, 1282, 1277, 1237, 1185, 1125, 1104, 1021, 984, 846, 807, 782, and 704 $cm^{-1}$.

The NMR ($CDCl_3$; TMS, δ) spectrum reveals peaks at 8.45-8.15, 7.75-7.00, 5.30, 5.20, 4.60-4.20, and 1.35.

The mass spectrum reveals ions at m/e 297.1082, 252, 222, 203, 175, 158, 147, 13, 102, 91, 77, 58, and 43.

TLC (silica gel GF) yields an $R_f$ of 0.21 (50% ethyl acetate/hexane).

Fractions 112-115 from the above chromatogram contain a total of 128 mg of mixtures, strongly favoring the more polar product.

Fractions 116-132 are completely clean by TLC and upon combination yield 388 mg of 5-substituted title product. This product crystallizes on standing in the cold, and trituration with ether affords the analytical sample, melting point of 74-75° C.

The IR (νmax (mull)) spectrum reveals peaks at 1725, 1569, 1472, 1380, 1370, 1324, 1297, 1273, 1214, 1204, 1152, 1130, 992, 811, 799, 768, and 706 $cm^{-1}$.

The NMR ($CDCl_3$; TMS, δ) spectrum reveals peaks at 8.40-8.21, 7.75-7.22, 5.20, 4.58-4.32, and 1.43.

The mass spectrum reveals ions at m/e 297.1003, 252, 203, 175, 157, 130, 77, 66, 51, and 39.

TLC (silica gel GF) yields an $R_f$ of 0.19 (50% ethyl

acetate/hexane).

Example A-40 5-[(3-Pyridinyloxy)methyl]-benzofuran-2-carboxylic acid, sodium salt

A solution of 263 mg of 5-substituted title product ester from the preceding experiment (assuming approximately 98% purity, 0.88 mmol) in 5 ml of tetrahydrofuran is treated with 8.2 ml of 0.100 M aqueous sodium hydroxide, and the clear solution is stirred under a nitrogen atmosphere for 18 hr at 25°. Following removal of solvents on the rotary evaporator, the residue is triturated with 3 ml of acetonitrile (2 hr), filtered, washed with 1 ml of acetonitrile and dried (0.1 mm, 25°, 16 hr), thereby affording 225 mg (88% of theory) of pure title compound sodium salt, a white, non-hydroscopic solid, melting point > 300°.

The NMR ($d_6$-DMSO; TMS, $\delta$) spectrum reveals peaks at 8.32-8.18, 7.71-7.09, and 5.23.

The IR ($\nu$max (mull)) spectrum reveals peaks at 1601, 1572, 1474, 1465, 1448, 1418, 1377, 1338, 1279, 1253, 1202, 1052, 944, 839, 787, 771, and 705 $cm^{-1}$.

Example A-41 4-[(3-Pyridinyloxy)methyl]-benzofuran-2-carboxylic acid, sodium salt

A solution of 128 mg (0.43 mmol) of 7-substituted title product from Example D-21 in 4.3 ml of methnaol is treated with 4.3 ml of 0.100 M aqueous sodium hydroxide. The clear solution is stirred under nitrogen at 25° for 24 hr, then concentrated in vacuo. The residue is triturated with 3 ml of acetonitrile (3 hr), filtered, washed with 1 ml of acetonitrile, and dried (0.1 mm, 16 hr, 25°), thereby affording 100 mg (80% of theory) of pure 7-substituted isomer, title product sodium salt, melting point 224-230°.

The NMR ($d_6$-DMSO; TMS, $\delta$) spectrum reveals peaks at 8.38-8.12, 7.56-7.16, 5.41, and 5.27.

The IR ($\nu$max (mull)) spectrum reveals peaks at 1636, 1625, 1612, 1597, 1576, 1496, 1485, 1476, 1462, 1456, 1430, 1408, 1392, 1379, 1345, 1278, 1265, 1260, 1237, 1182, 1016, 942, 838, 804, 793, 775, and 706 $cm^{-1}$.

PREPARATION OF CLASS "B" COMPOUNDS.

Preparation B-1 (p-Tolythio)acetaldehyde diethyl acetal

Refer to Chart J (conversion of LXXXV to LXXXVI).

Using the procedure described in Chap, et al., J. Chem. Soc. 514

(1968), and starting with p-methylthiophenol, the titled compound is prepared.

Preparation B-2 5-Methylbenzo[b]thiophene

Refer to Chart J (conversion of LXXXVI to LXXXVII).

Using the procedures of Chapman, et al., J. Chem. Soc. 514 (1968), and starting with Preparation B-1 the titled compound is prepared, having a melting point of 37-38° C.

Preparation B-3 5-Bromomethylbenzo[b]thiophene

Refer to Chart J (conversion of LXXXVII to XCII).

The titled compound is prepared from the compound of Preparation B-1 using the procedure of Y. Matsuki, et al., Nippon Kugaku Zasshi 87:186 (1966). The compound has a melting point of 97° C.

Preparation B-4 5-Formylbenzo[b]thiophene

Refer to Chart J (conversion of XCII to XCIII).

The titled compound is obtained from the compound of Preparation B-2, using the procedure of Matsuki, et al., Nippon Kasaku Zasshi 87:186 (1966).

Example B-5 5-(3-Pyridinylhydroxymethyl)-benzo[b]thiophene (Formula B-I: $Z_2$ is 3-pyridyl, $X_2$ is -CH(OH)- and is para to the sulfur, $R_2$, $R_7$, $R_9$, and $R_{12}$ are hydrogen, m is zero, and D is a double bond)

Refer to Chart J (conversion of XCIII to LXXXVI).

To a magnetically stirred solution of 3-bromopyridine (9.74 g, 61.63 mmol) in 130 ml of ether, cooled in a -78° C bath, is added over a 5 min period 35.60 ml of 1.6M n-butyllithium in hexane. Stirring is continued at -78° C for 1 hr. At the end of this period, the aldehyde of Preparation B-4 (7.68 g, 47.41 mmol) in 60 ml of ether is added over a 13 min period to the 3-lithiopyridine solution at -78° C. Stirring is then maintained at 0° C to -10° C for 1 hr. The reaction is quenched by addition of 5 ml of saturated $Na_2SO_4$ solution, stirred and diluted with additional ether solvent. The reaction solution is dried in the anhydrous $Na_2SO_4$ powder, and the solvent is removed in vacuo. Chromatography of the resulting oil with 350 g of silica gel in acetone-$CH_2Cl_2$ solvent (1:1) affords 8.3 g of the titled compound as a white solid with melting point 134.5-136.5° C, as recrystallized from ethyl acetate-Skellysolve B (SSB - a commercial mixture of essentially n-hexane.)

TLC using acetone-$CH_2Cl_2$ (1:6) yields an $R_f$ of 0.18.

The C:H:N:S ratio is 69.43:4.48:5.86:13.41.

The mass spectrum yields an ion at 241.0556

The NMR ($CDCl_3$, $\delta$) spectrum reveals peaks at 8.60, 8.40, 7.80, 7.60-7.20, 6.00, and 4.00.

Preparation B-6 5-(3-Pyridinylchloromethyl)-benzo[b]thiophene

Refer to Chart J (conversion of LXXXVIII to LXXXIX).

To a magnetically stirred suspension of the alcohol of Example B-4 (1.50 g, 6.22 mmol) in 15 ml of chloroform is added 3.70 g (31.12 mmol) of thionyl chloride. The resulting solution is refluxed for 1 hr, then cooled to room temperature and poured into 100 ml of ice cold saturated $NaHCO_3$ solution. The aqueous solution is extracted with chloroform, the chloroform solution is washed with saturated $NaHCO_3$ solution, saturated brine and dried through anhydrous $Na_2SO_4$. Removal of the solvent in vacuo gives 1.60 g of the titled product as a golden oil. This material is used without further purification.

TLC using acetone-$CH_2CL_2$ (1:6) yields an $R_f$ of 0.57.

The mass spectrum reveals ions at m/e 259.0241.

The NMR ($CDCl_3$, $\delta$) spectrum reveals peaks at 8.70, 8.55, 7.80, 7.50-7.15, and 6.20.

Example B-7 5-(3-Pyridinylmethyl)-benzo[b]thiophene (Formula B-I: $Z_2$ is 3-pyridyl, $X_2$ is $-CH_2-$ and is para to the sulfur, $R_7$, $R_9$, and $R_{12}$ are hydrogen, m is zero)

To a magnetically stirred solution of Preparation B-5 (1.50 g, 5.79 mmol) in 30 ml of chloroform is added 2.26 g of Zn dust and 0.514 g of propionic acid. Stirring is continued at 25° C for 15 min. The contents are poured into 150 ml of saturated $NaHCO_3$ solution and worked up in the same manner as described above. The crude product is chromatographed with 75 g of silica gel. Elution with EtOAc-SSB (1:1) afforded 0.850 g of the titled product as a yellow oil. Crystallization from ether-hexane yields 0.727 g of product as a crystalline white solid with a melting point 71.0-73.5° C.

TLC using EtOAc-SSB (1:1) yields an $R_f$ of 0.30.

The C:H:N ratio is 74.75:5.14:6.17:14.32.

The mass spectrum reveals ions at m/e 224.052 g.

The NMR ($CDCl_3$, $\delta$) spectrum reveals peaks at 8.60, 7.90-7.10, and 4.05.

Example B-8 5-(3-Pydridinylmethyl)-benzo[b]thiophene-2-carboxylic acid, methyl ester; 5-(3-Pydridinylmethyl)-benzo[b]-

thiophene-2-carboxylic acid; and 5-(3-Pydridinylmethyl)-benzo[b]thiophene-2-carboxylic acid, sodium salt (Formula B-I: $Z_2$ is 3-pyridyl, $X_2$ is $-CH_2-$ and is para to the sulfur, $R_2$, $R_9$, and $R_{12}$ are hydrogen, m is zero, D is a double bond, $R_7$ is -COOH, $-COOCH_3$, or -COONa)

To a magnetically stirred solution of the compound of Example B-6 (0.739 g, 3.28 mmol) in 30 ml of tetrahydrofuran is added 1 equivalent of 1.6M n-BuLi in hexane (2.05 ml). The dark solution is stirred at 25° C for 1 hr. The contents are poured into crushed dry ice in 100 ml of ether and allowed to stand at ambient temperature. 10 ml of water and 25 ml of 1N NaOH are added, and the ether layer is separated. The ether extract is further extracted with 20 ml of 1N NaOH. The combined basic extracts are acidified with 2N $KHSO_4$ to pH 5 and extracted thoroughly with chloroform. The aqueous solution is adjusted to pH 3 and again thoroughly extracted with chloroform. The combined chloroform solution is dried with anhydrous $Na_2SO_4$, and concentrated in vacuo to yield 0.650 g of a foamy solid. This material is triturated with acetone, filtered, and the solid is washed with ether to yield 0.269 g of the free acid corresponding to the titled products as a white powder, melting point 210-212° C.

A sample of this acid in methanol is treated with etheral-$CH_2N_2$ to give the methyl ester having a melting point of 106-108° C.

The corresponding sodium salt of this compound is prepared by treatment of the free acid (80 mg, 0.30 mmol) in 5 ml of methanol with 1 equivalent of 1N NaOH. After complete hydrolysis, the resulting solution is freeze-dried to afford 73 mg of sodium salt with melting point of greater than 300° C.

TLC for the free acid in $CHCl_3$-MeOH-HOAc (92:7:1) yields an $R_f$ of 0.37; and for the ester in acetone-$CH_2Cl_2$ (1:4) an $R_f$ of 0.44.

The mass spectrum for the free acid reveals an ion at m/e 269.0491; for the ester, an ion at m/e 283.0646.

The NMR ($d_6$-DMSO, $\delta$) spectrum for the free acid reveals peaks at 8.70-8.40, 8.10-7.20, and 4.10. For the ester, NMR peaks are observed at 8.65, 8.10, 7.90-7.20, 4.10, and 4.00.

PREPARATION OF CLASS "C" AND CLASS "D" COMPOUNDS

Preparation C-1 Ethyl Benzofuran-2-carboxylate

Refer to Chark K (conversion of XCV to XCVI).

A 3-neck round bottomed flask equipped with a mechanical stirrer, a dropping funnel, a gas inlet tube, and a thermometer is charged with 24.4 g (0.2 mol) of salicyl aldehyde (Aldrich) and 800 ml of THF-EtOH (19:1) under a nitrogen atmosphere. To this solution 150 ml (0.24 mol) of potassium t-butoxide in THF (1.6 M) is added dropwise over 20 min at room temperature. A milky yellow precipitate is formed during the addition. The mixture is stirred at room temperature for one hr. A solution of 57.4 g (0.24 mol) of diethyl bromomalonate (Aldrich) in 20 ml of THF is added dropwise over 10 min. The mixture becomes grayish in color. After stirring for one hr at room temperature, another portion of potassium t-butoxide in THF (150 ml, 0.24 mol) is added dropwise over 40 min at room temperature. TLC shows no starting material remaining after an additional 40 min of stirring. The mixture is poured into 500 ml of brine mixed with crushed ice and extracted twice with ethyl acetate (1L). The organic phase is washed with brine and dried over anhydrous magnesium sulfate. Filtration and concentration affords a deep brown oil. Vacuum distillation affords pure ethyl benzofuran-2-carboxylate (boiling point 88-91° C/0.03 mm, 32.5 g, 85%.)

The NMR ($CDCl_3$; TMS, δ) spectrum reveals peaks at 7.85-7.22, 4.42, and 1.40.

The IR spectrum (film, νmax) reveals peaks at 1720, 1575, 1560, 1480, 1180, 1140, 1090, 1010, 950, 890, 840, and 750 $cm^{-1}$.

The mass spectrum reveals an ion at m/e 190.0622.

The C:H ratio is 69.24:5.32.

Preparation C-2 Ethyl 5-chloromethylbenzofuran-2-carboxylate and Ethyl 4-chloromethylbenzofuran-2-carboxylate

Refer to Chart K (conversion of XCVI to XCVII).

A 250 ml 3-neck (24/40) round bottomed flask, equipped with a magnetic stirring bar, a condenser and a gas bubbler, is charged with a solution of 32.13 g (0.169 mol) of ethyl benzofuran-2-carboxylate (Preparation C-1) dissolved in 85 ml of chloroform. Paraformaldehyde (6.7 g, 0.22 mol) and zinc chloride (6.1 g, 0.045 mol) (dried at 100° C under vacuum for 2 days) are added. The resulting mixture is heated to 50° C and anhydrous hydrogen chloride gas is bubbled slowly through the magnetically stirred mixture. The mixture gradually turns black in color and after stirring for 4 hr TLC analysis indicates only about a 50% conversion to the titled products. Another 6.7 g of

paraformaldehyde (0.22 mol) is added and the resulting mixture is stirred for an additional hr. Little change in the course of the reaction is observed by TLC. The mixture is cooled, diluted with chloroform and washed consecutively with water, saturated aqueous sodium bicarbonate and brine. Drying ($MgSO_4$), filtration and concentration afford 40.98 g of crude product mixture as a dark brown oil.

This mixture is chromatographed on a column containing 1.5 kg of silica gel 60 eluting with Skellysolve B-ethyl acetate (19:1) fractions 7-13 afford unreacted starting material (10.6 g, 33%) (fractions 1-9, 1000 ml; fractions 10-13, 400 ml). Fractions 15-29 (400 ml) contained a mixture of the desired products ethyl 5- (and 4-)chloromethyl-benzofuran-2-carbonylate (18.2 g, 45%) and fractions 30-47 (400 ml) afford 2.1 g (4.3%) of ethyl 4,5-bischloromethyl-benzofuran-2-carboxylate.

The NMR ($CDCl_3$; TMS, $\delta$) spectrum reveals peaks at 7.82-7.10, 4,82, 4.45, and 1.40.

The IR (film, $\nu$max) spectrum reveals peaks are observed at 2970, 1720, 1570, 1470, 1440, 1370, 1320, 1300, 1230, 1190, 1140, 1005, 945, 770, 750, 700, and 620 $cm^{-1}$.

The mass spectrum reveals ions at m/e 238.0384, 204, 193, 175, 159, 131, and 102. (The methyl ester of this compound is also disclosed in U.S. patent 2,754,286.)

Preparation C-3 Ethyl 5-formylbenzofuran-2-carboxylate and Ethyl -formylbenzofuran-2-carboxylate

Refer to Chart K (conversion of XCVII to XCVIII).

To 10 ml of absolute ethanol under a nitrogen atmosphere is added 0.40 g (17.4 mmol) of sodium metal in small pieces over 2-3 min. When gas evolution has ceased and all of the sodium has dissolved, 2-nitropropane (1.55 ml, 16.2 mmol) is added via syringe in one portion. Almost immediately a white precipitate forms which stops the stirring bar. This is broken up by a spatula to give a milky suspension. The resulting magnetically stirred mixture is heated to 65° C for 30 min under a nitrogen atmosphere. To this mixture is added a solution of the starting material dissolved in 20 ml of absolute ethanol. After stirring at 65° C for 3 hr TLC analysis indicates only a trace of starting material remaining. The reaction is quenched by the addition of saturated aqueous ammonium chloride and

ethanol is removed under reduced pressure. The concentrate is diltued with 25 ml of brine and extracted with chloroform (75 ml). This results in an emulsion which can not be broken up. Consequently this mixture is filtered through a Celite pad and the filtrate is diluted with 25 ml brine and equilibrated. The organic layer is drawn off and the aqueous wash is extracted twice with 50 ml portions of chloroform. The organic layers are combined, dried ($MgSO_4$), filtered and concentrated to give 3.19 g of crude titled product as a yellow oil.

The crude product is chromatographed on 192 g of HPLC grade silica gel eluting with hexane-acetone (6:1) and collecting 50 ml fractions. Fractions 10-12 contain a residual amount of unreacted starting material. Partial separation of the aldehyde isomers is observed. Fractions 13-15 afford primarily the 4-formyl isomers, fractions 16-18 give approximately a 1:1 mixture and fractions 19-24 give primarily the 5-formyl derivative. Altogher 2.26 g (77%) of a mixture of isomeric products is obtained. Fraction 19 (essentially pure ethyl 5-formyl-benzofuran-2-carboxylate) is set aside for analysis. This material later solidifies and is recrystallized from ethyl acetate/hexane to give white needles with a melting point of 101-102° C.

The NMR ($CDCl_3$; TMS, $\delta$) spectrum reveals peaks at 10.20, 8.37-7.58, 4.50, and 1.47.

The IR (Nujol, νmax) spectrum reveals peaks at 1726, 1692, 1615, 1588, 1570, 1400, 1350, 1322, 1300, 1266, 1220, 1198, 1145, 1116, 1100, 1021, 949, 940, 919, 842, 834, 781, 767, 750 and 683 $cm^{-1}$.

The mass spectrum reveals ions at m/e 218.0588, 203, 189, 173, 161, 146, and 117.

The C:H ratio is 66.03:4.64.

Example C-4 Ethyl 5-N-imidazolylmethyl-benzofuran-2-carboxylate and Ethyl 4-N-Imidazolylmethyl-benzofuran-2-carboxylate (Formula C-I: $Z_3$ is imidazolyl, $X_3$ is $-CH_2-$ and is para or meta to the oxygen, $R_9$, $R_{12}$, and $R_2$ are hydrogen, D is a double bond, m is zero, and $R_7$ is $-COOCH_2CH_3$)

Refer to Chart K (conversion of XCVII to XCIX).

A two-neck round-bottomed flask equipped with a magnetic stirring bar is charged with 0.264 g (5.5 mmol) of sodium hydride (50% active) under a nitrogen atmosphere. The hydride is washed twice with dry

hexane and suspended in 10 ml of DMF. Imidazole (0.375 g, 5.5 mmol) dissolved in 2 ml of DMF is added dropwise over a period of 5 min. Gas evolution started immediately. The mixture is warmed to 90° C with stirring for one hr. The resulting clear, yellow solution is then cooled to room temperature and a solution of 1.2 g (5.0 mmol) of ethyl 5- (and 4-) chloro-benzyofuran-2-carboxylate in DMF is added dropwise over a period of 5 min. A white precipitate starts to appear. The mixture is stirred at room temperature for 40 min and quenched with brine. Extraction with ethyl acetate is followed by washing the organic layer with water and brine. After drying the solution over anhydrous magnesium sulfate, the solution is filtered and concentrated in vacuo. Liquid chromatography (LC) is carried out by using 324 g silica gel 60 (40-63 μg), eluting with methylene chloride-acetone-ethanol (5:10:0.1) and collecting 40 ml fractions. Fractions homogeneous on TLC are combined and concentrated in vacuo to give the following products: Fractions 27-30 give a pure ethyl 4-N-imidazolylmethyl-benzofuran-2-carboxylate as an oil, (0.272 g, 20%) and fractions 32-42 give pure ethyl 5-N-imidazolylmethyl-benzofuran-2-carboxylate (0.825 g, 61%) (crystallized from ethyl acetate-hexane, with melting point of 96-98° C).

The NMR ($CDCl_3$; TMS, δ) spectrum of the former compound yields peaks at 7.85-6.85, 5.48, 5.44, 4.43, and 1.40.

The IR (film, νmax) spectrum reveals peaks at 1725, 1600, 1570, 1505, 1445, 1430, 1390, 1370, 1330, 1310, 1290, 1280, 1240, 1230, 1190, 1155, 1110, 1080, 1030, 1020, 905, 825, 760, 730, and 660 $cm^{-1}$.

The mass spectrum reveals an ion at m/e 270.1020.

The C:H:N ratio is 66.13:5.48:10.16.

The NMR ($CDCl_3$; TMS, δ) spectrum of the latter compound reveals peaks at 7.82-6.92, 5.24, 4.45, and 1.42.

The IR (Nujol, νmax) spectrum reveals peaks at 1725, 1700, 1570, 1510, 1320, 1300, 1230, 1220, 1200, 1150, 1100, 1080, 840, 760, and 750 $cm^{-1}$.

The mass spectrum reveals an ion at m/e 270.1012.

The C:H:N ratio is 66.79:5.53:10.21.

Example C-5 4-N-Imidazolylmethyl-benzofuran-2-carboxylic Acid, Sodium Salt (Formula C-I: $Z_3$ is imidazoly, $X_3$ is $-CH_2-$ and is meta to the oxygen, $R_2$, $R_9$, and $R_{12}$ are hydrogen, D is a double bond, m is zero, and $R_7$ is

chloromethyl isomer, dissolved in dimethylformamide (2 ml) was added. The resulting solution is stirred under a nitrogen atmosphere at room temperature for 1 hr before another 10 mg of sodium hydride (60% oil dispension) is added. The reaction mixture is stirred for an additional 1 hr more before again adding 20 mg of sodium hydride (60% oil dispersion). After 0.5 hr the reaction mixture is poured into 1:1 brine/water (100 ml) and the solution is extracted with methylene chloride (3 x 100 ml). The combined organic layers are washed with brine (100 ml), dried over magnesium sulfate and concentrated in vacuo to afford 1.68 g of residue. The residue is chromatographed on a 285 g of 40-60 μ silica gel which is eluted with 5% methanol/methylene chloride and fractions of 25 ml are collected. Fractions 51-101 are combined and concentrated in vacuo to yield 1.00 g of residue. Fractions 159-260 are combined and concentrated in vacuo to yield approximately 0.2 g of material. Thin layer chromatography of the 1.0 g residue employing various solvent systems showed it to be two different materials. The 1.0 g product is therefore chromatographed on a 285 g high pressure liquid chromatography column which is eluted with 40% isopropanol/ethyl acetate and 25 ml fractions are collected. Fractions 41-45 are combined and concentrated in vacuo to yield 0.21 g of 5-substituted tilte compound. Fractions 48-86 are combined and concentrated in vacuo to yield 0.59 g of 7-substituted title compound. The combined yield of the two isomers is 51% of theory. A small amount of each isomer is recrystallized in diethyl ether. 5-Substituted title compound had a melting point range of 115-117° C and 4-substituted product has a melting point range of 134-136° C.

The IR (νmax (mull)) spectrum of the 5-substituted title compound reveals peaks at 1721, 1565, 1457, 1376, 1317, 1273, 1213, 1203, 1151, 764, and 741 $cm^{-1}$.

The NMR ($CDCl_3$; TMS, δ) spectrum reveals peaks at 7.70-6.83, 5.17, 4.63-4.27, 2.35, and 1.57-1.27.

The mass spectrum reveals ions at m/e 284.1162, 285, 239, 204, 203, 175, 131, 102, 77, and 39.

TLC (silica gel GF) yields an $R_f$ of 0.32 (40% isopropanol/ethyl acetate).

The IR (νmax (mull)) spectrum of the 4-substituted title product reveals peaks at 1717, 1698, 1466, 1427, 1392, 1376, 1371, 1329, 1303, 1290, 1234, 1187, 1113, 792, 772, 765, 749 $cm^{-1}$.

-COONa)

A round-bottomed flask equipped with a magnetic stirring bar is charged with 135.2 mg (0.5 mmol) of the corresponding ester of Example C-4, 0.55 ml of 1N sodium hydroxide and 1.1 ml of methanol. The resulting mixture is stirred at room temperature for 18 hr.

The mixture is then lyophilized to give a white solid. This solid does not crystallize from acetone-water.

The NMR ($CD_3OD+D_2O$, TMS, $\delta$) spectrum reveals peaks at 8.02-7.00, 5.60 and 5.56.

Example C-6 5-N-Imidazolylmethyl-benzofuran-2-carboxylic Acid, Sodium Salt (Formula C-I: $Z_3$ is imidazolyl, $X_3$ is $-CH_2-$ and is para to the oxygen, $R_2$, $R_9$ and $R_{12}$ are hydrogen, D is a double bond, m is zero, and $R_7$ is -COONa)

A round-bottomed flask equipped with a magnetic stirring bar is charged with 540.6 mg (2.0 mmol) of the corresponding ester of Example C-4, 2.2 ml of 1N sodium hydroxide and 4.4 ml of methanol. The resulting mixture is stirred at room temperature for 18 hr. The mixture is then lyophilized to give a white solid. Crystallization from acetone-water gives 503.5 mg (95%) of the sodium salt, with a melting point of greater than 270° C.

The NMR ($CD_3OD + D_2O$, TMS, $\delta$) spectrum reveals peaks at 7.85-7.02 and 5.34.

Example C-7 5-[(2-Methyl-1-imidazolyl)methyl]-benzofuran-2-carboxylic acid, ethyl ester and 4-[(2-methyl-1-imidazolyl)methyl]-benzofuran-2-carboxylic acid, ethyl ester (Formula C-I: $Z_3$ is 2-methyl-1-imidazolyl, $X_3$ is $-CH_2-$ and is para or meta to the oxygen, $R_9$, $R_{12}$, and $R_2$ are hydrogen, m is zero, D is a double bond, and $R_7$ is $-COOCH_2CH_3$)

Refer to Chart K (conversion of XCVII to CIX).

To a solution of 0.220 g of sodium hydride (60% oil dispersion) in dimethylformamide (10 ml) is added to a solution of 0.455 g of 2-methylimidazole in dimethylformamide (5 ml) over a five min period. Hydrogen gas evolution occurs. The stirred solution is heated to 90° C for 1 hr under a nitrogen atmosphere then cooled to room temperature, after which 1.2 g of a 3:1 mixture of 5-(chloromethyl)-benzofuran-2-carboxylic acid, ethyl ester and the corresponding 4-

The NMR ($CDCl_3$; TMS, $\delta$) spectrum reveals peaks at 7.65-6.73, 5.36, 5.30, 4.58-4.25, 2.44, 2.34, and 1.51-1.33.

The mass spectrum reveals ions at m/e 284.1168, 285, 211, 204, 203, 176, 175, 131, 102, and 77.

TLC (silica gel GF) yields an $R_f$ of 0.41 (40% isopropanol/ethyl acetate).

Example C-8 5-[(2-Methyl-1-imidazolyl)methyl]-benzofuran-2-carboxylic acid, sodium salt (Formula C-I: $Z_3$ is 2-methyl-1-imidazolyl, $X_3$ is para to the oxygen, $R_9$, $R_{12}$, and $R_2$ are hydrogen, m is zero, D is a double bond, and $R_7$ is -COONa)

To a solution of 0.4457 g of 5-[(2-methyl-1-imidazolyl)methyl]-benzofuran-2-carboxylic acid, ethyl ester in 8 ml of tetrahydrofuran is added 14.89 ml of 0.10M aqueous sodium hydroxide. The reaction mixture is stirred under a nitrogen atmosphere at room temperature for 26 hr before concentrating in vacuo to dryness. The resulting residue is triturated with acetonitrile (5 ml) for 2 hr. The mixture is filtered and the crystals washed with acetonitrile (2 ml), then dried in vacuo for 33 hr to yield 0.395 g of product (95% of theory) with a melting point greater than 300° C.

The IR ($\nu$max, (mull)) spectrum reveals peaks at 1603, 1566, 1533, 1429, 1407, 1391, 1378, 1357, 1284, 1138, 943, 935, 805, 793, 766, and 762 $cm^{-1}$.

Example C-9 4-[(2-Methyl-1-imidazolyl)methyl]-benzofuran-2-carboxylic acid, sodium salt (Formula C-I: $Z_3$ is 2-methyl-1-imidazolyl, $X_3$ is meta to the oxygen, $R_9$, $R_{12}$, and $R_2$ are hydrogen, m is zero, D is a double bond, and $R_7$ is -COONa)

To a solution of 0.1138 g of 4-[(2-methyl-1-imidazolyl)methyl]-benzofuran-2-carboxylic acid, ethyl ester in 8 ml of tetrahydrofuran is added 3.80 ml of 0.10M aqueous sodium hydroxide. The reaction stirred at room temperature under a nitrogen atmosphere for 26 hr. The solution is concentrated in vacuo to dryness and the residue is triturated with acetonitrile (3 ml) for 2 hr. The solution is filtered and the crystals washed with acetonitrile ( 2 ml), and then dried in vacuo for 33 hr to yield 66 mg of pure product (79% of theory) with melting point 180-200° C.

The IR ($\nu$max, (mull)) spectrum reveals peaks at 3367, 1615, 1568,

1530, 1501, 1495, 1462, 1459, 1426, 1387, 1346, 1285, 1186, 1155, 1132, 1077, 944, 811, 784, 766, 745, and 723 $cm^{-1}$.

Example C-10 5-[(4-Methyl-1-imidazolyl)methyl]-benzofuran-2-carboxylic acid, ethyl ester and 4-[(4-methyl-1-imidazolyl)methyl]-benzofuran-2-carboxylic acid, ethyl ester (Formula C-I: $Z_3$ is 4-methyl-1-imidazolyl, $X_3$ is $-CH_2-$ and is para to the oxygen, $R_9$, $R_{12}$, and $R_2$ are hydrogen, D is a double bond, m is zero, and $R_7$ is $-COOCH_2CH_3$)

Refer to Chart K (conversion of XCVII to CIX).

A 50 ml round-bottomed, 2-necked flask fitted with a condenser and magnetic stirrer, is flame-dried, then cooled in an atmosphere of nitrogen. The flask is then charged with 220 mg (5.5 mmol) of sodium hydride (60% dispension), from which the oil is washed with two 5 ml portions of hexane. (Hexane is removed with vacuum via a micro gas dispersion tube.) Dimethylformamide (10 ml) is added, and the stirred suspension is treated over 5 min with a solution of 455 mg (5.5 mmol) of 4-methylimidazole in 2 ml of dimethylformamide (hydrogen evolution). The mixture is stirred at 90° for 1 hr (probably more vigorous than necessary) and became a clear light yellow solution. This solution is recooled to ambient temperature and treated with a solution of 1.2 g (5 mmol) of a 3:1 mixture of 5-(chloromethyl)-benzofuran-2-carboxylic acid ethyl ester and the corresponding 4-chloromethyl isomer in 2 ml of dimethylformamide over 5 min. Although sodium chloride began to precipitate almost immediately, TLC analysis of an aliquot after 1 hr of stirring at 25° showed that about 10-15% of starting chloro-ester 1 remained. More sodium hydride (20 mg) is added, and the mixture is stirred for an additional 45 min.

The reaction mixture is poured into 1:1 brine/water and extracted thoroughly with methylene chloride. The extracts are washed with water several times, then with brine, dried over sodium sulfate and concentrated in vacuo. Residual dimethylformamide is removed in a stream of nitrogen (16 hr).

The crude product (1.5 g) is chromatographed on a column containing 150 g of silica gel. The column is packed and eluted (15 ml fractions) with 60% acetone/methylene chloride.

Fractions 90-108 are combined based on their TLC homogeneity and afford 233 mg of 4-substitued benzofuran derivative (title compound),

a light yellow oil.

The IR (νmax (neat)) spectrum reveals peaks at 3100, 1710, 1595, 1560, 1490, 1440, 1365, 1300, 1290, 1240, 1180, 1100, 1015, 945, 760, 660, and 620 $cm^{-1}$.

The NMR ($CDCl_3$; TMS, δ) spectrum reveals peaks at 7.6-6.6, 5.41, 5.30, 4.58-4.31, 2.21 and 1.42.

The mass spectrum reveals ions at m/e 284.1160, 239, 203, 175, 131, 102, and 77.

TLC (silica gel GF) yields an $R_f$ of 0.26 (70% acetone/methylene chloride).

Continued elution of the above chromatogram with 60% acetone/-methylene chloride affords (fractions 120-161) 618 mg of 5-substituted title compound. This material, homogeneous by TLC, cyrstallizes spontaneously and upon recrystallization from ether/hexane yields 320 mg of 5-substituted title compound with melting point 68-71° C.

The IR (νmax (mull)) spectrum reveals peaks at 2930, 2856, 1725, 1466, 1375, 1332, 1316, 1298, 1234, 1200, 1143, 1010, 835, 766, 760, 742 $cm^{-1}$.

The NMR ($CDCl_3$; TMS, δ) spectrum reveals peaks at 7.63-6.62, 5.15, 4.58-4.31, 2.23, 2.10, and 1.42.

The mass spectrum reveals ions at m/e 284.1154, 239, 203, 175, 157, 131, 119, 102, 77, 63, and 51.

TLC (silica gel GF) yields an $R_f$ of 0.19 (70% acetone/methylene chloride).

Example C-11 5-[(4-Methyl-1-imidazolyl)methyl]-benzofuran-2-carboxylic acid, sodium salt (Formula C-I: $Z_3$ is 4-methyl-1-imidazolyl, $X_3$ is $-CH_2-$ and is para to the oxygen, $R_9$, $R_{12}$, and $R_2$ are hydrogen, D is a double bond, m is zero, and $R_7$ is -COONa)

A solution of 150 mg of the 5-substituted isomer from Example C-10 in 5 ml of methanol is treated with 5.0 ml of 0.1M aqueous sodium hydroxide and the resulting clear solution is stirred for 64 hr at 25° in an atmosphere of nitrogen. The methanol and water are removed on the rotary evaporator, and the residue is triturated with acetonitrile (approximately 5 ml). Filtration, washing of the solids with about 1 ml of fresh acetonitrile and drying (25°, 0.1 mm, 16 hr) affords 123 mg (85% of theory) of pure sodium salt title product melting point of 290-294° C.

The IR (νmax (mull)) spectrum reveals peaks at 3350, 1614, 1570, 1502, 1450, 1378, 942, 789 and 763 $cm^{-1}$.

Example C-12 4-[(4-Methyl-1-imidazolyl)methyl]-benzofuran-2-carboxylic acid, sodium salt (Formula C-I: $Z_3$ is 4-methyl-1-imidazolyl, $X_3$ is meta to the oxygen, $R_9$, $R_{12}$, and $R_2$ are hydrogen, D is a double bond, m is zero, and $R_7$ is -COONa)

Using a procedure and amounts identical to those in the preceding experiment, 150 mg of 4-substituted ethyl ester is converted to the sodium salt. The crude salt is triturated with 5 ml of acetonitrile, filtered, washed with additional acetonitrile, and dried (16 hr, 25° , 0.1 mm), thereby affording 84 mg of pure title compound, melting point 237-240° C.

The IR (νmax (mull)) spectrum reveals peaks at 3371, 1613, 1567, 1504, 1455, 1380, 1345, 1185, 1157, 1107, 944, 812, 785 and 760 $cm^{-1}$.

TABLE I

FORMULAS

F-I

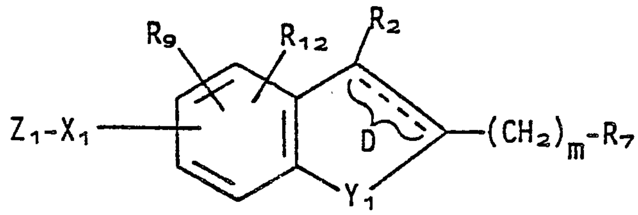

Class "A"

A-I

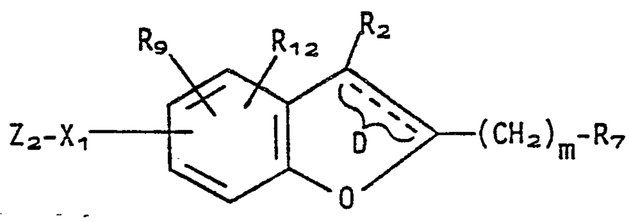

Class "B"

B-I

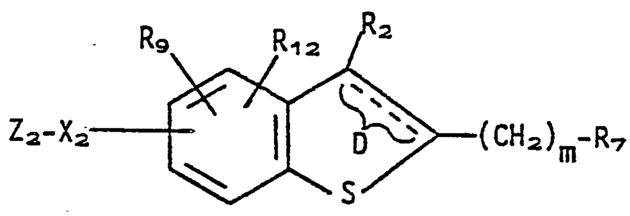

Class "C"

C-I

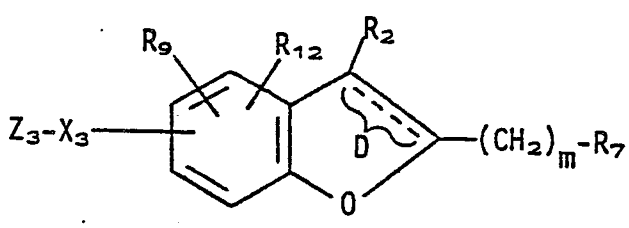

Class "D"

D-I

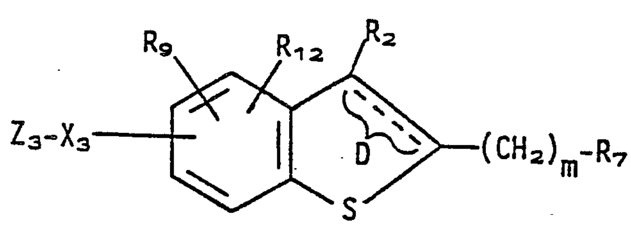

CHART B

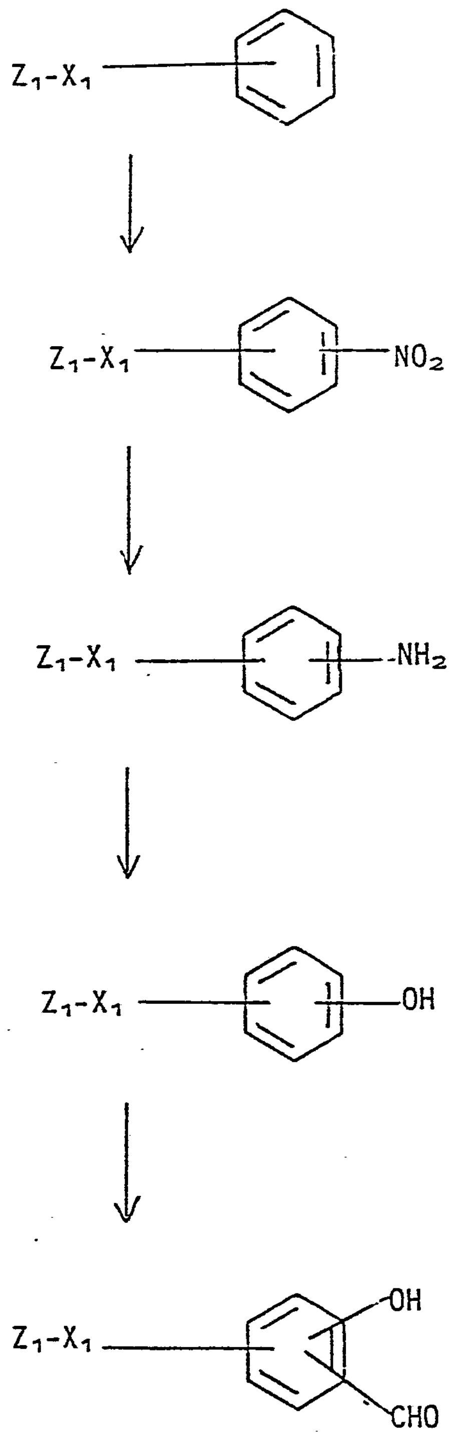

XX

XXI

XXII

XXIII

XXIV

CHART A

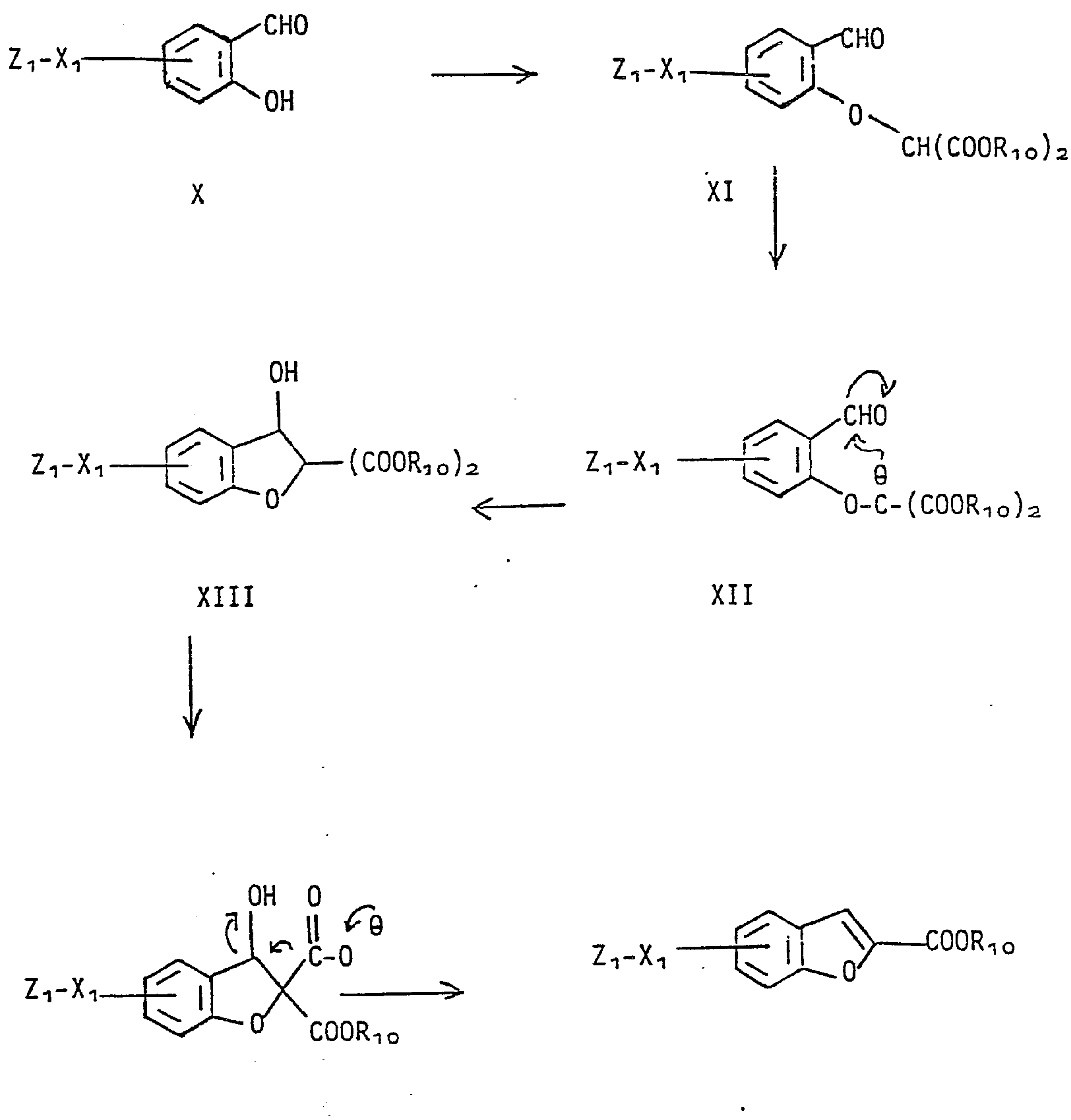

X

XI

XII

XIII

XIV

XV

CHART C

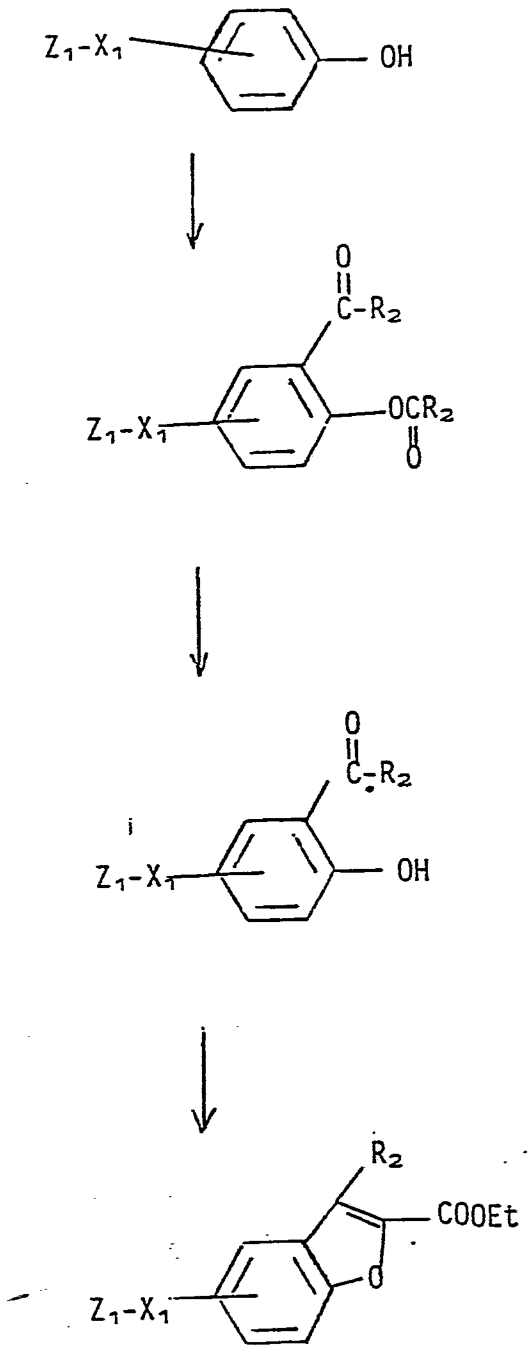

XXX

XXXI

XXXII

XXXIII

CHART E

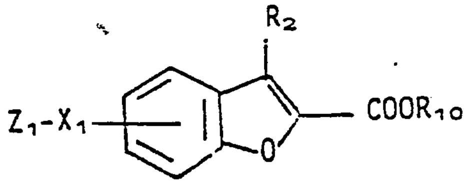

L

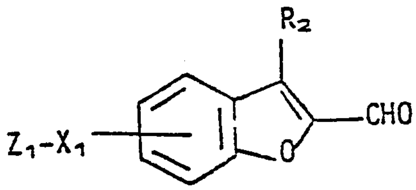

LI

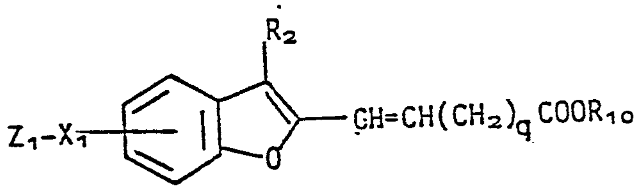

LII

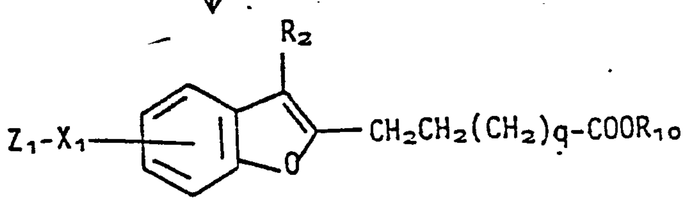

LIII

CHART D

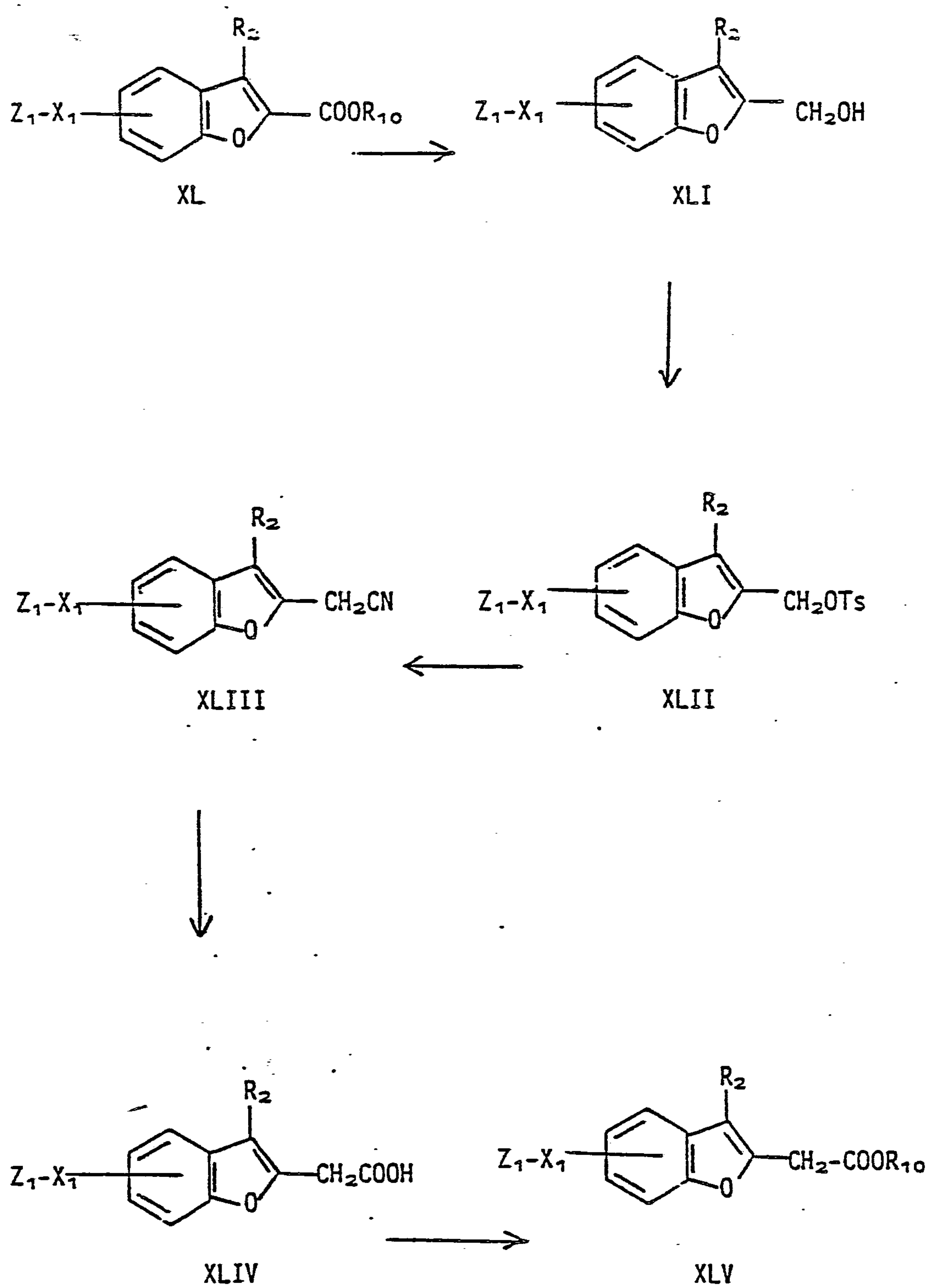

XL

XLI

XLII

XLIII

XLIV

XLV

CHART G

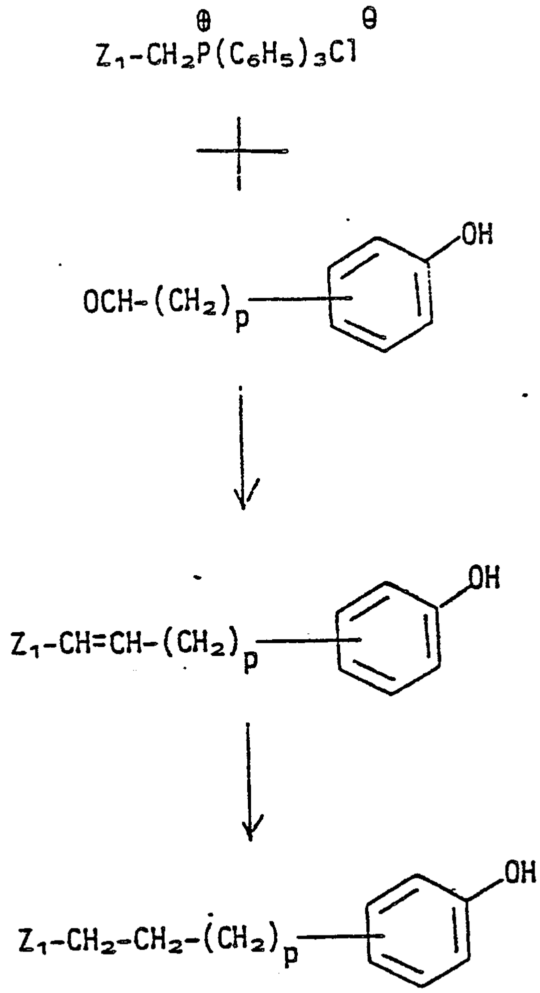

LXXII

LXXI

LXXIII

LXXIV

CHART F

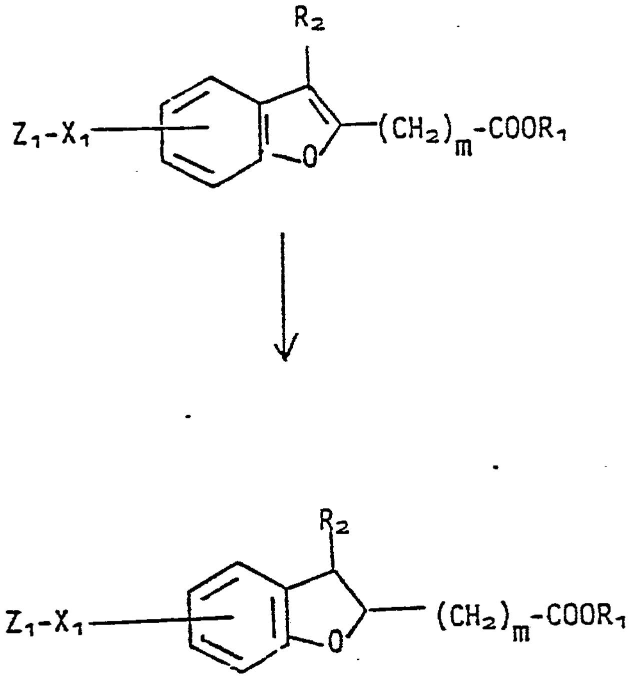

LX

LXI

CHART H

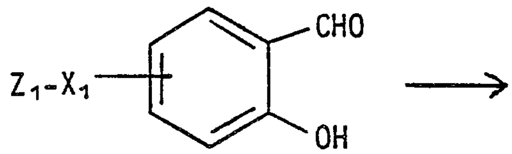

LXXV

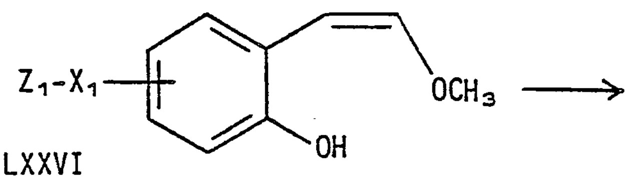

LXXVI

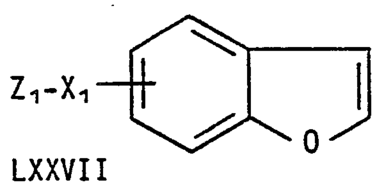

LXXVII

CHART I

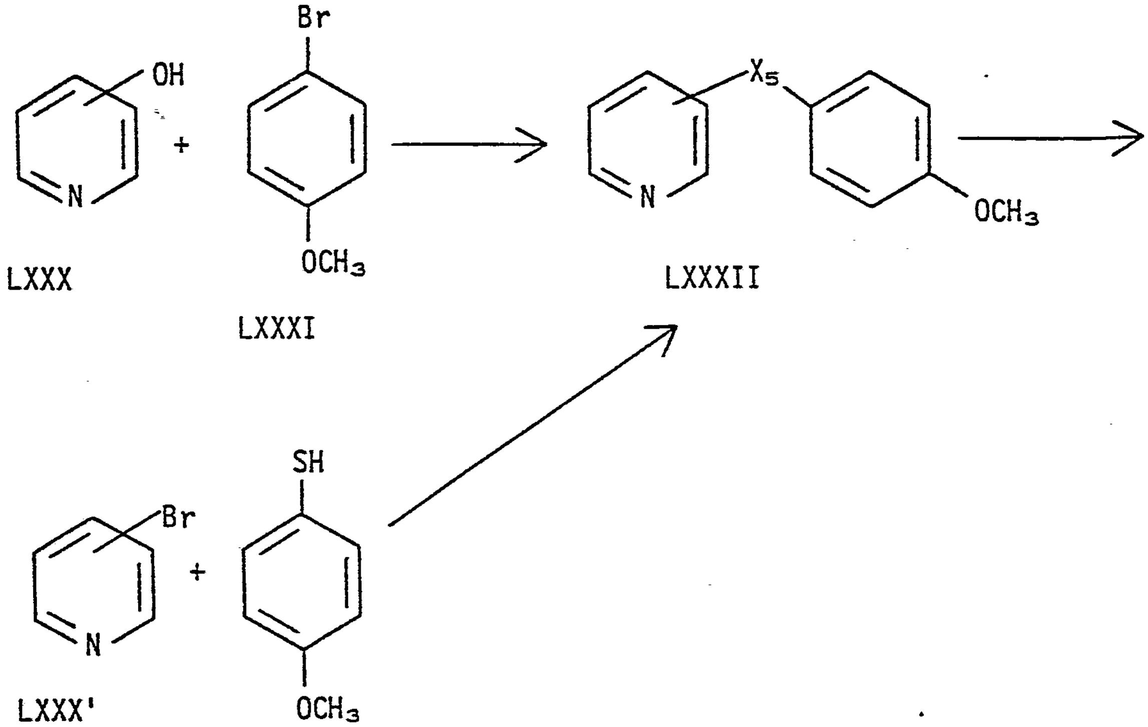

LXXX

LXXXI

LXXXII

LXXX'

LXXXI'

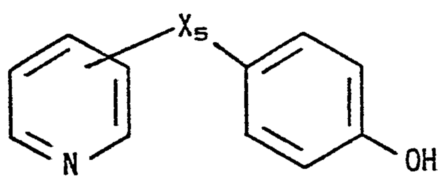

LXXXIII

CHART J

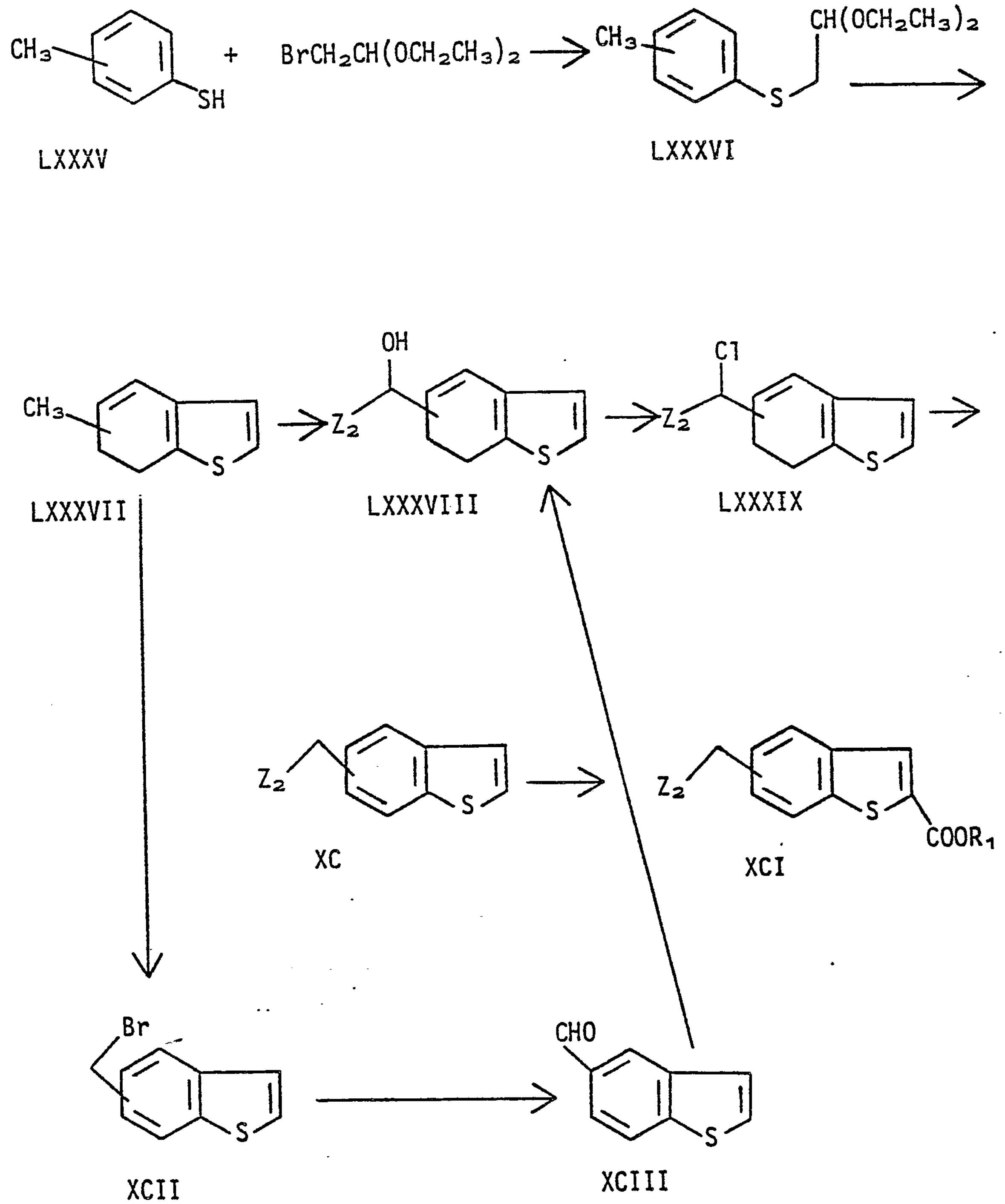

LXXXV LXXXVI LXXXVII LXXXVIII LXXXIX XC XCI XCII XCIII

CHART K

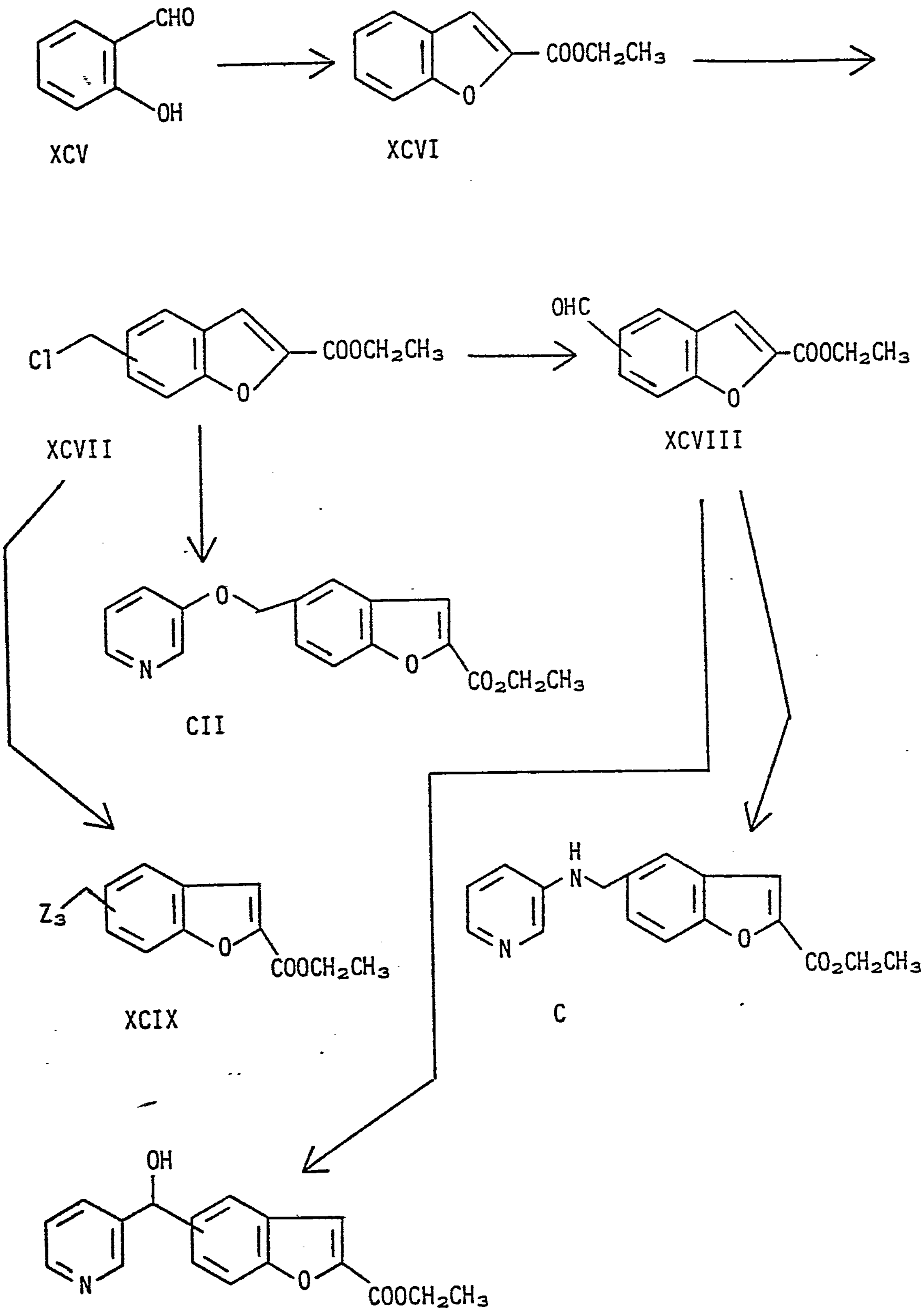

XCV

XCVI

XCVII

XCVIII

CII

XCIX

C

CI

CHART L

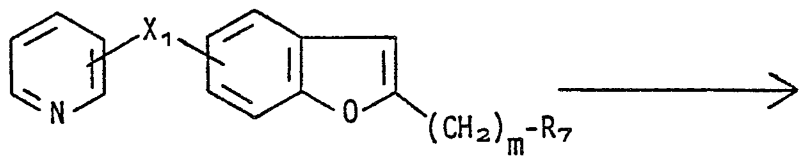

CV

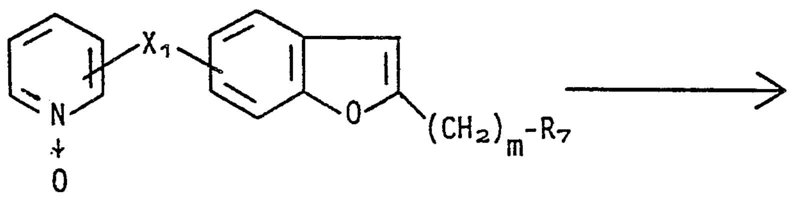

CVI

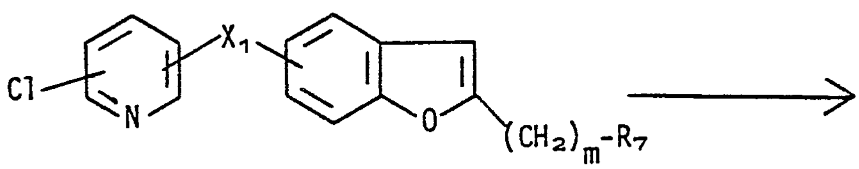

CVII

CHART M

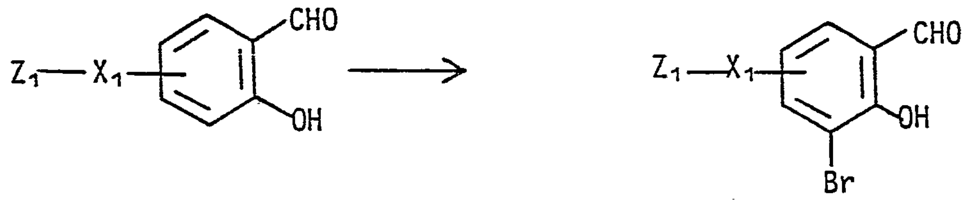

CX
(XXIV, Chart B)

CXI

CHART N

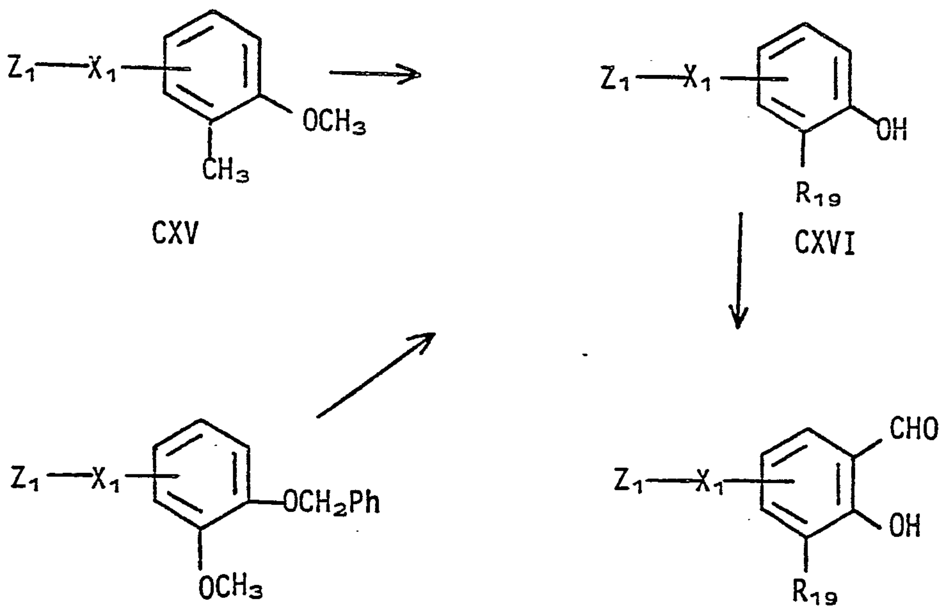

CXV

CXVI

CXV'

CXVII

CHART O

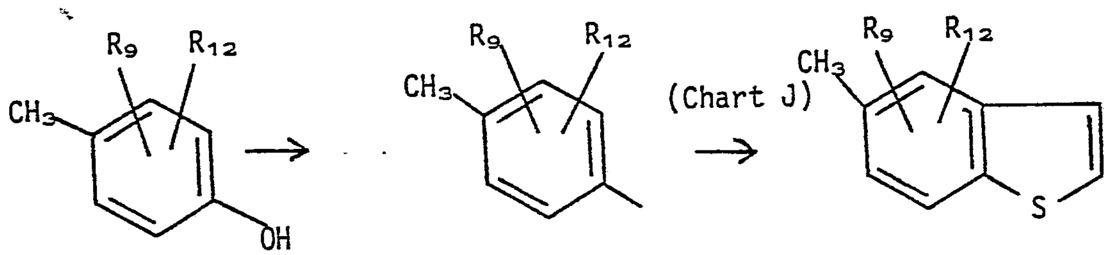


CXXX CXXXI CXXXII

CHART P

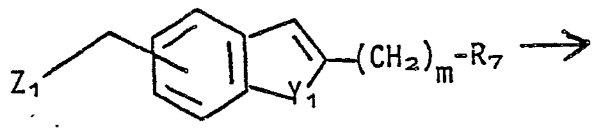

CXX

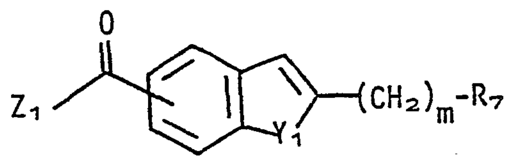

CXXI

CHART Q

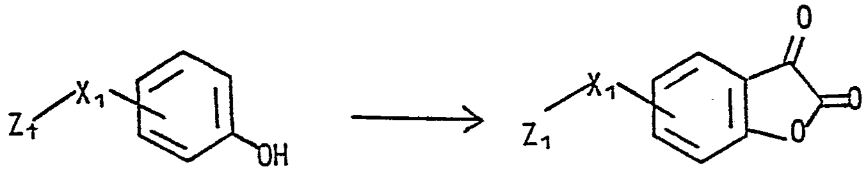

CL CLI

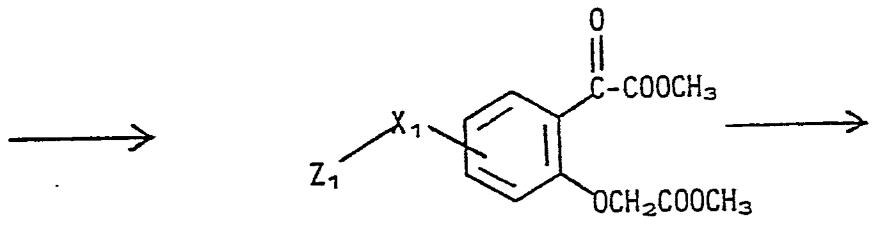

CLII

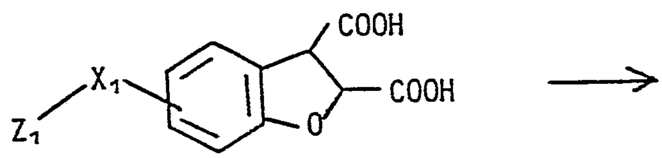

CLIII

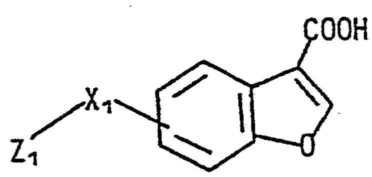

CLIV

CHART R

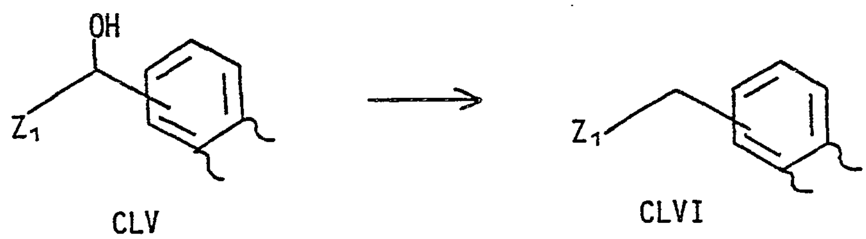

CLV CLVI

CLAIMS

1. A compound of the formula

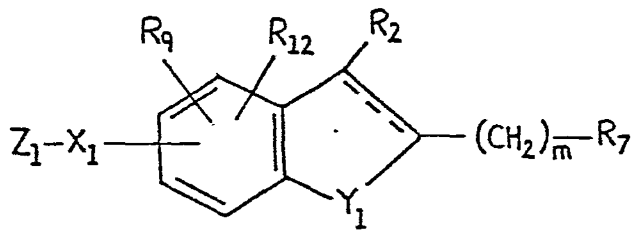

wherein $Z_1$ is 4-pyridinyl, 3-pyridinyl, 4-methyl-3-pyridinyl, 4-methoxy-3-pyridinyl, 4-dimethylamino-3-pyridinyl, 4-amino-3-pyridinyl, 2-, 4-, 5- or 6-chloro-3-pyridinyl, imidazolyl or ($C_{1-3}$ alkyl)imidazolyl;

$X_1$ is $-(CH_2)_n-$, -O-, -S-, -SO-, $-SO_2-$, $-CH_2-O-$, $-O-CH_2-$, $-CH_2-NR_3-$, $-NR_3-CH_2-$, -CHOH- or -CO- in which n is 0, 1, 2, 3 or 4 and $R_3$ is hydrogen or methyl, provided that $Z_1$ is optionally substituted pyridinyl as defined above when $X_1$ is not $-(CH_2)_n-$, -CHOH- or $-O-CH_2-$;

$Y_1$ is -O- or -S-, provided that $X_1$ is not -SO- or $-SO_2-$ when $Y_1$ is -S-;

$R_2$ is hydrogen, $C_{1-3}$ alkyl, phenyl or -COOR; in which $R_1$ is hydrogen, a pharmacologically acceptable cation, $C_{1-12}$ alkyl, $C_{3-10}$ cycloalkyl, $C_{7-12}$ aralkyl, phenyl optionally substituted by up to 3 substituents independently selected from chlorine and $C_{1-3}$ alkyl, or phenyl para-substituted by $-NHCO-R_{25}$, $-O-CO-R_{26}$, $-CO-R_{24}$, $-O-CO-(p-Ph)-R_{27}$ or $-CH=N-NH-CO-NH_2$ in which $R_{24}$ is phenyl or acetamidophenyl, $R_{25}$ is methyl, phenyl, acetamidophenyl, benzamidophenyl or amino, $R_{26}$ is methyl, phenyl, amino or methoxy, $R_{27}$ is hydrogen or acetamido, and p-Ph is 1,4-phenylene;

$R_7$ is hydrogen, $-CH_2OH$, -COOR, in which $R_1$ is as defined above, -CN or $-CH_2N(R_4)_2$, $-CON(R_4)_2$ or $-CO-R_4$ in

which $R_4$ is hydrogen, $C_{1-4}$ alkyl or phenyl;

either $R_9$ and $R_{12}$ are independently selected from hydrogen, hydroxy, $C_{1-4}$ alkyl, fluorine, chlorine, bromine and methoxy, or $R_9$ and $R_{12}$ are attached to adjacent carbon atoms and together are $-O-CH_2-O-$;

$\underline{---}$ represents a single bond (in which case the compound may be in enantiomeric or racemic form) or a double bond; and

m is 0,1, 2, 3 or 4;

or a pharmacologically acceptable acid addition salt thereof.

2. A compound as claimed in claim 1, wherein $Y_1$ is -O-.

3. A compound as claimed in claim 1, wherein $Y_1$ is -S-.

4. A compound as claimed in any preceding claim, wherein $Z_1$ is optionally substituted pyridinyl as defined in claim 1.

5. A compound as claimed in any of claims 1 to 3, wherein $Z_1$ is optionally substituted imidazolyl as defined in claim 1.

6. A compound as claimed in any preceding claim, wherein $X_1$ is $-CH_2-$ or $-(CH_2)_2-$, m is 0, D is a double bond, $R_2$, $R_9$ and $R_{12}$ are each hydrogen, and $R_7$ is $-COOR_1$ in which $R_1$ is as defined in claim 1.

7. A compound selected from

5-(3-pyridinoyl)-2-benzofurancarboxylic acid,

7-methoxy-5-(3-pyridinylmethyl)-2-benzofurancarboxylic acid, ethyl ester or sodium salt,

7-bromo-5-(3-pyridylmethyl)-2-benzofurancarboxylic acid, ethyl ester or sodium salt,

7-methyl-5-(3-pyridylmethyl)-2-benzofurancarboxylic acid, ethyl ester or sodium salt,

5-(3-pyridyl)-2-benzofurancarboxylic acid, ethyl ester or sodium salt,

5-[(3'-pyridinyl)aminomethyl]-benzofuran-2-carboxylic acid, ethyl ester or sodium salt,

4-[(3'-pyridinyl)aminomethyl]-benzofuran-2-carboxylic acid, ethyl ester or sodium salt,

5-[(3'-pyridinyl)hydroxymethyl]-benzofuran-2-carboxylic acid, ethyl ester or sodium salt,

ethyl 4-[(3'-hydroxymethyl]-benzofuran-2-carboxylate,

sodium 4-[(3'-pyridinyl)hydroxymethyl]-benzofuran-2-carboxylate,

5-(3-pyridinyloxy)benzofuran-2-carboxylic acid, ethyl ester, or sodium salt,

5-(3-pyridinylthio)-benzofuran-2-carboxylic acid, ethyl ester or sodium salt,

5-[(3-pyridinyloxy)methyl]-benzofuran-2-carboxylic acid, ethyl ester, or sodium salt, and

4-[(3-pyridinyloxy)methyl]-benzofuran-2-carboxylic acid, ethyl ester or sodium salt.

8. A compound selected from

5-(3'-pyridinylmethyl)benzofuran-2-carboxylic acid, ethyl ester or sodium salt,

5-[2-(3'-pyridinyl)ethyl]benzofuran-2-carboxylic acid, ethyl ester or sodium salt,

6-[2-(3'-pyridinyl)ethyl]-benzofuran-2-carboxylic acid, ethyl ester or sodium salt,

2-hydroxymethyl-5-(3'-pyridinylmethyl)benzofuran or its hydrochloride,

5-(3-pyridinylmethyl)-2-benzofurancarboxylic acid or its hydrochloride or amide,

5-[3-(2-chloropyridyl)methyl]-2-benzofurancarboxylic acid, ethyl ester or sodium salt,

5-[3-(4-chloropyridyl)methyl]-2-benzofurancarboxylic acid, ethyl ester or sodium salt,

5-[3-(5-chloropyridyl)methyl-2-benzofurancarboxylic acid, ethyl ester,

5-[3-(5-chloropyridyl)methyl]-2-benzofurancarboxylic acid, ethyl ester or sodium salt,

6-(3-pyridylmethyl)benzofuran-2-carboxylic acid, or its ethyl ester or sodium salt, and

5-(3'-pyridinylmethyl)benzofuran.

9. A compound selected from 5-(3-pyridinylhydroxymethyl)benzo[b]thiophene, 5-(3-pyridinylmethyl)benzo[b]thiophene, and 5-(3-pyridinylmethyl)benzo[b]thiophene-2-carboxylic acid or its methyl ester or sodium salt.

10. A compound selected from 4-N-Imidazolylmethyl-benzofuran-2-carboxylic acid, ethyl ester or sodium salt, 5-N-imidazolylmethyl-benzofuran-2-carboxylic acid, ethyl ester or sodium salt, 5-[(2-methyl-1-imidazolyl)methyl]-benzofuran-2-carboxylic acid, ethyl ester or sodium salt, 4-[(2-methyl-1-imidazolyl)methyl]-benzofuran-2-carboxylic acid, ethyl ester or sodium salt, 5-[(4-methyl-1-imidazolyl)methyl]-benzofuran-2-carboxylic acid, ethyl ester or sodium salt, and 4-[(4-methyl-1-imidazolyl)methyl]-benzofuran-2-carboxylic acid, ethyl ester or sodium salt.